(19) Europäisches Patentamt
European Patent Office
Office européen des brevets

(11) **EP 4 144 751 A1**

(12) **EUROPEAN PATENT APPLICATION**

(43) Date of publication:
**08.03.2023 Bulletin 2023/10**

(21) Application number: **21194414.5**

(22) Date of filing: **01.09.2021**

(51) International Patent Classification (IPC):
**C07K 14/005** (2006.01) **A61K 39/12** (2006.01)

(52) Cooperative Patent Classification (CPC):
**C07K 14/005; A61K 39/12; G16B 15/30;**
C12N 2770/20022; C12N 2770/20034

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Max-Delbrück-Centrum für Molekulare Medizin in der**
  **Helmholtz-Gemeinschaft**
  **13125 Berlin (DE)**
- **ETH Zürich**
  **8092 Zürich (CH)**

(72) Inventors:
- **DE LA ROSA, Kathrin**
  **10437 Berlin (DE)**
- **LEBEDIN, Mikhail**
  **16321 Bernau (DE)**

- **RATSWOHL, Christoph**
  **13051 Berlin (DE)**
- **VAZQUEZ GARCIA, Clara**
  **13349 Berlin (DE)**
- **SILVIS, Casper**
  **13088 Berlin (DE)**
- **EYER, Klaus**
  **8049 Zürich (CH)**
- **SIGVALDADÓTTIR, Ingibjörg**
  **8032 Zürich (CH)**
- **OLSSON, Simon**
  **41265 Gothenburg (SE)**

(74) Representative: **Kador & Partner PartG mbB**
**Corneliusstraße 15**
**80469 München (DE)**

Remarks:
The complete document including Reference Table(s) and the Sequence Listing(s) can be downloaded from the EPO website

(54) **NEW CORONAVIRUS VACCINE AND METHOD FOR DESIGNING AND OBTAINING A VIRUS VACCINE**

(57) The present invention relates to a mutant receptor-binding domain (mRBD) of a coronavirus (mRBD-CORONA) or a fragment thereof and mutant spike protein of the coronavirus (CORONA-mSpike) or a fragment thereof comprising the CORONA-mRBD or the fragment thereof.

Furthermore, the present invention relates to a polypeptide or protein comprising the mRBD-CORONA or the fragment thereof or CORONA-mSpike or the fragment thereof and a nucleic acid comprising a nucleotide sequence encoding for the mRBD-CORONA or the fragment thereof or the CORONA-mSpike or the fragment thereof.

Furthermore, the present invention relates to a vaccine composition comprising one or more CORONA-mRBDs or fragments thereof, one or more CORONA-mSpikes, one or more polypeptides or proteins and/or one or more nucleic acids according to the present invention.

Furthermore, the present invention relates to the one or more CORONA-mRBDs or fragments thereof, the one or more CORONA-mSpikes, the one or more polypeptides or proteins, the one or more nucleic acids and/or the vaccine composition according to the present invention for use in the prevention and/or treatment of diseases caused by coronaviruses in a subject. Furthermore, the present invention relates to a method for designing and/or obtaining an active ingredient for a vaccine composition and to a VIRUS-mRBD or a fragment thereof designed and/or obtained by the method for obtaining the VIRUS-mRBD according to the present invention.

Fig. 7

**Description**

The present invention relates to a mutant receptor-binding domain (mRBD) of a coronavirus (mRBD-CORONA) or a fragment thereof and mutant spike protein of the coronavirus (CORONA-mSpike) or a fragment thereof comprising the CORONA-mRBD or the fragment thereof.

Furthermore, the present invention relates to a polypeptide or protein comprising the mRBD-CORONA or the fragment thereof or CORONA-mSpike or the fragment thereof and a nucleic acid comprising a nucleotide sequence encoding for the mRBD-CORONA or the fragment thereof or the CORONA-mSpike or the fragment thereof.

Furthermore, the present invention relates to a vaccine composition comprising one or more CORONA-mRBDs or fragments thereof, one or more CORONA-mSpikes, one or more polypeptides or proteins and/or one or more nucleic acids according to the present invention.

Furthermore, the present invention relates to the one or more CORONA-mRBDs or fragments thereof, the one or more CORONA-mSpikes, the one or more polypeptides or proteins, the one or more nucleic acids and/or the vaccine composition according to the present invention for use in the prevention and/or treatment of diseases caused by coronaviruses in a subject. Furthermore, the present invention relates to a method for designing and/or obtaining an active ingredient for a vaccine composition and to a VIRUS-mRBD or a fragment thereof designed and/orobtained by the method for obtaining the VIRUS-mRBD according to the present invention.

Angiotensin-converting enzyme 2 (ACE2) serves as a receptor for severe acute respiratory syndrome coronavirus 2 (SARS-CoV-2) mediating cell entry. The membrane bound form of the ACE2 receptor is cleaved by proteases, such as ADAM17 (Tumor necrosis factor-alpha convertase) and TMPRSS2 (transmembrane protease serine subtype) that promote shedding of soluble ACE2 (sACE2) into the extracellular space. Soluble ACE2 exists in an enzymatically active form (active ACE2), and in an enzymatically inactive form (inactive ACE2).

Importantly, the ability of the serum to neutralize the virus depends on neutralizing antibodies (nABs). The induction of antibodies that interfere with ACE2-spike interaction are crucial to develop a neutralizing immune response and confer protective immunity. However, activation of B cells and affinity maturation of potent antibodies strongly depends on the availability of the antigen, i.e. the spike proteins or receptor binding domains (RBD) of the spike protein.

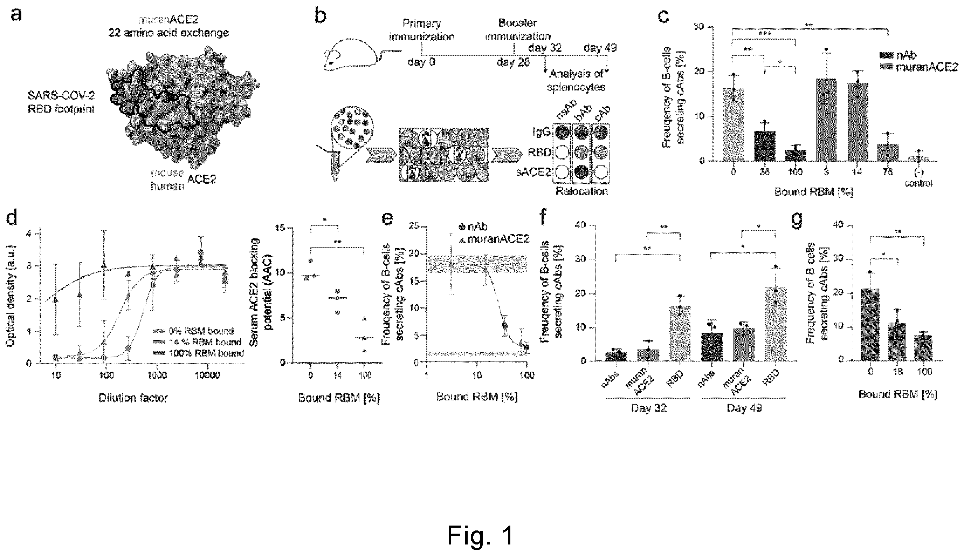

The present invention is based on the discovery of the inventors, that binding of soluble receptors to an immunogen hinder humoral immunity by masking the antigen (Fig. 1). For SARS-CoV-2 we could show that high concentrations of sACE2 masks the SARS-CoV-2 spike during infection and immunization, which consequently interferes with antibody maturation towards the SARS-CoV-2 receptor-binding motif (RBM), which is the receptor binding interface of the RBD. We could show that high concentrations of the soluble receptor and/or high binding affinity augment epitope masking. Importantly, we could show that introduction of mutations preventing ACE2 binding abrogated masking by soluble ACE2 and thereby fostered generation of RBM-binding antibodies in presence of the soluble receptor. Our discovery is of particular relevance for the design of vaccine antigens, because immune responses to vaccines can be improved by prevention of binding to host receptors.

Similar to ACE2, other receptors for viruses can be shed, for instance DPP4, which is the receptor for MERS-CoV coronavirus and present at high concentrations in the blood of healthy human subjects. Likewise, other viruses do use receptors for viral entry that are also present in a soluble version at a concentration which might interfere with immune responses.

Meanwhile there is evidence that certain vaccinations/immunizations/natural infections do not confer sufficient immunity to SARS-CoV-2 in 5-40% of individuals. And that antibodies raised against the RBD decline faster than those against other parts of the viral spike (https://www.cell.com/action/showPdf?pii=S0092-8674%2821%2900706-6). Furthermore, it has been shown that antibodies produced by memory B cells that are elicited after vaccination are of lower potency compared to antibodies elicited by natural infection (https://www.biorxiv.org/content/10.1101/2021.07.29.454333v1.full.pdf ).

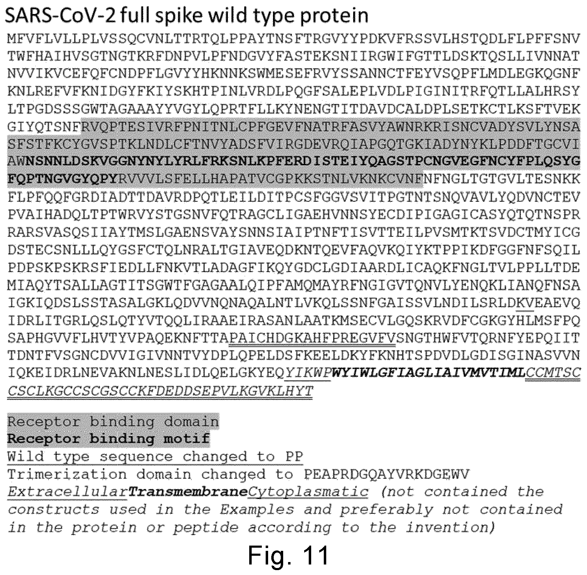

Furthermore, is has been found that newly emerging virus variants may have increased affinities for their receptors (Fig. 11). Therefore, the inventors of the present invention conclude that future vaccines that will be based on these higher affine variants might be more susceptible for a masking by sACE2. To achieve the same level of protection against novel variants, higher affinity antibodies are needed. Hence, a vaccine that avoids masking of soluble receptors would be of even higher relevance if ongoing viral evolution leads to the emergence of higher affinity virus strains.

In view of the above, there is an urgent need for improved vaccines, which overcome the above mentioned problems. It is the key object of the present invention to provide a method for obtaining such vaccines.

The present invention is based on the surprising finding that all the above mentioned objects can be solved by using a receptor-binding domain of a coronavirus for immunization that has a reduced binding strength to its receptor.

The present invention is further based on the surprising finding that all the above mentioned objects can be solved by using a mutated receptor-binding domain of a coronavirus having one or more amino acid substitutions.

Furthermore, the present invention is further based on the surprising finding that all the above mentioned objects can be solved by using a combination of mutated receptor-binding domains of a coronavirus having each a different amino acid substitution.

The present invention is further based on the surprising finding that all the above mentioned objects can be solved by using a method to design and/or obtain receptor-binding domains of a virus by screening for receptor-binding domains having a reduced binding strength to its receptor.

Therefore, the present invention provides a mutant receptor-binding domain (mRBD) of a coronavirus (CORONA-mRBD) or a fragment thereof having a reduced binding strength to the RBD-receptor of the coronavirus (CORONA-RBD-receptor) compared to a wild type receptor-binding domain of the coronavirus (CORONA-wtRBD).

The inventors of the present invention could show that sACE2 levels in some individuals are elevated and developed assays to properly assess the concentration in blood. Therefore, people with elevated sACE2 are at risk of developing a less protective immune response. Individuals with increased release of sACE2 could be identified and benefit from the improved vaccine design described above. Additionally, the inventors could show that shortly after infection, sACE2 levels rise in severely sick patients. Thus, an improved vaccine may not only be relevant before the infection but especially useful when given shortly after infection to promote the development of protective antibodies.

Hence, it is an object of the present invention to provide a mutant receptor-binding domain (mRBD) of a coronavirus (CORONAmRBD) which allows to elicit a highly efficient immune response against the coronavirus in a subject also in the presence of high levels of soluble receptors and/or against coronavirus mutants having an increased masking effect. It is a further object of the present invention to provide a method to obtain such mutant receptor-binding domain (mRBD) in a timely and efficient manner to prevent a virus, particularly a newly emerging virus variant, from spreading and to protect as many individuals as possible, including individuals in which conventional vaccinations/immunizations and/or natural infections do not confer sufficient immunity.

Collectively, the inventors of the present invention have found in both, human and mice, that soluble virus receptors, particularly soluble coronavirus receptors, through binding to their antigen mediate masking of crucial epitopes thereby impairing the protective antibody response. This mechanism of epitope masking was so far only described for antibodies but not for viral receptors.

The inventors of the present invention could show that elevated levels of sACE2 in the blood are associated with severe COVID-19 disease. Furthermore, patients with high levels of sACE2 showed significantly lower antibody titers specific for the receptor binding domain (RBD) of the SARS-CoV-2 spike protein. Consequently, these patients also showed a significantly reduced potential to block binding of the SARS-CoV-2 spike proteins to ACE2.

Particularly, the inventors of the present invention have shown for the first time and provided mechanistic evidence that soluble receptors interfere with protective immune responses.

Furthermore, the inventors of the present invention could show in mice, that sACE2 significantly reduces the generation of B cells producing RBD specific antibodies that block the virus from binding to its receptor ACE2 (Fig. 1). Analysis of the murine sera confirmed less efficient antibody responses to immunizations.

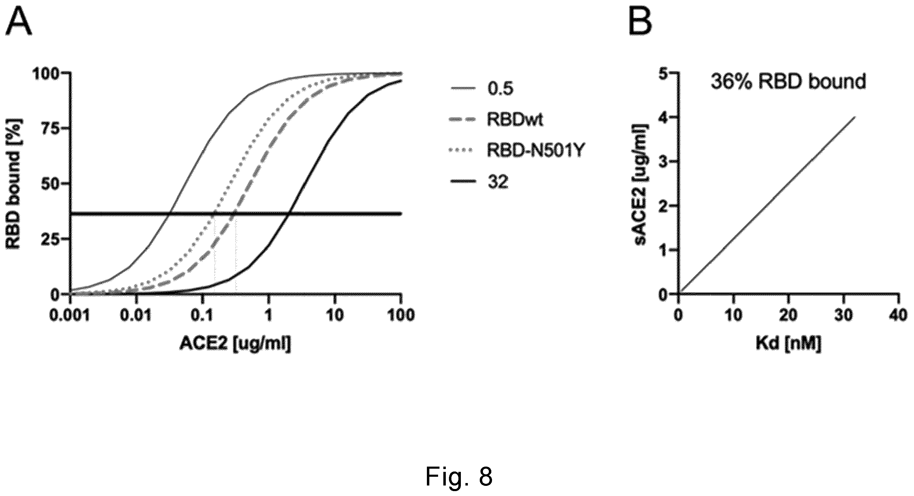

Moreover, the inventors of the present invention could show by *in silico* calculations, that mutations such as the SARS-CoV-2 variant B.1.1.7 and 501.V2 that emerged in UK and South Africa, respectively, would increase the masking effect (Fig. 8).

The present invention provides a mutant spike protein of the coronavirus (CORONA-mSpike) or a fragment thereof comprising the CORONA-mRBD or the fragment thereof according to any of claims 1 to 6.

Furthermore, the present invention provides a polypeptide or protein comprising the CORONA-mRBD or the fragment thereof according to the present invention or the CORONA-mSpike or the fragment thereof according to the present invention.

Furthermore, the present invention provides a nucleic acid comprising a nucleotide sequence encoding for:

- the CORONA-mRBD or the fragment thereof according to the present invention;

- the CORONA-mSpike or the fragment thereof according to the present invention; or

- the polypeptide or protein according to the present invention.

Furthermore, the present invention provides a vaccine composition comprising as an active ingredient:

- one or more CORONA-mRBDs or the fragments thereof according to the present invention; and/or

- one or more CORONA-mSpikes or the fragments thereof according to the present invention; and/or

- one or more polypeptides or proteins according to the present invention; and/or

- one or more nucleic acids according to the present invention.

Furthermore, the present invention provides:

one or more CORONA-mRBDs or the fragments thereof according to the present invention; and/or

one or more CORONA-mSpikes or the fragmentss according to the present invention; and/or

one or more polypeptides or proteins according to the present invention; and/or

one or more nucleic acids according to the present invention; and/or the vaccine composition according to the present invention,

for use in the prevention and/or treatment of diseases caused by coronaviruses in a subject.

Furthermore, the present invention relates to a method for designing and/or obtaining an active ingredient for a vaccine composition comprising the steps of:

(i) providing a mutant receptor binding domain of a virus (VIRUS-mRBD) or a fragment thereof, comprising one or more mutations in a wildtype receptor binding domain of the virus (VIRUS-wtRBD);

(ii) determining, whether the VIRUS-mRBD or the fragment thereof exhibits:

a) a reduced binding strength to a receptor of the receptor binding domain (RBD-receptor) of the virus (VIRUS-RBD-receptor) compared to the VIRUS-wtRBD; and

(iii) selecting the VIRUS-mRBD or the fragment thereof when a) is fulfilled as the active ingredient.

Furthermore, the present invention provides a VIRUS-mRBD or fragment thereof obtained by the method for designing and/or obtaining an active ingredient for a vaccine composition according to the present invention as the active ingredient.

### Mutant receptor-binding domain of a coronavirus (CORONA-mRBD)

The present invention provides a mutant receptor-binding domain (mRBD) of a coronavirus (CORONA-mRBD) or a fragment thereof.

Particularly, the present invention provides a CORONA-mRBD or a fragment thereof having a reduced binding strength to a RBD-receptor of the coronavirus (CORONA-RBD-receptor) compared to a wild type receptor-binding domain of the coronavirus (CORONA-wtRBD).

Preferably, the CORONA-mRBD or the fragment thereof is a mutant receptor-binding domain (mRBD) of a coronavirus spike protein (CORONA-mRBD) or a fragment thereof.

Preferably, the CORONA-mRBD or the fragment thereof is a peptide or protein, preferably a protein.

Preferably, the CORONA-mRBD or the fragment thereof obtained by the method for designing and/or obtaining an active ingredient for a vaccine composition according to the present invention as the active ingredient, which is described below.

Preferably, the CORONA-RBD-receptor is a soluble and/or membrane-bound form of the CORONA-RBD-receptor, preferably the soluble form of the CORONA-RBD-receptor (soluble CORONA-RBD-receptor). The soluble CORONA-RBD-receptor may comprise an active and an inactive form of the soluble CORONA-RBD-receptor.

The binding strength of the CORONA-mRBD or the fragment thereof and of the CORONA-wtRBD to the CORONA-RBD-receptor can be measured by any method known to the person skilled in the art. For instance, the binding strength can be determined by flow cytometry analysis, e.g. if cells expressing the CORONA-mRBD or CORONA-wtRBD on the cell surface are stained with the CORONA-RBD-receptor, directly or indirectly labelled with a fluorophore, or *vice versa* if cells expressing the CORONA-RBD-receptor on the cell surface are stained with a soluble CORONA-mRBD or soluble CORONA-wtRBD, directly or indirectly labelled with a fluorophore, and counting positively stained cells, or by determining the Kd (dissociation constant) or the EC50 (effective concentration with 50% of maximal binding) of the CORONA-wtRBD or the CORONA-mRBD, respectively, to the CORONA-RBD-receptor by using an ELISA. The reduced binding strength is then obtained by comparing the binding strength of the CORONA-wtRBD to the CORONA-RBD-receptor with the binding strength of the CORONA-mRBD to the CORONA-RBD-receptor.

Preferably, the binding strength is measured by determining the EC50 or the Kd, wherein a higher EC50 or Kd indicates a lower binding strength, or by flow cytometry analysis, wherein a reduction in the counts of positively stained cells indicates a lower binding strength. Consequently, the reduced binding strength is preferably determined by comparing the EC50, Kd and/or the counts of positively stained cells resulting from the binding between the CORONA-wtRBD and the CORONA-RBD-receptor to the EC50, Kd and/or the counts of positively stained cells resulting from the binding between the CORONA-mRBD and the CORONA-RBD-receptor, respectively. An example for determining the EC50 based on ACE2 as the CORONA-RBD-receptor, SARS-CoV-2 as the coronavirus and correspondingly the wildtype and mutated RBD of SARS-CoV-2 (SARS-wtRBD and SARS-mRBD) is provided in example 4. Another example for determining the EC50 is based on DPP4 as the CORONA-RBD-receptor, MERS-CoV as the coronavirus and the wildtype and mutated RBD of MERS-CoV (MERS-wtRBD and MERS-mRBD) is provided in example 7.

In case the reduced binding strength is determined by determining the EC50 or Kd, a reduced binding strength of the CORONA-mRBD to the CORONA-RBD-receptor is preferably indicated by an increase in the EC50 and/or Kd of 1.5-fold or more, more preferably of 2-fold or more, even more preferably of 10-fold or more, still more preferably of 100-fold or more, still even more preferably of 500-fold or more and most preferably of 1000-fold or more. In order to obtain the best effect in vaccination in case the CORONA-mRBD is used a vaccine, the binding strength to the CORONA-RBD-receptor should be reduced as much as possible.

Hence, preferably, there is no upper limit for the reduction in the binding strength to the CORONA-RBD-receptor. Hence, preferably there is no upper limit for the increase in the EC50 or Kd. Nevertheless, an upper limit for an increase in the EC50 or Kd may be 500.000-fold or less, more preferably 100.000-fold or less, still more preferably of 10.000-fold or less. An example for determining the EC50 based on ACE2 as the CORONA-RBD-receptor, SARS-CoV-2 as the coronavirus and the wildtype and mutated RBD of SARS-CoV-2 (SARS-wtRBD and SARS-mRBD) is provided in example 4. Another example for determining the EC50 is based on DPP4 as the CORONA-RBD-receptor, MERS-CoV as the coronavirus and the wildtype and mutated RBD of MERS-CoV (MERS-wtRBD and MERS-mRBD) is provided in example 7.

In case the reduced binding strength is determined by flow cytometry analysis, a reduced binding strength of the CORONA-mRBD to the CORONA-RBD-receptor is preferably indicated by a reduction in the counts of positively stained cells of 50% or more, more preferably of 80% or more, even more preferably of 90% or more, still more preferably of 95% or more and still even more preferably of 98% or more. In order to obtain the best effect in vaccination in case the CORONA-mRBD is used as a vaccine, the binding strength to the CORONA-RBD-receptor should be reduced as much as possible. Hence, preferably, there is no upper limit for the reduction in the binding strength to the CORONA-RBD-receptor. Hence, preferably there is no upper limit for the reduction in positively stained cells and the reduction may be even 100%. Nevertheless, an upper limit for the reduction in the positively stained cells may be 100 % or less, more preferably 99% or less.

Furthermore, the CORONA-mRBD or the fragment thereof preferably exhibits a binding to anti-CORONA-wtRBD neutralizing antibodies (anti-CORONA-wtRBD-nABs). That is, a binding strength to anti-CORONA-wtRBD neutralizing antibodies (anti-CORONA-wtRBD-nABs) is preferably maintained, i.e. in the range of an only moderate reduction in the binding strength up to an increase of the binding strength when compared with the CORONA-wtRBD.

The anti-CORONA-wtRBD-nABs may belong to different antibody classes like Class I, Class II, Class III and/or Class IV, and/or may be specific for different epitopes in the CORONA-wtRBD. The anti-CORONA-wtRBD-nABs may be monoclonal and/or polyclonal antibodies reactive against the CORONA-wtRBD. Monoclonal anti-CORONA-wtRBD-nABs may be obtained recombinantly. Polyclonal anti-CORONA-wtRBD-nABs may be obtained from blood plasma or blood serum derived from an immune subject like a convalescent subject, which has been passed through a coronavirus infection, and/or an immunized subject, e.g. immunized by vaccination, wherein such immune subjects are preferably immunologically reactive to the CORONA-wtRBD. The subject preferably is a vertebrate, more preferably an immune mammal, more preferably an ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human. Still more preferably the immune subject is a mouse, rat, rabbit, hamster, goat, donkey, horse, dog, cat, sheep or human, etc., most preferably a human.

The binding strength of the CORONA-mRBD or the fragment thereof and of the CORONA-wtRBD to anti-CORONA-wtRBD-nABs can be measured by any method known to the person skilled in the art. For instance, the binding strength can be determined by flow cytometry analysis, e.g. if cells expressing the CORONA-mRBD or CORONA-wtRBD on the cell surface are stained with an monoclonal anti-CORONA-wtRBD-nAB, directly or indirectly labelled with a fluorophore, or *vice versa* if cells expressing an monoclonal anti-CORONA-wtRBD-nAB on the cell surface are stained with a soluble CORONA-mRBD or soluble CORONA-wtRBD, directly or indirectly labelled with a fluorophore, and counting positively stained cells, or by determining the Kd (dissociation constant) or the EC50 (effective concentration with 50% of maximal binding) of the CORONA-wtRBD and the CORONA-mRBD to the monoclonal anti-CORONA-wtRBD-nAB by using an ELISA, or the ED50 (effective dilution at which 50% of serum antibodies is bound) of polyclonal antibodies of blood plasma or blood serum derived from an immune subject like a convalescent subject, which has been passed through a coronavirus infection, and/or an immunized subject, e.g. immunized by vaccination, wherein such immune subjects are preferably immunologically reactive to the CORONA-wtRBD. The immune subject is defined as above. An example for determining the EC50, Kd and the ED50 based on anti-SARS-CoV-2-wtRBD neutralizing antibodies (anti-SARS-wtRBD-nABs) as the anti-CORONA-wtRBD-nABs, SARS-CoV-2 as the coronavirus and the wildtype and mutated RBD of SARS-CoV-2 (SARS-wtRBD and SARS-mRBD) is provided in example 4. An example for determining the EC50 based on anti-SARS-CoV-2-wtRBD neutralizing antibodies (anti-SARS-wtRBD-nABs) as the anti-CORONA-wtRBD-nABs, SARS-CoV-2 as the coronavirus and the wildtype and mutated RBD of SARS-CoV-2 (SARS-wtRBD and SARS-mRBD) is provided in example 4. Another example for determining the EC50 based on anti-MERS-CoV-wtRBD neutralizing antibodies (anti-MERS-wtRBD-nABs) as the anti-CORONA-wtRBD-nABs, MERS-CoV as the coronavirus and the wildtype and mutated RBD of MERS-CoV (MERS-wtRBD and MERS-mRBD) is provided in example 7.

In case of determining the binding strength of the anti-CORONA-wtRBD-nABs by flow cytometry analysis, the EC50 and/or the Kd, the binding strength preferably is the average binding strength of several different monoclonal anti-CORONA-wtRBD-nABs, preferably of four or more different arbitrarily selected monoclonal anti-CORONA-wtRBD-nABs. When exemplified again on Example 4 for SARS-CoV-2 and anti-SARS-wtRBD-nABs, the average EC50 of anti-SARS-wtRBD-nABs CC12.1, P2C-1F11, REGN10933 and S309 in view of SARS-wtRBD or SARS-mRBD is determined. When exemplified again on Example 4 for MERS-CoV and anti-MERS-wtRBD-nABs, the average EC50 of anti-MERS-wtRBD-nABs 4C2h, D12, LCA60, and MERS4V2 is determined.

The maintained binding strength, i.e. the binding strength in the range of an only moderate reduction in the binding strength up to an increase in the binding strength, is then obtained by comparing the binding strength or the average binding strength of the CORONA-wtRBD to the anti-CORONA-wtRBD-nABs with the binding strength or the average binding strength, respectively, of the CORONA-mRBD to the anti-CORONA-wtRBD-nABs.

Preferably, the binding strength is measured by determining the EC50 or the Kd, wherein a higher EC50 or Kd indicates a lower binding strength, or by flow cytometry analysis or determining the ED50, wherein a reduction in the counts of positively stained cells or a lower ED50, respectively, indicate a lower binding strength. Consequently, the maintained, i.e. whether the binding strength is in the range of an only moderate reduction in the binding strength up to an increase in the binding strength, is preferably determined by comparing the EC50, Kd, the counts of positively stained cells and/or the ED50 resulting from the binding between the CORONA-wtRBD and the anti-CORONA-wtRBD-nABs to the EC50, Kd, the counts of positively stained cells and/or the ED50 resulting from the binding between the CORONA-mRBD and the anti-CORONA-wtRBD-nABs, respectively.

In the case of determining the binding strength by determining the EC50 or Kd, an only moderate reduction in the binding strength of the CORONA-mRBD to the anti-CORONA-wtRBD-nABs is preferably indicated by an increase in the EC50 and/or Kd of 3-fold or less, even more preferably of 2-fold or less, still more preferably of 1.5-fold or less and still even more preferably 1.2-fold or less. In the case of determining the binding strength by flow cytometry analysis or determining the ED50, an only moderate reduction in the binding strength of the CORONA-mRBD to the RBDanti-CORONA-wtRBD-nABs is preferably indicated by a reduction in the counts of positively of stained cells and/or in the ED50 of 50% or more, more preferably of 80% or more and even more preferably of 90% or more.

As already stated above, when determining the binding strength by the EC50, the Kd or flow cytometric analysis, the binding strength is preferably an average binding strength as defined above. Consequently, the only moderate reduction in the binding strength as defined above is preferably an only moderate reduction in the average binding strength as defined above.

In order to obtain the best effect in vaccination in case the CORONA-mRBD is used a vaccine, the binding strength to the anti-CORONA-wtRBD-nABs should be as high as possible. Hence, preferably, the reduction in the binding strength to the anti-CORONA-wtRBD-nABs should be as moderate as possible and preferably the binding strength of the anti-CORONA-wtRBD-nABs to the CORONA-mRBD is preferably not reduced or even increased, i.e. EC50 or Kd are not increased and/or the counts of positively of stained cells or the ED50 are not reduced.

Consequently, in the case of determining the binding strength by determining the EC50 or Kd, an increase in the binding strength of the CORONA-mRBD to the anti-CORONA-wtRBD-nABs is preferably indicated by an reduction in the EC50 and/or Kd of 10 % or more, more preferably of 25% or more, still even more preferably of 50% or more, still even more preferably of 80% or more and most preferably 90% or more. As stated above, in order to obtain the best effect in vaccination in case the CORONA-mRBD is used as a vaccine, the binding strength to the anti-CORONA-wtRBD-nABs should be as high as possible. Hence, preferably, there is no upper limit for the increase in the binding strength to the anti-CORONA-wtRBD-nABs. Hence, preferably there is no upper limit for the reduction in the EC50 or Kd and the reduction may be even 100%. Nevertheless, an upper limit for the reduction in the EC50 and/or Kd may be 100 % or less, more preferably 99% or less.

In the case of determining the binding strength by flow cytometry analysis or ED50, an increased binding strength of the CORONA-mRBD to the anti-CORONA-wtRBD-nABs is preferably indicated by an increase in the counts of positively of stained cells and/or in the ED50, respectively, by 1.1-fold or more, more preferably by 1.2-fold or more, even more preferably by 1.5-fold or more, still even more preferably by 2-fold or more. As stated above, in order to obtain the best effect in vaccination in case the CORONA-mRBD is used as a vaccine, the binding strength to the anti-CORONA-wtRBD-nABs should be as high as possible. Hence, preferably, there is no upper limit for the increase in the binding strength to the anti-CORONA-wtRBD-nABs. Hence, preferably there is no upper limit for the increase in positively stained cells and/or the ED50. Nevertheless, an upper limit for an increase in positively stained cells and/or the ED50 may be 10.000-fold or less, more preferably of 1.000-fold or less and still more preferably 100-fold or less.

As already stated above, when determining the binding strength by the EC50, the Kd or flow cytometric analysis, the binding strength is preferably an average binding strength as defined above. Consequently, the increased binding strength as defined above is preferably an increase in the average binding strength as defined above.

Preferably, the coronavirus is of the family *coronaviridae,* more preferably of the genus *betacoronavirus.*

Even more preferably:

(A) the coronavirus is SARS-CoV-2 (severe acute respiratory syndrome coronavirus type 2),

the CORONA-mRBD is a mutant receptor-binding domain of SARS-CoV-2 (SARS-mRBD) or a fragment thereof,

the wild type receptor-binding domain is SARS-wtRBD (wildtype receptor binding domain of SARS-CoV-2)

the CORONA-RBD-receptor is ACE2 (angiotensin-converting enzyme 2), and

preferably, the anti-SARS-wtRBD neutralizing antibodies (anti-CORONA-wtRBD-nABs) are anti-SARS-wtRBD-nABs and/or the blood plasma or blood serum are as defined above derived from an immune subject like a convalescent subject, wherein such immune subjects are preferably immunologically reactive to the SARS-wtRBD; or

(B) the coronavirus is MERS-CoV (middle east respiratory syndrome coronavirus),

the CORONA-mRBD is a mutant receptor-binding domain of MERS-CoV (MERS-mRBD) or a fragment thereof,

the wild type receptor-binding domain is MERS-wtRBD (wildtype receptor binding domain of MERS-CoV),

the CORONA-RBD-receptor is DPP4 (dipeptidylpeptidase 4), and

preferably, the anti-MERS-wtRBD neutralizing antibodies (anti-CORONA-wtRBD-nABs) are anti-MERS-wtRBD-nABs and/or the blood plasma or blood serum are as defined above derived from an immune subject like a convalescent subject, wherein such immune subjects are preferably immunologically reactive to the MERS-wtRBD.

Consequently, preferably, the present invention relates to (A) a SARS-mRBD or a fragment thereof having a reduced binding strength to ACE2 compared to a SARS-wtRBD and/or the present invention relates to (B) a MERS-mRBD or a fragment thereof having a reduced binding strength to DDP4 compared to a MERS-wtRBD.

*(A) Mutant receptor binding domain of SARS-CoV-2 (SARS-mRBD)*

As stated above, the present invention preferably relates to (A) a SARS-mRBD or a fragment thereof having a reduced binding strength to ACE2 compared to a SARS-wtRBD.

Preferably, the SARS-mRBD (mutant receptor-binding domain of SARS-CoV-2) is a mutant receptor-binding domain (mRBD) of a SARS-CoV-2 spike protein (SARS-mRBD) or a fragment thereof.

Preferably, the SARS-mRBD or the fragment thereof is a peptide or protein, preferably a protein.

Preferably, the SARS-mRBD or the fragment thereof obtained by the method for designing and/or obtaining an active ingredient for a vaccine composition according to the present invention as the active ingredient, which is described below.

Preferably, the ACE2 is a soluble and/or membrane-bound form of the ACE2, preferably the soluble form of the ACE2 (sACE2). The sACE2 may comprise an active and an inactive form of the sACE2.

The binding strength of the SARS-mRBD or the fragment thereof and of the SARS-wtRBD to ACE2 can be measured by any method known to the person skilled in the art. For instance, the binding strength can be determined by flow cytometry analysis, e.g. if cells expressing the SARS-mRBD or SARS-wtRBD on the cell surface are stained with ACE2, directly or indirectly labelled with a fluorophore, or *vice versa* if cells expressing ACE2 on the cell surface are stained with a soluble SARS-mRBD or soluble SARS-wtRBD, directly or indirectly labelled with a fluorophore, and counting positively stained cells, or by determining the Kd (dissociation constant) or the EC50 (effective concentration with 50% of maximal binding) of the SARS-wtRBD or the SARS-mRBD, respectively, to ACE2 by using an ELISA. The reduced binding strength is then obtained by comparing the binding strength of the SARS-wtRBD to ACE2 with the binding strength of the SARS-mRBD to ACE2.

Preferably, the binding strength is measured by determining the EC50 or the Kd, wherein a higher EC50 or Kd indicates a lower binding strength, or by flow cytometry analysis, wherein a reduction in the counts of positively stained cells indicates a lower binding strength. Consequently, the reduced binding strength is preferably determined by comparing the EC50, Kd and/or the counts of positively stained cells resulting from the binding between the SARS-wtRBD and ACE2 to the EC50, Kd and/or the counts of positively stained cells resulting from the binding between the SARS-mRBD and ACE2, respectively. Preferably, the EC50 and Kd of the SARS-wtRBD or the SARS-mRBD to ACE2 is determined as described in example 4.

In case the reduced binding strength is determined by determining the EC50 or Kd, a reduced binding strength of the SARS-mRBD to ACE2 is preferably indicated by an increase in the EC50 and/or Kd of 1.5-fold or more, more preferably of 2-fold or more, even more preferably of 10-fold or more, still more preferably of 100-fold or more, still even more preferably of 500-fold or more and most preferably of 1000-fold or more. In order to obtain the best effect in vaccination in case the SARS-mRBD is used a vaccine, the binding strength to ACE2 should be reduced as much as possible. Hence, preferably, there is no upper limit for the reduction in the binding strength to ACE2. Hence, preferably there is no upper limit for the increase in the EC50 or Kd. Nevertheless, an upper limit for an increase in the EC50 or Kd may be 500.000-fold or less, more preferably 100.000-fold or less, still more preferably of 10.000-fold or less. Preferably, the EC50 and Kd of the SARS-wtRBD or the SARS-mRBD to ACE2 is determined as described in example 4.

In case the reduced binding strength is determined by flow cytometry analysis, a reduced binding strength of the SARS-mRBD to ACE2 is preferably indicated by a reduction in the counts of positively stained cells of 50% or more, more preferably of 80% or more, even more preferably of 90% or more, still more preferably of 95% or more and still even more preferably of 98% or more. In order to obtain the best effect in vaccination in case the SARS-mRBD is used as a vaccine, the binding strength to ACE2 should be reduced as much as possible. Hence, preferably, there is no upper limit for the reduction in the binding strength to ACE2. Hence, preferably there is no upper limit for the reduction in positively stained cells and the reduction may be even 100%. Nevertheless, an upper limit for the reduction in the positively stained cells may be 100 % or less, more preferably 99% or less.

Furthermore, the SARS-mRBD or the fragment thereof preferably exhibits a binding to anti-SARS-wtRBD neutralizing antibodies (anti-SARS-wtRBD-nABs). That is, a binding strength to anti-SARS-wtRBD neutralizing antibodies (anti-SARS-wtRBD-nABs) is preferably maintained, i.e. in the range of an only moderate reduction in the binding strength up to an increase of the binding strength when compared with the SARS-wtRBD.

The anti-SARS-wtRBD-nABs may belong to different antibody classes like Class I, Class II, Class III and/or Class IV, and/or may be specific for different epitopes in the SARS-wtRBD. The anti-SARS-wtRBD-nABs may be monoclonal and/or polyclonal antibodies reactive against the SARS-wtRBD. Monoclonal anti-SARS-wtRBD-nABs may be obtained recombinantly. Polyclonal anti-SARS-wtRBD-nABs may be obtained from blood plasma or blood serum derived from an immune subject like a convalescent subject, which has been passed through a coronavirus infection, and/or an immunized subject, e.g. immunized by vaccination, wherein such immune subjects are preferably immunologically reactive to the SARS-wtRBD. The subject preferably is a vertebrate, more preferably an immune mammal, more preferably an ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human. Still more preferably the immune subject is a mouse, rat, rabbit, hamster, goat, donkey, horse, dog, cat, sheep or human, etc., most preferably a human.

The binding strength of the SARS-mRBD or the fragment thereof and of the SARS-wtRBD to anti-SARS-wtRBD-nABs can be measured by any method known to the person skilled in the art. For instance, the binding strength can be determined by flow cytometry analysis, e.g. if cells expressing the SARS-mRBD or SARS-wtRBD on the cell surface are stained with an monoclonal anti-SARS-wtRBD-nAB, directly or indirectly labelled with a fluorophore, or *vice versa* if cells expressing an monoclonal anti-SARS-wtRBD-nAB on the cell surface are stained with a soluble SARS-mRBD or soluble SARS-wtRBD, directly or indirectly labelled with a fluorophore, and counting positively stained cells, or by determining the Kd (dissociation constant) or the EC50 (effective concentration with 50% of maximal binding) of the SARS-wtRBD and the SARS-mRBD to the monoclonal anti-SARS-wtRBD-nAB by using an ELISA, or the ED50 (effective dilution at which 50% of serum antibodies is bound) of polyclonal antibodies of blood plasma or blood serum derived from an immune subject like a convalescent subject, which has been passed through a coronavirus infection, and/or an immunized subject, e.g. immunized by vaccination, wherein such immune subjects are preferably immunologically reactive to the SARS-wtRBD. The immune subject is defined as above. Preferably, the EC50, Kd and the ED50 the SARS-wtRBD or SARS-mRBD to the anti-SARS-wtRBD-nABs is determined as described in example 4.

In case of determining the binding strength of the anti-SARS-wtRBD-nABs by flow cytometry analysis, the EC50 and/or the Kd, the binding strength preferably is the average binding strength of several different monoclonal anti-SARS-wtRBD-nABs, preferably of four or more different arbitrarily selected monoclonal anti-SARS-wtRBD-nABs. Preferably, the four different monoclonal anti-SARS-wtRBD-nABs are CC12.1, P2C-1F11, REGN10933 and S309 as described in examples 4 and the average binding strength is the average EC50 or average Kd of CC12.1, P2C-1F11, REGN10933 and S309, respectively.

The maintained binding strength, i.e. the binding strength in the range of an only moderate reduction in the binding strength up to an increase in the binding strength, is then obtained by comparing the binding strength or the average binding strength of the SARS-wtRBD to the anti-SARS-wtRBD-nABs with the binding strength or the average binding strength, respectively, of the SARS-mRBD to the anti-SARS-wtRBD-nABs.

Preferably, the binding strength is measured by determining the EC50 or the Kd, wherein a higher EC50 or Kd indicates a lower binding strength, or by flow cytometry analysis or determining the ED50, wherein a reduction in the counts of positively stained cells or a lower ED50, respectively, indicate a lower binding strength. Consequently, the maintained, i.e. whether the binding strength is in the range of an only moderate reduction in the binding strength up to an increase in the binding strength, is preferably determined by comparing the EC50, Kd, the counts of positively stained cells and/or the ED50 resulting from the binding between the SARS-wtRBD and the anti-SARS-wtRBD-nABs to the EC50, Kd, the counts of positively stained cells and/or the ED50 resulting from the binding between the SARS-mRBD and the anti-SARS-wtRBD-nABs, respectively.

In the case of determining the binding strength by determining the EC50 or Kd, an only moderate reduction in the binding strength of the SARS-mRBD to the anti-SARS-wtRBD-nABs is preferably indicated by an increase in the EC50 and/or Kd of 3-fold or less, even more preferably of 2-fold or less, still more preferably of 1.5-fold or less and still even more preferably 1.2-fold or less. In the case of determining the binding strength by flow cytometry analysis or determining the ED50, an only moderate reduction in the binding strength of the SARS-mRBD to the RBDanti-SARS-wtRBD-nABs is preferably indicated by a reduction in the counts of positively of stained cells and/or in the ED50 of 50% or more, more preferably of 80% or more and even more preferably of 90% or more.

As already stated above, when determining the binding strength by the EC50, the Kd or flow cytometric analysis, the binding strength is preferably an average binding strength as defined above. Consequently, the only moderate reduction in the binding strength as defined above is preferably an only moderate reduction in the average binding strength as defined above.

In order to obtain the best effect in vaccination in case the SARS-mRBD is used a vaccine, the binding strength to the anti-SARS-wtRBD-nABs should be as high as possible. Hence, preferably, the reduction in the binding strength to the anti-SARS-wtRBD-nABs should be as moderate as possible and preferably the binding strength of the anti-SARS-wtRBD-nABs to the SARS-mRBD is preferably not reduced or even increased, i.e. EC50 or Kd are not increased and/or the counts of positively of stained cells or the ED50 are not reduced.

Consequently, in the case of determining the binding strength by determining the EC50 or Kd, an increase in the binding strength of the SARS-mRBD to the anti-SARS-wtRBD-nABs is preferably indicated by an reduction in the EC50 and/or Kd of 10 % or more, more preferably of 25% or more, still even more preferably of 50% or more, still even more preferably of 80% or more and most preferably 90% or more. As stated above, in order to obtain the best effect in vaccination in case the SARS-mRBD is used as a vaccine, the binding strength to the anti-SARS-wtRBD-nABs should be as high as possible. Hence, preferably, there is no upper limit for the increase in the binding strength to the anti-SARS-wtRBD-nABs. Hence, preferably there is no upper limit for the reduction in the EC50 or Kd and the reduction may be even 100%. Nevertheless, an upper limit for the reduction in the EC50 and/or Kd may be 100 % or less, more preferably 99% or less.

In the case of determining the binding strength by flow cytometry analysis or ED50, an increased binding strength of the SARS-mRBD to the anti-SARS-wtRBD-nABs is preferably indicated by an increase in the counts of positively of stained cells and/or in the ED50, respectively, by 1.1-fold or more, more preferably by 1.2-fold or more, even more preferably by 1.5-fold or more, still even more preferably by 2-fold or more. As stated above, in order to obtain the best effect in vaccination in case the SARS-mRBD is used as a vaccine, the binding strength to the anti-SARS-wtRBD-nABs should be as high as possible. Hence, preferably, there is no upper limit for the increase in the binding strength to the anti-SARS-wtRBD-nABs. Hence, preferably there is no upper limit for the increase in positively stained cells and/or the ED50. Nevertheless, an upper limit for an increase in positively stained cells and/or the ED50 may be 10.000-fold or less, more preferably of 1.000-fold or less and still more preferably 100-fold or less.

As already stated above, when determining the binding strength by the EC50, the Kd or flow cytometric analysis, the binding strength is preferably an average binding strength as defined above. Consequently, the increased binding strength as defined above is preferably an increase in the average binding strength as defined above.

Preferably, the present invention relates to a SARS-mRBD or a fragment thereof comprising, more preferably consisting of,

an amino acid sequence or a fragment thereof comprising one or more, preferably one to three, more preferably one or two, even more preferably one, substitutions of amino acid residues at positions selected from G184, Y187, L137, Y171, F138, Q180, F168, Y131 or S55 of the SARS-wtRBD of SEQ ID NO: 1, wherein the SARS-mRBD or the fragment thereof has, except for the substitutions, an amino acid sequence identity of 85% or more to SEQ ID NO: 1.

Preferably, the SARS-mRBD or the fragment thereof has, except for the substitutions, an amino acid sequence identity of 90% or more, more preferably of 95% or more, even more preferably of 97% or more, still even more preferably 98% or more and most preferably of 100% to SEQ ID NO: 1.

The SARS-wtRBD of SEQ ID NO: 1 is the SARS-wtRBD of the Wuhan variant.

The amino acid sequence identity of the SARS-mRBD to the SARS-wtRBD of SEQ ID NO: 1 as defined above should be low enough to still cover SARS-RBDs of SARS-CoV-2-variants other than the Wuhan-variant, SEQ ID NO: 1, in order to allow adaptions of the SARS-mRBD or the fragment thereof thereto or to combinations of the mutations, like substitutions, deletions and insertions, comprised in such variants when compared with SEQ ID NO: 1. Examples for such SARS-CoV-2-variants are:

B1.351 (Beta) comprising substitutions K99N, E166K and N183Y;

B.1.1.7 (Alpha; 501Y.V1) comprising the substitution N183Y;

P.1 (Gamma; 501Y.V3) comprising substitutions K99T, E166K and N183Y; and/or

B.1.617.2 (Delta) comprising substitutions L134R and T160K,

wherein the position of each substitution is indicated in view of the SARS-wtRBD of SEQ ID NO: 1.

Preferably, the one or more substitutions of amino acid residues are one to three, preferably one or two, most preferably one, substitutions of the amino acid residues at positions selected from G184, L137, Y171 or Y187, more preferably selected from G184, L137 or Y187 of the SARS-wtRBD of SEQ ID NO: 1.

More preferably, the one or more substitutions of amino acid residues are one or both, preferably one, substitutions of the amino acid residues at positions selected from G184 or Y187 or from G184 or Y137 of the SARS-wtRBD of SEQ ID NO: 1.

Still more preferably, the one or more substitutions of amino acid residues are a substitution of the amino acid residue at the position G184 of the SARS-wtRBD of the SARS-wtRBD of SEQ ID NO: 1.

Preferably, the one or more substitutions of amino acid residues are one or more, preferably one to three, more preferably one or two, even more preferably one, substitutions of amino acid residues at positions selected from G184, Y187, L137, Y171, F138, Q180, F168, Y131 or S55 of the SARS-wtRBD of SEQ ID NO: 194, wherein the substitution of the amino acid residue at the position:

- G184 is selected from G184A, G184C, G184D, G184E, G184F, G184H, G184I, G184K, G184L, G184M, G184N, G184P, G184Q, G184R, G184S, G184T, G184V, G184W or G184Y;

- Y187 is selected from Y187A, Y187C, Y187D, Y187E, Y187G, Y187I, Y187K, Y187L, Y187M, Y187N, Y187Q, Y187R, Y187S, Y187T or Y187V;

- L137 is selected from L137D, L137E, L137K, L137R and L137Y;

- Y171 is selected from Y171A, Y171C, Y171E, Y171I, Y171K, Y171L, Y171M, Y171N, Y171P, Y171Q, Y171R, Y171S, Y171T or Y171V;

- F138 is selected from F138A, F138C, F138E, F138G, F138I, F138K, F138N, F138Q, F138R, F138S, F138T, F138W or F138Y;

- Q180 is selected from Q180C, Q180D, Q180I, Q180K, Q180L or Q180V;

- F168 is selected from F168C, F168D or F168E;

- Y131 is selected from Y131A, Y131C, Y131D, Y131E, Y131F, Y131G, Y131H, Y131I, Y131L, Y131M, Y131N, Y131P, Y131Q, Y131S, Y131T, Y131V or Y131W; and/or

- S55 is S55N.

More preferably, the one or more substitutions of amino acid residues are one to three, preferably one or two, more preferably one, substitutions of amino acid residues at positions selected from G184, L137, Y171 or Y187, more preferably from G184, L137, or Y187 of the SARS-wtRBD with SEQ ID NO: 1, wherein the substitution of the amino acid residue at the position:

- G184 is selected from G184R, G184D, G184Y, G184K G184S, G184P, G184E, G184A, G184V, G184N, G184Q, G184T, G184M, G184H or G184L;

- L137 is selected from L137R or L137E;

- Y187 is selected from Y187N or Y187Q; and

- Y171 is selected from Y171S or Y171T.

Even more preferably, the one or more substitutions of amino acid residues are one or both, still even more preferably one, substitutions of amino acid residues at positions selected from G184 or Y187 or from G184 or L137, more preferably from G184 or L137, of the SARS-wtRBD with SEQ ID NO: 1, wherein the substitution of the amino acid residue at the position:

- G184 is selected from G184R, G184D, G184Y, G184K G184S, G184P, G184E, G184A, G184V, G184N, G184Q, G184T, G184M, G184H or G184L, most preferably is G184R;

- L137 is L137R; and

- Y187 is Y187N.

Still even more preferably, the one or more substitutions of amino acid residues are one substitution of an amino acid residue at position G184 of the SARS-wtRBD with SEQ ID NO: 1, wherein the substitution is G184R, and/or the one or more substitutions of amino acid residues are one substitution of an amino acid residue at position L137 of the SARS-wtRBD with SEQ ID NO: 1, wherein the substitution is L137R, most preferably, the substitution is G184R
Preferably, the SARS-mRBD or the fragment thereof comprises, preferably consists of, an amino acid sequence selected from:
SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93 or SEQ ID NO: 94;

more preferably selected from:
SEQ ID NO: 15, SEQ ID NO: 17, SEQ ID NO: 5, SEQ ID NO: 20, SEQ ID NO: 9, SEQ ID NO: 16, SEQ ID NO: 13, SEQ ID NO: 2, SEQ ID NO: 3, SEQ ID NO: 18, SEQ ID NO: 12, SEQ ID NO: 14, SEQ ID NO: 11, SEQ ID NO: 7, SEQ ID NO: 10, SEQ ID NO: 55, SEQ ID NO: 53, SEQ ID NO: 30, SEQ ID NO: 31, SEQ ID NO: 92 or SEQ ID NO: 93;

even more preferably selected from:
SEQ ID NO: 15, SEQ ID NO: 17, SEQ ID NO: 5, SEQ ID NO: 20, SEQ ID NO: 9, SEQ ID NO: 16, SEQ ID NO: 13, SEQ ID NO: 2, SEQ ID NO: 3, SEQ ID NO: 18, SEQ ID NO: 12, SEQ ID NO: 14, SEQ ID NO: 11, SEQ ID NO: 7 or SEQ ID NO: 10; SEQ ID NO: 55 or SEQ ID NO: 30;

still more preferably selected from:
SEQ ID NO: 15, SEQ ID NO: 55 or SEQ ID NO: 30;

still even more preferably selected from:

SEQ ID NO: 15 or SEQ ID NO: 55; and

most preferably comprises, preferably consists of, the amino acid sequence of SEQ ID NO: 15.

*(B) Mutant receptor binding domain of MERS-CoV (MERS-mRBD)*

As stated above, the present invention further preferably relates to (B) a MERS-mRBD or a fragment thereof having a reduced binding strength to DDP4 compared to a MERS-wtRBD.

Preferably, the MERS-mRBD (mutant receptor-binding domain of MERS-CoV) is a mutant receptor-binding domain (mRBD) of a MERS-CoV spike protein (MERS-mRBD) or a fragment thereof.

Preferably, the MERS-mRBD or the fragment thereof is a peptide or protein, preferably a protein.

Preferably, the MERS-mRBD or the fragment thereof obtained by the method for designing and/or obtaining an active ingredient for a vaccine composition according to the present invention as the active ingredient, which is described below.

Preferably, the DPP4 is a soluble and/or membrane-bound form of the DPP4, preferably the soluble form of the DPP4 (sDPP4).

The binding strength of the MERS-mRBD or the fragment thereof and of the MERS-wtRBD to DPP4 can be measured by any method known to the person skilled in the art. For instance, the binding strength can be determined by flow cytometry analysis, e.g. if cells expressing the MERS-mRBD or MERS-wtRBD on the cell surface are stained with DPP4, directly or indirectly labelled with a fluorophore, or *vice versa* if cells expressing DPP4 on the cell surface are stained with a soluble MERS-mRBD or soluble MERS-wtRBD, directly or indirectly labelled with a fluorophore, and counting positively stained cells, or by determining the Kd (dissociation constant) or the EC50 (effective concentration with 50% of maximal binding) of the MERS-wtRBD or the MERS-mRBD, respectively, to DPP4 by using an ELISA. The reduced binding strength is then obtained by comparing the binding strength of the MERS-wtRBD to DPP4 with the binding strength of the MERS-mRBD to DPP4.

Preferably, the binding strength is measured by determining the EC50 or the Kd, wherein a higher EC50 or Kd indicates a lower binding strength, or by flow cytometry analysis, wherein a reduction in the counts of positively stained cells indicates a lower binding strength. Consequently, the reduced binding strength is preferably determined by comparing the EC50, Kd and/or the counts of positively stained cells resulting from the binding between the MERS-wtRBD and DPP4 to the EC50, Kd and/or the counts of positively stained cells resulting from the binding between the MERS-mRBD and DPP4, respectively. Preferably, the EC50 of the MERS-wtRBD or the MERS-mRBD to DPP4 is determined as described in example 7.

In case the reduced binding strength is determined by determining the EC50 or Kd, a reduced binding strength of the MERS-mRBD to DPP4 is preferably indicated by an increase in the EC50 and/or Kd of 1.5-fold or more, more preferably of 2-fold or more, even more preferably of 10-fold or more, still more preferably of 100-fold or more, still even more preferably of 500-fold or more and most preferably of 1000-fold or more. In order to obtain the best effect in vaccination in case the MERS-mRBD is used a vaccine, the binding strength to DPP4 should be reduced as much as possible. Hence, preferably, there is no upper limit for the reduction in the binding strength to DPP4. Hence, preferably there is no upper limit for the increase in the EC50 or Kd. Nevertheless, an upper limit for an increase in the EC50 or Kd may be 500.000-fold or less, more preferably 100.000-fold or less, still more preferably of 10.000-fold or less. Preferably, the EC50 of the MERS-wtRBD or the MERS-mRBD to DPP4 is determined as described in example 7.

In case the reduced binding strength is determined by flow cytometry analysis, a reduced binding strength of the MERS-mRBD to DPP4 is preferably indicated by a reduction in the counts of positively stained cells of 50% or more, more preferably of 80% or more, even more preferably of 90% or more, still more preferably of 95% or more and still even more preferably of 98% or more. In order to obtain the best effect in vaccination in case the MERS-mRBD is used as a vaccine, the binding strength to DPP4 should be reduced as much as possible. Hence, preferably, there is no upper limit for the reduction in the binding strength to DPP4. Hence, preferably there is no upper limit for the reduction in positively stained cells and the reduction may be even 100%. Nevertheless, an upper limit for the reduction in the positively stained cells may be 100 % or less, more preferably 99% or less.

Furthermore, the MERS-mRBD or the fragment thereof preferably exhibits a binding to anti-MERS-wtRBD neutralizing antibodies (anti-MERS-wtRBD-nABs). That is, a binding strength to anti-MERS-wtRBD neutralizing antibodies (anti-MERS-wtRBD-nABs) is preferably maintained, i.e. in the range of an only moderate reduction in the binding strength up to an increase of the binding strength when compared with the MERS-wtRBD.

The anti-MERS-wtRBD-nABs may belong to different antibody classes like Class I, Class II, Class III and/or Class IV, and/or may be specific for different epitopes in the MERS-wtRBD. The anti-MERS-wtRBD-nABs may be monoclonal and/or polyclonal antibodies reactive against the MERS-wtRBD. Monoclonal anti-MERS-wtRBD-nABs may be obtained recombinantly. Polyclonal anti-MERS-wtRBD-nABs may be obtained from blood plasma or blood serum derived from an immune subject like a convalescent subject, which has been passed through a coronavirus infection, and/or an immunized subject, e.g. immunized by vaccination, wherein such immune subjects are preferably immunologically reactive to the MERS-wtRBD. The subject preferably is a vertebrate, more preferably an immune mammal, more preferably an ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human. Still more preferably the immune subject is a mouse, rat, rabbit, hamster, goat, donkey, horse, dog, cat, sheep or human, etc., most preferably a human.

The binding strength of the MERS-mRBD or the fragment thereof and of the MERS-wtRBD to anti-MERS-wtRBD-nABs can be measured by any method known to the person skilled in the art. For instance, the binding strength can be determined by flow cytometry analysis, e.g. if cells expressing the MERS-mRBD or MERS-wtRBD on the cell surface are stained with an monoclonal anti-MERS-wtRBD-nAB, directly or indirectly labelled with a fluorophore, or *vice versa* if cells expressing an monoclonal anti-MERS-wtRBD-nAB on the cell surface are stained with a soluble MERS-mRBD or soluble MERS-wtRBD, directly or indirectly labelled with a fluorophore, and counting positively stained cells, or by determining the Kd (dissociation constant) or the EC50 (effective concentration with 50% of maximal binding) of the MERS-wtRBD and the MERS-mRBD to the monoclonal anti-MERS-wtRBD-nAB by using an ELISA, or the ED50 (effective dilution at which 50% of serum antibodies is bound) of polyclonal antibodies of blood plasma or blood serum derived from an immune subject like a convalescent subject, which has been passed through a coronavirus infection, and/or an immunized subject, e.g. immunized by vaccination, wherein such immune subjects are preferably immunologically reactive to the MERS-wtRBD. The immune subject is defined as above. Preferably, the EC50 the MERS-wtRBD or MERS-mRBD to the anti-MERS-wtRBD-nABs is determined as described in example 7.

In case of determining the binding strength of the anti-MERS-wtRBD-nABs by flow cytometry analysis, the EC50 and/or the Kd, the binding strength preferably is the average binding strength of several different monoclonal anti-MERS-wtRBD-nABs, preferably of four or more different arbitrarily selected monoclonal anti-MERS-wtRBD-nABs. Preferably, the four different monoclonal anti-MERS-wtRBD-nABs are 4C2h, D12, LCA60, and MERS4V2 as described in example 9 and the average binding strength is the average EC50 or average Kd of 4C2h, D12, LCA60, and MERS4V2, respectively.

The maintained binding strength, i.e. the binding strength in the range of an only moderate reduction in the binding strength up to an increase in the binding strength, is then obtained by comparing the binding strength or the average binding strength of the MERS-wtRBD to the anti-MERS-wtRBD-nABs with the binding strength or the average binding strength, respectively, of the MERS-mRBD to the anti-MERS-wtRBD-nABs.

Preferably, the binding strength is measured by determining the EC50 or the Kd, wherein a higher EC50 or Kd indicates a lower binding strength, or by flow cytometry analysis or determining the ED50, wherein a reduction in the counts of positively stained cells or a lower ED50, respectively, indicate a lower binding strength. Consequently, the maintained, i.e. whether the binding strength is in the range of an only moderate reduction in the binding strength up to an increase in the binding strength, is preferably determined by comparing the EC50, Kd, the counts of positively stained cells and/or the ED50 resulting from the binding between the MERS-wtRBD and the anti-MERS-wtRBD-nABs to the EC50, Kd, the counts of positively stained cells and/or the ED50 resulting from the binding between the MERS-mRBD and the anti-MERS-wtRBD-nABs, respectively.

In the case of determining the binding strength by determining the EC50 or Kd, an only moderate reduction in the binding strength of the MERS-mRBD to the anti-MERS-wtRBD-nABs is preferably indicated by an increase in the EC50 and/or Kd of 3-fold or less, even more preferably of 2-fold or less, still more preferably of 1.5-fold or less and still even more preferably 1.2-fold or less. In the case of determining the binding strength by flow cytometry analysis or determining the ED50, an only moderate reduction in the binding strength of the MERS-mRBD to the RBDanti-MERS-wtRBD-nABs is preferably indicated by a reduction in the counts of positively stained cells and/or in the ED50 of 50% or more, more preferably of 80% or more and even more preferably of 90% or more.

As already stated above, when determining the binding strength by the EC50, the Kd or flow cytometric analysis, the binding strength is preferably an average binding strength as defined above. Consequently, the only moderate reduction in the binding strength as defined above is preferably an only moderate reduction in the average binding strength as defined above.

In order to obtain the best effect in vaccination in case the MERS-mRBD is used a vaccine, the binding strength to the anti-MERS-wtRBD-nABs should be as high as possible. Hence, preferably, the reduction in the binding strength to the anti-MERS-wtRBD-nABs should be as moderate as possible and preferably the binding strength of the anti-MERS-wtRBD-nABs to the MERS-mRBD is preferably not reduced or even increased, i.e. EC50 or Kd are not increased and/or the counts of positively of stained cells or the ED50 are not reduced.

Consequently, in the case of determining the binding strength by determining the EC50 or Kd, an increase in the binding strength of the MERS-mRBD to the anti-MERS-wtRBD-nABs is preferably indicated by an reduction in the EC50 and/or Kd of 10 % or more, more preferably of 25% or more, still even more preferably of 50% or more, still even more preferably of 80% or more and most preferably 90% or more. As stated above, in order to obtain the best effect in vaccination in case the MERS-mRBD is used as a vaccine, the binding strength to the anti-MERS-wtRBD-nABs should be as high as possible. Hence, preferably, there is no upper limit for the increase in the binding strength to the anti-MERS-wtRBD-nABs. Hence, preferably there is no upper limit for the reduction in the EC50 or Kd and the reduction may be even 100%. Nevertheless, an upper limit for the reduction in the EC50 and/or Kd may be 100 % or less, more preferably 99% or less.

In the case of determining the binding strength by flow cytometry analysis or ED50, an increased binding strength of the MERS-mRBD to the anti-MERS-wtRBD-nABs is preferably indicated by an increase in the counts of positively of stained cells and/or in the ED50, respectively, by 1.1-fold or more, more preferably by 1.2-fold or more, even more preferably by 1.5-fold or more, still even more preferably by 2-fold or more. As stated above, in order to obtain the best effect in vaccination in case the MERS-mRBD is used as a vaccine, the binding strength to the anti-MERS-wtRBD-nABs should be as high as possible. Hence, preferably, there is no upper limit for the increase in the binding strength to the anti-MERS-wtRBD-nABs. Hence, preferably there is no upper limit for the increase in positively stained cells and/or the ED50. Nevertheless, an upper limit for an increase in positively stained cells and/or the ED50 may be 10.000-fold or less, more preferably of 1.000-fold or less and still more preferably 100-fold or less.

As already stated above, when determining the binding strength by the EC50, the Kd or flow cytometric analysis, the binding strength is preferably an average binding strength as defined above. Consequently, the increased binding strength as defined above is preferably an increase in the average binding strength as defined above.

Preferably, the present invention relates to a MERS-mRBD or a fragment thereof comprising, more preferably consisting of,

an amino acid sequence or a fragment thereof comprising one or more, preferably one to three, or two, preferably one or two, more preferably one, substitutions of amino acid residues at positions selected from L140, D144, E170, D171 or D173 of the MERS-wtRBD with SEQ ID NO: 194, wherein the MERS-mRBD or the fragment thereof has, except for the substitutions, an amino acid sequence identity of 85% or more to SEQ ID NO: 194.

Preferably, the MERS-mRBD or the fragment thereof has, except for the substitutions, an amino acid sequence identity of 90% or more, more preferably of 95% or more, even more preferably of 98% or more, still even more preferably 99% or more and most preferably of 100% to SEQ ID NO: 194.

The MERS-wtRBD of SEQ ID NO: 1 is a MERS-wtRBD, which for instance is found in the AGN70929.1-variant.

The amino acid sequence identity of the MERS-mRBD to the MERS-wtRBD of SEQ ID NO: 194 as defined above should be low enough to still cover MERS-RBDs of MERS-CoV-variants other than SEQ ID NO 194 in order to allow adaptions of the MERS-mRBD or the fragment thereof thereto or to combinations of the mutations, like substitutions, deletions and insertions, comprised in such variants when compared with SEQ ID NO: 194. Examples for such MERS-CoV-variants and/or the comprised mutations are:

- in the receptor-binding domain (RBD) of the S1 subunit, substitution T424I in 2/8 isolates, and substitutions S459 T in 1/8 isolates;
- in the overlapping RBD/RBM (receptor binding motif) region, substitution W553R, was found in the overlapping RBD/RBM (receptor binding motif) region in 2/8 isolates;
- in the fusion peptide, Q1009 L in hepatad repeat region 1 (HR1), and in the transmembrane (TM) region C1313S (supplement Table 2B, the S2 subunit substitutions included S950 T in the fusion peptide, Q1009 L in hepatad repeat region 1 (HR1), and C1313S in the transmembrane (TM) region (supplement Table 2B; and
- the MERS-CoV-variants listed in the following table (retrieved from https://www.ncbi.nlm.nih.gov/pmc/articles/PMC5165220/):

| GenBank protein ID | Yr isolated | Host | Region | Mutation in MERS-CoV RBD residue: | | | | | | | | | | | | | | | | No. of mutations |
|---|---|---|---|---|---|---|---|---|---|---|---|---|---|---|---|---|---|---|---|---|
| | | | | 400 | 424 | 431 | 434 | 457 | 460 | 482 | 506 | 509 | 510 | 520 | 522 | 529 | 530 | 534 | 582 | |
| AFS88936.1 | 2012 | Human | EMC | K | T | A | A | S | S | A | L | D | D | A | Q | I | V | V | N | 0 |
| AFY13307.1 | 2012 | Human | England | | | | | | | | F | | | | | | | | | 1 |
| AGG22542.1 | 2012 | Human | England | | | | | | | | F | | | | | | | | | 1 |
| AGV08379.1 | 2012 | Human | KSA | | | | | | | | | G | | | | | | | | 1 |
| AGV08584.1 | 2012 | Human | KSA | | | | | | | | | | | | | | | A | | 1 |
| AHI48528.1 | 2013 | Human | KSA | | | | P | | | V | | | | | | | | | | 2 |
| AHI48733.1 | 2013 | Human | KSA | | | | V | | | | | | | | | | | | | 1 |
| AHC74088.1 | 2013 | Human | Qatar | | | | | | F | | | | | | | | | | | 1 |
| AGV08438.1 | 2013 | Human | KSA | | | | | | | | | | | | | | | | 1 | 1 |
| AID55090.1 | 2014 | Human | KSA | | I | | | | | | | | | | | | | | | 1 |
| AID55095.1 | 2014 | Human | KSA | | I | | | | | | | | | | | | | | | 1 |
| AID55087.1 | 2014 | Human | KSA | | | | | | | | | | | | H | | | | | 1 |
| AKL59401.1 | 2015 | Human | Korea | | | | | | | | | | | | | | L | | | 1 |
| ALB08322.1 | 2015 | Human | Korea | | | | | | | | | | G | | | | | | | 1 |
| ALB08289.1 | 2015 | Human | Korea | | | | | | | | | | | | | T | | | | 1 |
| AHY22545.1 | 2013 | Camel | KSA | N | | | | | | | | | | | | | | | | 1 |
| AHL18090,1 | 2013 | Camel | Egypt | | | | | S | | | | | | | | | | | | 1 |
| AHX00711.1 | 2013 | Camel | KSA | | | | | | G | | | | | | | | | | | 1 |
| AHX00721,1 | 2013 | Camel | KSA | | | | | | G | | | | | | | | | | | 1 |
| AHY22555.1 | 2013 | Camel | KSA | | | | | | | | | | S | | | | | | | 1 |

It shall be mentioned that the mutations relating to the MERS-RBD of the MERS-CoV-variants are indicated in view the position in the MERS-Spike protein. The sequence of the MERS-wtRBD of SEQ ID NO: 194 corresponds to the sequence starting at position 367 of the MERS-wtSpike of SEQ ID NO: 200. Consequently, when 366 amino acids are substracted from a certain amino acid position of the MERS-wtSpike or MERS-mSpike, this results in the corresponding amino acid position of the MERS-wtRBD or MERS-mRBD, respectively. A selection of corresponding amino acid positions in the MERS-wtRBD/MERS-mRBD and the MERS-wtSpike/MERS-mSpike is shown in Table B provided further below in the specification before the description of the figures.

Preferably, the one or more substitutions of amino acid residues are one to three, preferably one or two, preferably one, substitutions of the amino acid residues at positions selected from D173, D144 or D171 of the MERS-wtRBD of SEQ ID NO: 194.

More preferably, the one or more substitutions of amino acid residues one or both, preferably one, substitutions of the amino acid residues at positions selected from D144 or D171 of the MERS-wtRBD of SEQ ID NO: 194.

Preferably, the one or more substitutions of amino acid residues are one or more, preferably one to three, more preferably one or two, even more preferably one, substitutions of amino acid residues at positions selected L140, D144, E170, D171 or D173 of the SARS-wtRBD of SEQ ID NO: 194, wherein the substitution of the amino acid residue at the position:

- L140 is L140A;

- D144 is D144A;

- E170 is E170R;

- D171 is D171K; and/or

- D173 is D173K.

More preferably, the one or more substitutions of amino acid residues are one to three, preferably one or two, even more preferably one, substitutions of amino acid residues at positions selected D144, D171 or D173 of the MERS-wtRBD of SEQ ID NO: 194, wherein the substitution of the amino acid residue at the position:

- D144 is D144A;

- D171 is D171K; and

- D173 is D173K.

Even more preferably, the one or more substitutions of amino acid residues are one or both, more preferably one, substitutions of amino acid residues at positions selected D144 or D171 or D173 of the MERS-wtRBD of SEQ ID NO: 194, wherein the substitution of the amino acid residue at the position:

- D144 is D144A; and

- D171 is D171K.

Still more preferably, the MERS-mRBD or the fragment thereof comprises, preferably consists of, an amino acid sequence selected from SEQ ID NO: 195, SEQ ID NO: 196, SEQ ID NO: 197, SEQ ID NO: 198 or SEQ ID NO: 199, more preferably selected from SEQ ID NO: 195 or SEQ ID NO: 196.

## Mutant spike protein of a coronavirus (CORONA-mSpike)

The present invention further provides a mutant spike protein of a coronavirus (CORONA-mSpike) or a fragment thereof comprising the CORONA-mRBD or the fragment thereof according to the present invention.

Preferably, the CORONA-mSpike or the fragment thereof comprising the CORONA-mRBD or the fragment thereof is a recombinant polypeptide or protein or a chemically synthesized polypeptide or protein.

Preferably, the CORONA-mSpike or the fragment thereof comprises, preferably consists of, the CORONA-mSpike or a fragment thereof, wherein the CORONA-spike comprises the CORONA-mRBD or the fragment thereof according to the present invention.

Preferably, the CORONA-mSpike or the fragment thereof is:

- (A) a mutant spike protein of SARS-CoV-2 (SARS-mSpike) or a fragment thereof and the CORONA-mRBD or the fragment thereof is the SARS-mRBD or a fragment thereof according to the present invention; or

- (B) a mutant spike protein of MERS-CoV (SARS-mSpike) or a fragment thereof and the CORONA-mRBD or the fragment thereof is the MERS-mRBD or a fragment thereof according to the present invention.

Hence, preferably, the present invention relates to (A) a SARS-mSpike or a fragment thereof comprising the SARS-mRBD or the fragment thereof according to the present invention, and/or
the present invention relates to (B) a MERS-mSpike or a fragment thereof comprising the MERS-mRBD or the fragment thereof according to the present invention.
Furthermore, in the present invention, it is to be understood that any embodiment disclosed for the CORONA-mRBD or the fragment thereof is/are *mutatis mutandis* applicable to the CORONA-mRBD or the fragment thereof.

*(A) Mutant spike protein of SARS-CoV-2 (SARS-mSpike)*

As stated above, the present invention preferably relates to a SARS-mSpike or a fragment thereof comprising the SARS-mRBD or the fragment thereof according to the present invention.
Preferably, the SARS-mSpike or the fragment thereof comprising the SARS-mRBD or the fragment thereof is a recombinant polypeptide or protein or a chemically synthesized polypeptide or protein.
Preferably, the SARS-mSpike or the fragment thereof comprises, preferably consists of, the SARS-mSpike or a fragment thereof, wherein the SARS-mSpike comprises the SARS-mRBD or the fragment thereof according to the present invention.
Preferably, the SARS-mSpike or the fragment thereof comprises, preferably consists of, an amino acid sequence or a fragment thereof comprising the SARS-mRBD or the fragment thereof according to the present invention and, preferably, a 2P-mutation and/or an inoperative furin cleavage site, more preferably both, wherein the SARS-mSpike has, except for the SARS-mRBD or the fragment thereof and preferably the 2P mutation and/or the inoperative furin cleavage, an amino acid sequence identity of 85% or more to SEQ ID NO: 100.
The 2P-mutation of the SARS-wtSpike is a substitution of amino acid residues K986P and V987P of the SARS-wtSpike of SEQ ID NO: 100.
The inoperative furin cleavage site is a furin cleavage site, 682-RRAR-685, of the SARS-wtSpike of SEQ ID NO: 100 which is made inoperative by mutation, for instance by substitution, deletion or insertion of amino acid residues, preferably by being substituted with 682-QQAQ-685.
Preferably, the SARS-mSpike or the fragment thereof has, except for the SARS-mRBD or the fragment thereof and preferably the 2P-mutation and/or the inoperative furin cleavage site, an amino acid sequence identity of 90% or more, more preferably of 95% or more, even more preferably of 98% or more, still even more preferably of 99% or more and most preferably of 100% to SEQ ID NO: 100.
Furthermore, in the present invention, it is to be understood that any embodiment disclosed for the SARS-mRBD or the fragment thereof is/are *mutatis mutandis* applicable to the SARS-mRBD or the fragment thereof.
Preferably, the SARS-mSpike or the fragment thereof comprises, preferably consist of,
an amino acid sequence or a fragment thereof comprising one or more, preferably one to three, more preferably one or two, even more preferably one, substitutions of amino acid residues at positions selected from G502, Y505, L455, Y489, F456, Q498, F486, Y449 or S373 of the SARS-wtSpike of SEQ ID NO: 100 and preferably the 2P-mutation and/or the inoperative furin cleavage site, more preferably both, wherein the SARS-mSpike or the fragment thereof has, except for the substitutions and preferably the 2P mutation and/or the inoperative furin cleavage, an amino acid sequence identity of 85% or more to SEQ ID NO: 100.
Preferably, the SARS-mSpike or the fragment thereof has, except for the substitutions and preferably the 2P mutation and/or the inoperative furin cleavage site, an amino acid sequence identity of 90% or more, more preferably of 95% or more, even more preferably of 98% or more, still even more preferably 99% or more and most preferably of 100% to SEQ ID NO: 100.
The amino acid sequence identity of the SARS-mSpike or the fragment thereof to the SARS-wtSpike of SEQ ID NO: 100, except for the SARS-mRBD or the fragment thereof and preferably the 2P-mutation and/or the inoperative furin

cleavage site; and/or
the amino acid sequence identity of the SARS-mSpike or the fragment thereof to the SARS-wtSpike of SEQ ID NO: 100, except for the substitutions and preferably the 2P-mutation and/or the inoperative furin cleavage site,
should be low enough to still cover SARS-Spikes of SARS-CoV-2-variants other than the Wuhan-variant, SEQ ID NO: 100, in order to allow adaptions of the SARS-mSpike or the fragment thereof or to combinations of the mutations, like substitutions, deletions and insertions, comprised in such variants when compared with SEQ ID NO: 100. Examples for such SARS-CoV-2-variants are:

B1.351 (Beta) comprising substitutions D60A, D215G, L242H, K417N, E484K, N501Y, D614G and A701V;

B.1.1.7 (Alpha; 501Y.V1) comprising substitutions N501Y, A570D, D614G, P681H, T716I, S982A and D1118H, and deletion H69/V70 and deletion 144;;

P.1 (Gamma; 501Y.V3) comprising substitutions L18F, T20N, P26S, D138Y, R190S, K417T, E484K, N501Y, D614G, H655Y, T1027I and V1176F; and/or

B.1.617.2 (Delta) comprising substitutions T19R, L452R, T478K, D614G, P681R and D950N and deletion 157-158;

wherein the position of each substitution is indicated in view of the SARS-wtSpike of SEQ ID NO: 100.

The amino acids substitutions indicated underlined are located in the RBD of the SARS-Spike of the respective SARS-CoV-2-variant.

For a better understanding of the present invention, it shall be mentioned that the sequence of the SARS-wtRBD of SEQ ID NO: 1 corresponds to the sequence starting at position 319 of the SARS-wtSpike of SEQ ID NO: 100. Consequently, when 318 amino acids are substracted from a certain amino acid position of the SARS-wtSpike or SARS-mSpike, this results in the corresponding amino acid position of the SARS-wtRBD or SARS-mRBD, respectively. A selection of corresponding amino acid positions in the SARS-wtRBD/SARS-mRBD and the SARS-wtSpike/SARS-mSpike is shown in Table A provided further below in the specification before the description of the figures.

Preferably, the one or more substitutions of amino acid residues are one three, preferably one or two, most preferably one, substitutions of the amino acid residue at positions selected from G502, L455, Y489 or Y505, more preferably from G502, L455 or Y505 of the SARS-wtSpike of SEQ ID NO: 100.

More preferably, the one or more substitutions of amino acid residues are one or both, more preferably one, substitutions of the amino acid residue at positions selected from selected from G502 or Y505 or from G502 or L455 of the SARS-wtSpike of SEQ ID NO: 100.

Even more preferably, the one or more substitutions of amino acid residues area substitution of the amino acid residue at the position G502 of the SARS-wtSpike of the SARS-wtSpike of SEQ ID NO: 100.

Preferably, for the SARS-mSpike or the fragment thereof, the substitution of the amino acid residue at the position:

-    G502 is selected from G502A, G502C, G502D, G502E, G502F, G502H, G502I, G502K, G502L, G502M, G502N, G502P, G502Q, G502R, G502S, G502T, G502V, G502W or G502Y;

-    Y505 is selected from Y505A, Y505C, Y505D, Y505E, Y505G, Y505I, Y505K, Y505L, Y505M, Y505N, Y505Q, Y505R, Y505S, Y505T or Y505V;

-    L455 is selected from L455D, L455E, L455K, L455R and L455Y;

-    Y489 is selected from Y489A, Y489C, Y489E, Y489I, Y489K, Y489L, Y489M, Y489N, Y489P, Y489Q, Y489R, Y489S, Y489T or Y489V;

-    F456 is selected from F456A, F456C, F456E, F456G, F456I, F456K, F456N, F456Q, F456R, F456S, F456T, F456W or F456Y;

-    Q498 is selected from Q498C, Q498D, Q498I, Q498K, Q498L or Q498V;

-    F486 is selected from F486C, F486D or F486E;

-    Y449 is selected from Y449A, Y449C, Y449D, Y449E, Y449F, Y449G, Y449H, Y449I, Y449L, Y449M, Y449N, Y449P, Y449Q, Y449S, Y449T, Y449V or Y449W; and/or

-    S373 is S373N.

More preferably, the one or more substitutions of amino acid residues are one to all, preferably one or two, more preferably one, substitutions of amino acid residues at positions selected from G502, L455, Y489 or Y505, more preferably selected from G502, L455 or Y505 of the SARS-wtSpike with SEQ ID NO: 100, wherein the substitution of the amino acid residue at the position:

- G502 is selected from G502R, G502D, G502Y, G502K, G502S, G502P, G502E, G502V, G402A, G502N, G502Q, G502T, G502M, G502H or G502L;

- L455 is selected from L455R or L455E;

- Y505 is selected from Y505N und Y505Q; and

- Y489 is selected from Y489S and Y489T.

Even more preferably, the one or more substitutions of amino acid residues are one or both, preferably one, substitutions of amino acid residues at positions selected from G502 or L505, or from G502 or L455, more preferably from G502 or L455, of the SARS-wtSpike with SEQ ID NO: 100, wherein the substitution of the amino acid residue at the position:

- G502 is selected from G502R, G502D, G502Y, G502K, G502S, G502P, G502E, G502V, G402A, G502N, G502Q, G502T, G502M, G502H or G502L, most preferably is G502R;

- L455 is L455R; and

- Y505 is Y505N.

Still even more preferably, the one or more substitutions of amino acid residues are one substitution of an amino acid residue at position G502 of the SARS-wtSpike with SEQ ID NO: 100, wherein the substitution is G502R, and/or the one or more substitutions of amino acid residues are one substitution of an amino acid residue at position L455 of the SARS-wtRBD with SEQ ID NO: 100, wherein the substitution is L455R, most preferably, the substitution is G502R.

Preferably, the SARS-mSpike or the fragment thereof comprises, preferably consists of, an amino acid sequence selected from:

SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, SEQ ID NO: 181, SEQ ID NO: 182, SEQ ID NO: 183, SEQ ID NO: 184, SEQ ID NO: 185, SEQ ID NO: 186, SEQ ID NO: 187, SEQ ID NO: 188, SEQ ID NO: 189, SEQ ID NO: 190, SEQ ID NO: 191, SEQ ID NO: 192, SEQ ID NO: 193 or SEQ ID NO: 194;

more preferably selected from:
SEQ ID NO: 114, SEQ ID NO: 104, SEQ ID NO: 119, SEQ ID NO: 108, SEQ ID NO: 115, SEQ ID NO: 112, SEQ ID NO: 101, SEQ ID NO: 117, SEQ ID NO: 102, SEQ ID NO: 111, SEQ ID NO: 113, SEQ ID NO: 116, SEQ ID NO: 110, SEQ ID NO: 106, SEQ ID NO: 109, SEQ ID NO: 154, SEQ ID NO: 152, SEQ ID NO: 129, SEQ ID NO: 130, SEQ ID NO: 191 or SEQ ID NO: 192;

even more preferably selected from:
SEQ ID NO: 114, SEQ ID NO: 104, SEQ ID NO: 119, SEQ ID NO: 108, SEQ ID NO: 115, SEQ ID NO: 112, SEQ ID NO: 101, SEQ ID NO: 117, SEQ ID NO: 102, SEQ ID NO: 111, SEQ ID NO: 113, SEQ ID NO: 116, SEQ ID NO:

110, SEQ ID NO: 106, SEQ ID NO: 109, SEQ ID NO: 154 or SEQ ID NO: 129;

still more preferably selected from:
SEQ ID NO: 114, SEQ ID NO: 154, or SEQ ID NO: 129;

still even more preferably selected from:
SEQ ID NO: 114 or SEQ ID NO: 154; and

most preferably comprises, preferably consists of, the amino acid sequence of SEQ ID NO: 114.

*(B) Mutant spike protein of SARS-CoV (MERS-mSoike)*

As stated above, the present invention preferably relates to a MERS-mSpike or a fragment thereof comprising the MERS-mRBD or the fragment thereof according to the present invention.

Preferably, the MERS-mSpike or the fragment thereof comprising the MERS-mRBD or the fragment thereof is a recombinant polypeptide or protein or a chemically synthesized polypeptide or protein.

Preferably, the MERS-mSpike or the fragment thereof comprises, preferably consists of, the MERS-mSpike or a fragment thereof, wherein the MERS-spike comprises the MERS-mRBD or the fragment thereof according to the present invention.

Preferably, the MERS-mSpike or the fragment thereof comprises, preferably consists of, an amino acid sequence or a fragment thereof comprising the MERS-mRBD or the fragment thereof according to the present invention and, preferably, a 2P-mutation and/or an inoperative furin cleavage site, more preferably both, wherein the MERS-mSpike has, except for the MERS-mRBD or the fragment thereof and preferably the 2P mutation and/or the inoperative furin cleavage, an amino acid sequence identity of 85% or more to SEQ ID NO: 200.

The 2P-mutation of the MERS-wtSpike is a substitution of amino acid residues V1060P and L1061P of the MERS-wtSpike of SEQ ID NO: 200.

The inoperative furin cleavage site is a furin cleavage site, 748-RSVR-751, of the MERS-wtSpike of SEQ ID NO: 200, which is made inoperative by mutation, for instance by substitution, deletion or insertion of amino acid residues, preferably by being substituted with 748-ASVG-751.

Preferably, the MERS-mSpike or the fragment thereof has, except for the MERS-mSpike or the fragment thereof and preferably the 2P-mutation and/or the inoperative furin cleavage site, an amino acid sequence identity of 90% or more, more preferably of 95% or more, even more preferably of 98% or more, still even more preferably 99% or more and most preferably of 100% to SEQ ID NO: 200.

Furthermore, in the present invention, it is to be understood that any embodiment disclosed for the MERS-mRBD or the fragment thereof is/are *mutatis mutandis* applicable to the MERS-mRBD or the fragment thereof.

Preferably, the MERS-mSpike or the fragment thereof comprises, preferably consists of, an amino acid sequence or a fragment thereof comprising one or more, preferably one to three, or two, preferably one or two, more preferably one, substitutions of amino acid residues at positions selected from L506, D510, E536, D537 or D539 of the MERS-wtSpike with SEQ ID NO: 200, wherein the MERS-mSpike or the fragment thereof has, except for the substitutions, an amino acid sequence identity of 90% or more to SEQ ID NO: 200.

Preferably, the MERS-mSpike or the fragment thereof has, except for the substitutions, an amino acid sequence identity of 95% or more, more preferably of 98% or more, even more preferably of 99% or more and most preferably of 100% to SEQ ID NO: 200.

The amino acid sequence identity of the MERS-mSpike to the MERS-wtSpike of SEQ ID NO: 200, except for the MERS-mRBD or the fragment thereof and preferably the 2P-mutation and/or the inoperative furin cleavage site; and/or the amino acid sequence identity of the MERS-mSpike to the MERS-wtSpike of SEQ ID NO: 200, except for the substitutions and preferably the 2P-mutation and/or the inoperative furin cleavage site, should be low enough to still cover MERS-Spikes of MERS-CoV-variants other than SEQ ID NO: 200 in order to allow adaptions of the MERS-mSpike thereto or to combinations of the mutations, like substitutions, deletions and insertions, comprised in such variants when compared with SEQ ID NO: 200. Examples for such existing MERS-CoV-variants and/or the comprised mutations are:

- in the receptor-binding domain (RBD) of the S1 subunit, substitution T424I in 2/8 isolates, and substitutions S459 T in 1/8 isolates;

- in the overlapping RBD/RBM (receptor binding motif) region, substitution W553R, was found in the overlapping RBD/RBM (receptor binding motif) region in 2/8 isolates.

- in the fusion peptide, Q1009 L in hepatad repeat region 1 (HR1), and in the transmembrane (TM) region C1313S (supplement Table 2B, the S2 subunit substitutions included S950 T in the fusion peptide, Q1009 L in hepatad repeat region 1 (HR1), and C1313S in the transmembrane (TM) region (supplement Table 2B; and

- the MERS-CoV-variants listed in the following table (retrived from https://www.ncbi.nlm.nih.gov/pmc/articles/PMC5165220/):

| GenBank pro-tein ID | Yr isolat-ed | Host | Region | Mutation in MERS-CoV RBD residue: | | | | | | | | | | | | | | | | No. of mutations |
|---|---|---|---|---|---|---|---|---|---|---|---|---|---|---|---|---|---|---|---|---|
| | | | | 400 | 424 | 431 | 434 | 457 | 460 | 482 | 506 | 509 | 510 | 520 | 522 | 529 | 530 | 534 | 582 | |
| AFS88936.1 | 2012 | Human | EMC | K | T | A | A | S | S | A | L | D | D | A | Q | I | V | V | N | 0 |
| AFY13307.1 | 2012 | Human | | | | | | | | | F | | | | | | | | | 1 |
| AGG22542.1 | 2012 | Human | | | | | | | | | F | | | | | | | | | 1 |
| AGV08379.1 | 2012 | Human | KSA | | | | | | | | | G | | | | | | | | 1 |
| AGV08584.1 | 2012 | Human | KSA | | | | | | | | | | | | | | | A | | 1 |
| AHI48528.1 | 2013 | Human | KSA | | | | P | | | V | | | | | | | | | | 2 |
| AHI48733.1 | 2013 | Human | KSA | | | | | V | | | | | | | | | | | | 1 |
| AHC74088.1 | 2013 | Human | Qatar | | | | | | F | | | | | | | | | | | 1 |
| AGV08438.1 | 2013 | Human | KSA | | | | | | | | | | | | | | | | I | 1 |
| AID55090.1 | 2014 | Human | KSA | | | I | | | | | | | | | | | | | | 1 |
| AID55095.1 | 2014 | Human | KSA | | | I | | | | | | | | | | | | | | 1 |
| AID55087.1 | 2014 | Human | KSA | | | | | | | | | | | | H | | | | | 1 |
| AKL59401.1 | 2015 | Human | Korea | | | | | | | | | | | | | | L | | | 1 |
| ALB08322.1 | 2015 | Human | Korea | | | | | | | | | | G | | | | | | | 1 |
| ALB08289.1 | 2015 | Human | Korea | | | | | | | | | | | | | T | | | | 1 |
| AHY22545.1 | 2013 | Camel | KSA | N | | | | | | | | | | | | | | | | 1 |
| AHL18090,1 | 2013 | Camel | Egypt | | | | | S | | | | | | | | | | | | 1 |
| AHX00711.1 | 2013 | Camel | KSA | | | | | | G | | | | | | | | | | | 1 |
| AHX00721.1 | 2013 | Camel | KSA | | | | | | G | | | | | | | | | | | 1 |
| AHY22555.1 | 2013 | Camel | KSA | | | | | | | | | | | S | | | | | | 1 |

For a better understanding of the present invention, it shall be mentioned that the sequence of the MERS-wtRBD of SEQ ID NO: 194 corresponds to the sequence starting at position 367 of the MERS-wtSpike of SEQ ID NO: 200. Consequently, when 366 amino acids are substracted from a certain amino acid position of the MERS-wtSpike or MERS-mSpike, this results in the corresponding amino acid position of the MERS-wtRBD or MERS-mRBD, respectively. A selection of corresponding amino acid positions in the MERS-wtRBD/MERS-mRBD and the MERS-wtSpike/MERS-mSpike is shown in Table B provided further below in the specification before the description of the figures.

Preferably, the one or more substitutions of amino acid residues are one to all, preferably one or two, preferably one, substitutions of the amino acid residues at positions selected from D539, D510 or D537 of the MERS-wtSpike of SEQ ID NO: 200.

More preferably, the one or more substitutions of amino acid residues are one or both, preferably one, substitutions of the amino acid residues at positions selected from D510 or D537 of the MERS-wtSpike of SEQ ID NO: 200.

Preferably, the one or more substitutions of amino acid residues are one or more, preferably one to three, more preferably one or two, even more preferably one, substitutions of amino acid residues at positions selected L506, D510, E536, D537 or D539 of the MERS-wtSpike of SEQ ID NO: 200, wherein the substitution of the amino acid residue at the position:

- L506 is L506A;

- D510 is D510A;

- E536 is E536R;

- D537 is D537K; and/or

- D539 is 539K.

More preferably, the one or more substitutions of amino acid residues are one to three, preferably one or two, even more preferably one, substitutions of amino acid residues at positions selected D510, D537 or D539 of the MERS-wtSpike of SEQ ID NO: 200, wherein the substitution of the amino acid residue at the position:

- D510 is D510A;

- D537 is D537K; and

- D539 is D539K.

Even more preferably, the one or more substitutions of amino acid residues are one or both, more preferably one, substitutions of amino acid residues at positions selected D510 or D537 of the MERS-wtSpike of SEQ ID NO: 200, wherein the substitution of the amino acid residue at the position:

- D510 is D510A; and

- D537 is D537K.

Still more preferably, the MERS-mSpike or the fragment thereof comprises, preferably consists of, comprises, preferably consists of, an amino acid sequence selected from SEQ ID NO: 201, SEQ ID NO: 202, SEQ ID NO: 203, SEQ ID NO: 204 or SEQ ID NO: 205, more preferably selected from SEQ ID NO: 201 or SEQ ID NO: 202.

### Polypeptides and proteins comprising the CORONA-mRBD or the CORONA-mSpike

The present invention further relates to a polypeptide or protein comprising the CORONA-mRBD or the fragment thereof according to the present invention or the CORONA-mSpike or the fragment thereof according to the present invention.

Preferably, the polypeptide and/or protein comprising the CORONA-mRBD or the fragment thereof or the CORONA-mSpike or the fragment thereof is a recombinant polypeptide or protein, or a chemically synthesized polypeptide or protein.

The polypeptide or protein may comprise one or more additional amino acid sequences other than the CORONA-mRBD or the fragment thereof or the CORONA-mSpike or the fragment thereof. Such additional amino acid sequences may be a domain or a fragment of a protein different to the CORONA-mRBD or the fragment thereof and/or the CORONA-spike or the fragment thereof, a localisation signal like a secreting signal, a stability-regulating amino acid sequence like sequences promoting or preventing the active and/or passive degradation of the polypeptide or protein, polymerizsation domains, like di- or trimerisation domains, a spacer, a linker, etc. Furthermore, the polypeptide or protein may comprise one or more modifications.

Preferably, the CORONA-mRBD or the fragment thereof is (A) the SARS-mRBD or the fragment thereof according to the present invention or (B) the MERS-mRBD or the fragment thereof according to the present invention.

Hence, preferably, the present invention relates to a polypeptide or protein comprising(A) the SARS-mRBD or the fragment thereof according to the present invention or the SARS-mSpike or the fragment thereof according to the present invention; and/or

the present invention relates to a polypeptide or protein comprising (B) the MERS-mRBD or the fragment thereof according to the present invention or the MERS-mSpike or the fragment thereof according to the present invention.

Furthermore, in the present invention, it is to be understood that any embodiment disclosed for the CORONA-mRBD or the fragment thereof and/or for the CORONA-mSpike or the fragment thereof is/are *mutatis mutandis* applicable to the polypeptide or protein comprising the CORONA-mRBD or the fragment thereof or CORONA-mSpike or the fragment thereof.

## *(A) Polypeptides and proteins comprising the SARS-mRBD or the SARS-mSipke*

As stated above, the present invention preferably relates to a polypeptide or protein comprising the SARS-mRBD or the fragment thereof according to the present invention or the SARS-mSpike or the fragment thereof according to the present invention.

Preferably, the polypeptide and/or protein comprising the SARS-mRBD or the fragment thereof or the SARS-mSpike or the fragment thereof is a recombinant polypeptide or protein, or a chemically synthesized polypeptide or protein.

The polypeptide or protein may comprise one or more additional amino acid sequences other than the SARS-mRBD or the fragment thereof or the SARS-mSpike or the fragment thereof. Such additional amino acid sequences may be a domain or fragment of a protein different to the SARS-mRBD or the fragment thereof and/or the SARS-spike or the fragment thereof, a localisation signal like a cytosol localisation signal or a secreting signal, a stability-regulating amino acid sequence like sequences promoting or preventing the active and/or passive degradation of the polypeptide or protein, polymerizsation domains, like di- or trimerisation domains, a spacer, a linker, a tag like a FLAG-tag or His-tag, etc. Furthermore, the polypeptide or protein may comprise one or more modifications like a glycosylation.

Furthermore, in the present invention, it is to be understood that any embodiment disclosed for the SARS-mRBD or the fragment thereof and/or for the SARS-mSpike or the fragment thereof is/are *mutatis mutandis* applicable to the polypeptide or protein comprising the SARS-mRBD or the fragment thereof or SARS-mSpike or the fragment thereof.

## *(B) Polypeptides and proteins comprising the MERS-mRBD or the MERS-mSpike*

As stated above, the present invention preferably relates to a polypeptide or protein comprising the MERS-mRBD or the fragment thereof according to the present invention or the MERS-mSpike or the fragment thereof according to the present invention.

Preferably, the polypeptide and/or protein comprising the MERS-mRBD or the fragment thereof or the MERS-mSpike or the fragment thereof is a recombinant polypeptide or protein, or a chemically synthesized polypeptide or protein.

The polypeptide or protein may comprise one or more additional amino acid sequences other than the MERS-mRBD or the fragment thereof or the MERS-mSpike or the fragment thereof. Such additional amino acid sequences may be a domain or fragment of a protein different to the MERS-mRBD or the fragment thereof and/or the MERS-spike or the fragment thereof, a localisation signal like a cytosol localisation signal or a secreting signal, a stability-regulating amino acid sequence like sequences promoting or preventing the active and/or passive degradation of the polypeptide or protein, polymerizsation domains, like di- or trimerisation domains, a spacer, a linker, a tag like a FLAG-tag or His-tag, etc. Furthermore, the polypeptide or protein may comprise one or more modifications like a glycosylation.

Furthermore, in the present invention, it is to be understood that any embodiment disclosed for the MERS-mRBD or the fragment thereof and/or for the MERS-mSpike or the fragment thereof is/are *mutatis mutandis* applicable to the polypeptide or protein comprising the MERS-mRBD or the fragment thereof or MERS-mSpike or the fragment thereof.

## *Nucleic acid*

Furthermore, the present invention relates to a nucleic acid comprising a nucleotide sequence encoding for:

- the CORONA-mRBD or the fragment thereof according to the present invention;

- the CORONA-mSpike or the fragment thereof according to the present invention; or

- the polypeptide or protein according to the present invention comprising the CORONA-mRBD or the fragment thereof according to the present invention or the CORONA-mSpike or the fragment thereof according to the present invention.

Preferably, the nucleic acid is a DNA or RNA like a mRNA.

Preferably, the nucleic acid is a recombinant nucleic acid or a chemically synthesized nucleic acid.

The nucleic acid may comprise one or more additional nucleotide sequences other than the nucleotide sequence encoding for the

Furthermore, the nucleic acid may comprise one or more additional sequences other than the nucleotide sequence encoding for the CORONA-mRBD or the fragment thereof, the CORONA-mSpike or the fragment thereof or the polypeptide or protein according to the present invention. Such additional nucleotide sequences may be a transcription regulatory sequence like a promoter, an enhancer, terminator, a transcription factor binding motif or a DNA-polymerase or reverse transcriptase binding site, a translation regulatory sequence like a ribosome binding motif, an exon, a intron, a 5'-cap, a poly-A-tail, a localisation signal like a core or cytosol localisation signal, hybridisation stretches and/or a stability regulating sequence nucleotide sequences promoting or preventing the active and/or passive degradation of the nucleic acid, spacer, linker, sequences encoding a tag like a FLAG-tag or His-tag etc. Furthermore, the nucleic acid may comprise modifications like methylation.

More preferably, the nucleic acid preferably consists of a nucleic acid encoding for:

- the CORONA-mRBD or the fragment thereof according to the present invention;

- the CORONA-mSpike or the fragment thereof according to the present invention; or

- the polypeptide or protein according to the present invention comprising the CORONA-mRBD or the fragment thereof according to the present invention or the CORONA-mSpike or the fragment thereof according to the present invention.

Preferably, the CORONA-mRBD or the fragment thereof is (A) the SARS-mRBD according to the present invention and/or (B) the MERS-mRBD according to the present invention; and/or preferably, the CORONA-mSpike or the fragment thereof is (A) the SARS-mSPike according to the present invention and/or (B) the MERS-mSpike according to the present invention.

Hence, preferably, the present invention relates to (A) a nucleic acid comprising a nucleotide sequence encoding for:

- the SARS-mRBD or the fragment thereof according to the present invention;

- the SARS-mSpike or the fragment thereof according to the present invention; or

- the polypeptide or protein according to the present invention comprising the SARS-mRBD or the fragment thereof according to the present invention or the SARS-mSpike or the fragment thereof according to the present invention; and/or

the present invention relates to (B) a nucleic acid comprising a nucleotide sequence encoding for:

- the MERS-mRBD or the fragment thereof according to the present invention;

- the MERS-mSpike or the fragment thereof according to the present invention; or

- the polypeptide or protein according to the present invention comprising the MERS-mRBD or the fragment thereof according to the present invention or the MERS-mSpike or the fragment thereof according to the present invention.

Furthermore, in the present invention, it is to be understood that any embodiment disclosed for:

the CORONA-mRBD or the fragment thereof; and/or
the CORONA-mSpike or the fragment thereof; and/or
the polypeptide or protein comprising the CORONA-mRBD or the fragment

thereof or the CORONA-mSpike or the fragment thereof,

is/are *mutatis mutandis* applicable to the nucleic acid according to the present invention.

*(A) Nucleic acid*

Hence, the present invention preferably relates to (A) a nucleic acid comprising, preferably consisting of, a nucleotide sequence encoding for:

- the SARS-mRBD or the fragment thereof according to the present invention;

- the SARS-mSpike or the fragment thereof according to the present invention; or

- the polypeptide or protein according to the present invention comprising the SARS-mRBD or the fragment thereof according to the present invention or the SARS-mSpike or the fragment thereof according to the present invention.

Preferably, the nucleic acid is a DNA or RNA like a mRNA.
Preferably, the nucleic acid is a recombinant nucleic acid or a chemically synthesized nucleic acid.
The nucleic acid may comprise one or more additional nucleotide sequences other than the nucleotide sequence encoding for the
Furthermore, the nucleic acid may comprise one or more additional sequences other than the nucleotide sequence encoding for the SARS-mRBD or the fragment thereof, the SARS-mSpike or the fragment thereof or the polypeptide or protein according to the present invention. Such additional nucleotide sequences may be a transcription regulatory sequence like a promoter, an enhancer, terminator, a transcription factor binding motif or a DNA-polymerase or reverse transcriptase binding site, a translation regulatory sequence like a ribosome binding motif, an exon, an intron, a 5'-cap, a poly-A-tail, a localisation signal like a core or cytosol localisation signal, hybridisation stretches and/or a stability regulating sequence nucleotide sequences promoting or preventing the active and/or passive degradation of the nucleic acid, spacer, linker, sequences encoding a tag like a FLAG-tag or His-tag etc. Furthermore, the nucleic acid may comprise modifications like methylation.
Preferably, the nucleotide sequence encoding for the SARS-mRBD is SEQ ID NO: 257 or SEQ ID NO: 258.
Preferably, the nucleotide sequence encoding for the SARS-mSpike is SEQ ID NO: 255 or SEQ ID NO: 256.
Furthermore, in the present invention, it is to be understood that any embodiment disclosed for:

the SARS-mRBD or the fragment thereof; and/or
the SARS-mSpike or the fragment thereof, and/or
the polypeptide or protein comprising the SARS-mRBD or the fragment thereof or the SARS-mSpike or the fragment thereof,

is/are *mutatis mutandis* applicable to the nucleic acid according to the present invention.

*(B) Nucleic acid*

Furthermore, the present invention preferably relates to (B) a nucleic acid comprising, preferably consisting of, a nucleotide sequence encoding for:

- the MERS-mRBD or the fragment thereof according to the present invention;

- the MERS-mSpike or the fragment thereof according to the present invention; or

- the polypeptide or protein according to the present invention comprising the MERS-mRBD or the fragment thereof according to the present invention or the MERS-mSpike or the fragment thereof according to the present invention.

Preferably, the nucleic acid is a DNA or RNA like a mRNA.
Preferably, the nucleic acid is a recombinant nucleic acid or a chemically synthesized nucleic acid.
The nucleic acid may comprise one or more additional nucleotide sequences other than the nucleotide sequence encoding for the
Furthermore, the nucleic acid may comprise one or more additional sequences other than the nucleotide sequence encoding for the MERS-mRBD or the fragment thereof, the MERS-mSpike or the fragment thereof or the polypeptide or protein according to the present invention. Such additional nucleotide sequences may be a transcription regulatory sequence like a promoter, an enhancer, terminator, a transcription factor binding motif or a DNA-polymerase or reverse transcriptase binding site, a translation regulatory sequence like a ribosome binding motif, an exon, a intron, a 5'-cap, a poly-A-tail, a localisation signal like a core or cytosol localisation signal, hybridisation stretches and/or a stability regulating sequence nucleotide sequences promoting or preventing the active and/or passive degradation of the nucleic acid, spacer, linker, sequences encoding a tag like a FLAG-tag or His-tag etc. Furthermore, the nucleic acid may comprise modifications like methylation.

Furthermore, in the present invention, it is to be understood that any embodiment disclosed for:

the MERS-mRBD or the fragment thereof; and/or
the MERS-mSpike or the fragment thereof, and/or
the polypeptide or protein comprising the MERS-mRBD or the fragment thereof or the MERS-mSpike or the fragment thereof,

is/are *mutatis mutandis* applicable to the nucleic acid according to the present invention.

### Vaccine composition and Medical use

The present invention further relates to a vaccine composition comprising, preferably consisting of, as an active ingredient:

- one or more CORONA-mRBDs or the fragments thereof according to the present invention;

- one or more CORONA-mSpikes or the fragments thereof according to the present invention;

- one or more polypeptides or proteins according to the present invention comprising the CORONA-mRBD or the fragment thereof according to the present invention or the CORONA-mSpike or the fragment thereof according to the present invention; and/or

- one or more nucleic acids according to the present invention comprising a nucleotide sequence encoding for the one or more CORONA-mRBDs or the fragments thereof, the one or more CORONA-mSpikes or the fragments thereof or the one or more polypeptides or proteins.

Preferably, the vaccine composition further comprises a pharmaceuticallyacceptable adjuvant like Matrix-M1-Adjuvant, a aluminum-containing adjuvant, MF59, AS01, AS02, AS03, AS04, Virosomes, ISA51, CpG ODN, a carrier, a diluent, lipid nanoparticles and/or an excipient.

Preferably, the one or more nucleic acids according to the present invention are mRNA and the vaccine comprises the mRNA packed in a lipid nanoparticle.

Preferably, the vaccine uses a viral vector to deliver the nucleic acid such as DNA.

Preferably, the vaccine composition comprises the active ingredient in an effective amount.

Preferably, the vaccine composition is capable of inducing an immune response against a coronavirus in a subject. Preferably, the coronavirus is SARS-CoV-2 and/or the coronavirus is MERS-CoV.

Preferably, the vaccine composition is a vaccine for the prevention and/or treatment of diseases caused by coronaviruses in a subject. Preferably, the diseases caused by the coronaviruses is COVID-19 caused by SARS-CoV-2 and/or the diseases caused by the coronaviruses is MERS caused by MERS-CoV.

Furthermore, the present invention relates to:

one or more CORONA-mRBDs or the fragments thereof according to the present invention;

one or more CORONA-mSpikes or the fragments according to the present invention;

one or more polypeptides or proteins according to the present invention comprising the CORONA-mRBD or the fragment thereof according to the present invention or the CORONA-mSpike or the fragment thereof according to the present invention;

one or more nucleic acids according to the present invention comprising a nucleotide sequence encoding for the one or more CORONA-mRBDs or the fragments thereof, the one or more CORONA-mSpikes or the fragments thereof or the one or more polypeptides or proteins; and/or

the vaccine composition as described above in view of the CORONA-mRBD and the CORONA-mSpike,

for use in the prevention and/or treatment of diseases caused by coronaviruses in a subject.

Preferably, in the vaccine composition and/or the use in the prevention and/or treatment, one or more is one to three, more preferably one or two and even more preferably two. Preferably, in case e.g. two, three or more CORONA-mRBDs are present, these differ in their sequence to each other, for instance due to the presence of different susbstitutions. The same applies to the presence of two, three or more CORONA-mSpikes, polypeptides or proteins and/or nucleic acids.

Preferably, the term subject as used for the vaccine composition and/or the use in the prevention and/or treatment the subject is a vertebrate, more preferably a mammal, even more preferably an ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human, still more preferably the subject is a human.

Preferably, in the vaccine composition and/or the use in the prevention and/or treatment, the subject is non-infected with the coronavirus and/or is infected with the coronavirus. The subject non-infected with the coronavirus may previously had or had not passed through a coronavirus-infection. The subject infected with the coronavirus may presently has a coronavirus infection. For determining whether a subject is non-infected with the coronavirus or is infected with the coronavirus, any method known to the person skilled in the can be used. An example for a suitable method is a PCR-test.

Preferably, in the vaccine composition and/or the use in the prevention and/or treatment, the diseases caused by the coronaviruses is COVID-19 caused by SARS-CoV-2 and/or the diseases caused by the coronaviruses is MERS caused by MERs-CoV.

Preferably, the SARS-CoV-2 as used in the present invention includes any variants thereof, like Wuhan, B.1.1.7 (Alpha; United Kingdom), B.1.617.2 (Delta; India), B.1.351 (Beta; South Africa), P.1 (Gamma; Brasilia) and/or B.1.1.28., preferably the Wuhan-variant of SARS-CoV-2.

Preferably, the MERS-CoV as used in the present invention includes any variant thereof, like AF88936.1, AFY13307.1, AGG22542.1, AGV08379.1, AGV08584.1, AHI48528.1, AHI48733.1, AHC74088.1, AGV08438.1, AID55090.1, AID55095.1, AID55087.1, AKL59401.1, ALB08322.1, ALB08289.1, AHY22545.1, AHL18090.1, AHX00711.1, AHX0072.1 and AHY22555.1.

Preferably, in the vaccine composition and/or the use in the prevention and/or treatment:

the CORONA-mRBDs or the fragments thereof is (A) the SARS-mRBDs or the fragment thereof according to present invention or (B) the MERS-mRBDs or the fragment thereof according to present invention; and/or
the CORONA-mSpikes or the fragments thereof is (A) the SARS-mSpikes or the fragments thereof according to present invention or (B) the MERS-mSpikes or the fragments thereof according to present invention.

Furthermore, in the present invention, it is to be understood that any embodiment disclosed for the CORONA-mRBD or the fragment thereof and/or for the CORONA-mSpike or the fragment thereof, the polypeptide or protein comprising the CORONA-mRBD or the fragment thereof or the CORONA-mSpike and/or nucleic acid nucleic acid according encoding for the same is/are *mutatis mutandis* applicable to the vaccine composition.

*(A) Vaccine composition and Medical use*

Preferably, the present invention relates to (A) a vaccine composition comprising, preferably consisting of, as an active ingredient:

- one or more SARS-mRBDs or the fragment thereof according to the present invention;

- one or more SARS-mSpikes or the fragments thereof according to the present invention;

- one or more polypeptides or proteins according to the present invention comprising the SARS-mRBD or the fragment thereof according to the present invention or the SARS-mSpike or the fragment thereof according to the present invention; and/or

- one or more nucleic acids according to the present invention comprising a nucleotide sequence encoding for the one or more SARS-mRBDs or the fragments thereof, the one or more SARS-mSpikes or the fragments thereof or the one or more polypeptides or proteins.

Preferably, the vaccine composition (A) further comprises a pharmaceuticallyacceptable adjuvant, carrier, diluent and/or excipient.

Preferably, the vaccine composition (A) comprises the active ingredient in an effective amount.

Preferably, the vaccine composition (A) is capable of inducing an immune response against SARS-CoV-2.

Preferably, the vaccine composition (A) is a vaccine for the prevention and/or treatment of COVID-19 caused by SARS-CoV-2.

Furthermore, the present invention preferably relates to:

- one or more SARS-mRBDs or the fragments thereof according to the present invention;

- one or more SARS-mSpikes or the fragments according to the present invention;

- one or more polypeptides or proteins according to the present invention comprising the SARS-mRBD or the fragment thereof according to the present invention or the SARS-mSpike or the fragment thereof according to the present invention; and/or

- one or more nucleic acids according to the present invention comprising a nucleotide sequence encoding for the one or more SARS-mRBDs or the fragments thereof, the one or more SARS-mSpikes or the fragments thereof or the one or more polypeptides or proteins; and/or

- the vaccine composition as described above in view of the SARS-mRBD and the SARS-mSpike,

for use (A) in the prevention and/or treatment of COVID-19 caused by SARS-CoV-2 in a subject.

Preferably, in the vaccine composition (A) and/or the use (A) in the prevention and/or treatment, one or more is one to three, more preferably one or two and even more preferably two. Preferably, in case e.g. two, three or more SARS-mRBDs are present, these differ in their sequence to each other, for instance due to the presence of different substitutions. The same applies to the presence of two, three or more SARS -mSpikes, polypeptides or proteins and/or nucleic acids.

Preferably, the term subject as used for the vaccine composition (A) and/or the use (A) in the prevention and/or treatment is a vertebrate, more preferably a mammal, even more preferably an ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human, still more preferably the subject is a human.

Preferably, in the vaccine composition (A) and/or the use (A) in the prevention and/or treatment, the subject is non-infected with the SARS-CoV-2 and/or is infected with the SARS-CoV-2. The subject non-infected with the SARS-CoV-2 may previously had or had not passed through a SARS-CoV-2-infection. The subject infected with the SARS-CoV-2 may presently has a SARS-CoV-2 infection. For determining whether a subject is non-infected with the SARS-CoV-2 or is infected with the SARS-CoV-2, any method known to the person skilled in the can be used. An example for a suitable method is a PCR-test.

Preferably, the SARS-CoV-2 as used in the present invention includes any variants thereof, like Wuhan, B.1.1.7 (Alpha; United Kingdom), B.1.617.2 (Delta; India), B.1.351 (Beta; South Africa), P.1 (Gamma; Brasilia) and/or B.1.1.28., preferably the Wuhan-variant of SARS-CoV-2.

Preferably, one or more is two or more, more preferably one to five, even more preferably two to four and still more preferably two. Preferably, in case e.g. two or more SARS-mRBDs are present, these differ in their substituions. The same applies to the presence of two, three or more SARS-mSpikes, polypeptides or proteins and/or nucleic acids.

More preferably, the one or more SARS-mRBDs or the fragments thereof is any one or any combination of the SARS-mRBDs according to the present invention.

Even more preferably, the one or more SARS-mRBDs or the fragments thereof is one SARS-mRBD or the fragment thereof comprising an amino acid sequence or a fragment thereof comprising one substitution of the amino acid residue at the position selected from G184, L137, Y187 or Y171, preferably from G184, L137, or Y187, even more preferably from G184 or L137, of the SARS-wtRBD of SEQ ID NO: 1.

Still more preferably, the one or more SARS-mRBDs or the fragments thereof are a combination of two or more SARS-mRBDs or the fragments thereof comprising each an amino acid sequence or a fragment thereof comprising a substitution of the amino acid residue at the position selected from G184, L137, Y187 or Y171, preferably from G184, L137, or Y187, even more preferably from G184 or L137, of the SARS-wtRBD of SEQ ID NO: 1.

Preferably the substitution of the amino acid residue at the position:

- G184 is selected from is G184R, G184D, G184Y, G184K G184S, G184P, G184E, G184A, G184V, G184N, G184Q, G184T, G184M, G184H or G184L, more preferably is G184R, still more preferably the SARS-mRBD or the fragment thereof comprises, preferably consists of, an amino acid sequence selected from SEQ ID NO: 15, SEQ ID NO: 17, SEQ ID NO: 5, SEQ ID NO: 20, SEQ ID NO: 9, SEQ ID NO: 16, SEQ ID NO: 13, SEQ ID NO: 2, SEQ ID NO: 3, SEQ ID NO: 18, SEQ ID NO: 12, SEQ ID NO: 14, SEQ ID NO: 11, SEQ ID NO: 7 or SEQ ID NO: 10, most preferably comprises, preferably consists of SEQ ID NO: 15;

- L137 is selected from L137R or L137E, more preferably L137R, still more preferably the SARS-mRBD or the fragment thereof comprises, preferably consists of, an amino acid sequence selected from SEQ ID NO: 55 or SEQ ID NO: 53, most preferably comprises, preferably consists of SEQ ID NO: 55;

- Y187 is selected from Y187N or Y187Q, more preferably Y187N, still more preferably the SARS-mRBD or the fragment thereof comprises, preferably consists of, an amino acid sequence selected from SEQ ID NO: 30 or SEQ ID NO: 31, most preferably comprises, preferably consists of SEQ ID NO: 30; and

- Y171 is selected from Y171S or Y171T, still more preferably the SARS-mRBD or the fragment thereof comprises, preferably consists of, an amino acid sequence selected from SEQ ID NO: 92 or SEQ ID NO: 93.

Preferably, the two or more SARS-mRBDs or the fragments thereof differ in their substitution.

Still more preferably, the one or more SARS-mRBDs or the fragments thereof are a combination of two SARS-mRBDs or the fragments thereof, wherein one comprises an amino acid sequence or a fragment thereof comprising a substitution G184R and the other comprises an amino acid sequence or a fragment thereof comprising a substitution L137R of the SARS-wtRBD of SEQ ID NO: 1.

Still even more preferably, the one or more SARS-mRBDs or the fragments thereof are a combination of two SARS-mRBDs or the fragments thereof, wherein one consists of SEQ ID NO: 15 and the other consists of SEQ ID NO: 55.

More preferably, the one or more SARS-mSpikes or the fragments thereof is any one or any combination of the SARS-mSpikes according to the present invention.

Even more preferably, the one or more SARS-mSpikes or the fragments thereof is one SARS-mSpike or the fragment thereof comprising an amino acid sequence or a fragment thereof comprising one substitution of the amino acid residue at the position selected from G502, L455, Y505 or Y489, preferably from G502, L455, or Y505, even more preferably from G502 or L455, of the SARS-wtSpike of SEQ ID NO: 100.

Still more preferably, the one or more SARS-mSpikes or the fragments thereof are a combination of two or more SARS-mSpike or the fragment thereof comprising each an amino acid sequence or a fragment thereof comprising one substitution of the amino acid residue at the position selected from G502, L455, Y505 or Y489, preferably from G502, L455, or Y505, even more preferably from G502 or L455, of the SARS-wtSpike of SEQ ID NO: 100, wherein preferably the substitution of the amino acid residue at the position.

Preferably the substitution of the amino acid residue at the position:

- G502 is selected from is G502R, G502D, G502Y, G502K G502S, G502P, G502E, G502A, G502V, G502N, G502Q, G502T, G502M, G502H or G502L, more preferably of G502R, still more preferably the SARS-mSpike or the fragment thereof comprises, preferably consists of, an amino acid sequence selected from SEQ ID NO: 114, SEQ ID NO: 104, SEQ ID NO: 119, SEQ ID NO: 108, SEQ ID NO: 115, SEQ ID NO: 112, SEQ ID NO: 101, SEQ ID NO: 117, SEQ ID NO: 102, SEQ ID NO: 111, SEQ ID NO: 113, SEQ ID NO: 116, SEQ ID NO: 110, SEQ ID NO: 106 or SEQ ID NO: 109, most preferably comprises, preferably consists of SEQ ID NO: 114;

- L455 is selected from L455R or L455E, more preferably L455R, still more preferably the SARS-mSpike or the fragment thereof comprises, preferably consists of, an amino acid sequence selected from SEQ ID NO: 154 or SEQ ID NO: 152, most preferably comprises, preferably consists of, SEQ ID NO: 154;

- Y505 is selected from Y505N or Y505Q, more preferably Y505N, still more preferably the SARS-mSpike or the fragment thereof comprises, preferably consists of, an amino acid sequence selected from SEQ ID NO: 129 or SEQ ID NO: 130, more preferably comprises, preferably consists of SEQ ID NO: 129; and

- Y489 is selected from Y489S or Y489T, still more preferably the SARS-mSpike or the fragment thereof comprises, preferably consists of, an amino acid sequence selected from SEQ ID NO: 191 or SEQ ID NO: 192.

Preferablly, the two or more SARS-mSpikes or the fragments thereof differ in their substitution.

Still more preferably, the one or more SARS-mSPIKEs or the fragments thereof are a combination of two SARS-mSPIKEs or the fragments thereof, wherein one comprises an amino acid sequence or a fragment thereof comprising a substitution G502R and the other comprises an amino acid sequence or a fragment thereof comprising a substitution L455R of the SARS-wtSPIKE of SEQ ID NO: 1.

Still even more preferably, the one or more SARS-mSPIKEs or the fragments thereof are a combination of two SARS-mSPIKEs or the fragments thereof, wherein one consists of SEQ ID NO: 114 and the other consists of SEQ ID NO: 154.

More preferably, the one or more nucleic acids according to the present invention comprising a nucleotide sequence encoding for the one or more SARS-mRBDs or the fragments thereof, the one or more SARS-mSpikes or the fragments thereof or the one or more polypeptides or proteins is any one or any combination of the nucleic acids according to the present invention.

Even more preferably, the one or more nucleic acids according to the present invention or one nucleic acid selected from SEQ ID NO: 255 or SEQ ID NO: 256, still more preferably a combination of both.

Furthermore, in the present invention, it is to be understood that any embodiment disclosed for:

the SARS-mRBD or the fragment thereof; and/or
the SARS-mSpike or the fragment thereof; and/or
the polypeptide or protein comprising the SARS-mRBD or the fragment thereof or the SARS-mSpike; and/or

the nucleic acid nucleic acid according encoding for the same is/are *mutatis mutandis* applicable to the vaccine composition (A) and/or the use (A) in the prevention and/or treatment.

## (B) Vaccine composition and Medical use

Preferably, the present invention relates additionally or alternatively to (B)

Preferably, the present invention relates to (B) a vaccine composition comprising, preferably consisting of, as an active ingredient:

- one or more MERS-mRBDs or the fragment thereof according to the present invention;

- one or more MERS-mSpikes or the fragments thereof according to the present invention;

- one or more polypeptides or proteins according to the present invention comprising the MERS-mRBD or the fragment thereof according to the present invention or the MERS-mSpike or the fragment thereof according to the present invention; and/or

- one or more nucleic acids according to the present invention comprising a nucleotide sequence encoding for the one or more MERS-mRBDs or the fragments thereof, the one or more MERS-mSpikes or the fragments thereof or the one or more polypeptides or proteins.

Preferably, the vaccine composition (B) further comprises a pharmaceuticallyacceptable adjuvant, carrier, diluent and/or excipient.

Preferably, the vaccine composition (B) comprises the active ingredient in an effective amount.

Preferably, the vaccine composition (B) is capable of inducing an immune response against MERS-CoV.

Preferably, the vaccine composition (B) is a vaccine for the prevention and/or treatment of MERS caused by MERS-CoV.

Furthermore, the present invention preferably relates to:

- one or more MERS-mRBDs or the fragments thereof according to the present invention;

- one or more MERS-mSpikes or the fragments according to the present invention;

- one or more polypeptides or proteins according to the present invention comprising the MERS-mRBD or the fragment thereof according to the present invention or the MERS-mSpike or the fragment thereof according to the present invention; and/or

- one or more nucleic acids according to the present invention comprising a nucleotide sequence encoding for the one or more MERS-mRBDs or the fragments thereof, the one or more MERS-mSpikes or the fragments thereof or the one or more polypeptides or proteins; and/or

- the vaccine composition as described above in view of the MERS-mRBD and the MERS-mSpike,

for use (B) in the prevention and/or treatment of MERS caused by MERS-CoV in a subject.

Preferably, in the vaccine composition (B) and/or the use (B) in the prevention and/or treatment, one or more is two or more, more preferably one to five, even more preferably two to four and still more preferably two. Preferably, in case e.g. two or more MERS-mRBDs are present, these differ in their sequence to each other, for instance due to the presence of different substitutions. The same applies to the presence of two, three or more MERS-mSpikes, polypeptides or proteins and/or nucleic acids.

Preferably, the term subject as used for the vaccine composition (B) and/or the use (B) in the prevention and/or treatment is a vertebrate, more preferably a mammal, more preferably an ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human, still more preferably the subject is a human.

Preferably, in the vaccine composition (B) and/or the use (B) in the prevention and/or treatment, the subject is non-infected with the MERS-CoV and/or is infected with the MERS-CoV. The subject non-infected with the MERS-CoV may previously had or had not passed through a MERS-CoV-infection. The subject infected with the MERS-CoV may presently has a MERS-CoV infection. For determining whether a subject is non-infected with the MERS-CoV or is infected with the MERS-CoV, any method known to the person skilled in the can be used. An example for a suitable method is a PCR-test.

Preferably, the MERS-CoV as used in the present invention includes any variants thereof, like AF88936.1, AFY13307.1, AGG22542.1, AGV08379.1, AGV08584.1, AHI48528.1, AHI48733.1, AHC74088.1, AGV08438.1, AID55090.1, AID55095.1, AID55087.1, AKL59401.1, ALB08322.1, ALB08289.1, AHY22545.1, AHL18090.1, AHX00711.1, AHX0072.1 and AHY22555.1.

Preferably, one or more is two or more, more preferably one to five, even more preferably two to four and still more preferably two. Preferably, in case e.g. two or more MERS-mRBDs or MERS-mRBDs are present, these differ in their sequence to each other, for instance due to the presence of different substitutions. The same applies to the presence of two, three or more MERS-mSpikes or MERS-mSpikes, polypeptides or proteins and/or nucleic acids.

More preferably, the one or more MERS-mRBDs or the fragments thereof is any one or any combination of the MERS-mRBDs according to the present invention.

Even more preferably, the one or more MERS-mRBDs or the fragments thereof is one MERS-mRBD or the fragment thereof, comprising an amino acid sequence or a fragment thereof comprising one substitution of the amino acid residue at the position selected from D144, D171 or D173, preferably from D144 or D171 of the MERS-wtRBD of SEQ ID NO: 194.

Still more preferably, the one or more MERS-mRBDs or the fragments thereof are a combination of two or more MERS-mRBDs or the fragments thereof comprising each an amino acid sequence or a fragment thereof comprising one substitution of the amino acid residue at the position selected from D144, D171 or D173, preferably from D144 or D171 of the MERS-wtRBD of SEQ ID NO: 194.

Preferably the substitution of the amino acid residue at the position:

- D144 is D144A, more preferably the MERS-mRBD or the fragment thereof comprises, preferably consists of, an amino acid sequence selected from SEQ ID NO: 195;
- D171 is D171K, more preferably the MERS-mRBD or the fragment thereof comprises, preferably consists of, an amino acid sequence selected from SEQ ID NO: 196; and
- D173 is D173K, more preferably the MERS-mRBD or the fragment thereof comprises, preferably consists of, an amino acid sequence selected from SEQ ID NO: 197.

Preferablly, the two or more MERS-mRBDs or the fragments thereof differ in their substitution.

More preferably, the one or more MERS-mSpikes or the fragments thereof is any one or any combination of the MERS-mSpikes according to the present invention.

Even more preferably, the one or more MERS-mSpikes or the fragments thereof is one SARS-mSpike or the fragment thereof comprising an amino acid sequence or a fragment thereof comprising one substitution of the amino acid residue at the position selected from D510, D537 or D539, preferably from D510 or D537 of the MERS-wtRBD of SEQ ID NO: 200.

Still more preferably, the one or more MERS-mSpikes or the fragments thereof are a combination of two or more MERS-mSpikes or the fragments thereof comprising each an amino acid sequence or a fragment thereof comprising one substitution of the amino acid residue at the position selected from D510, D537 or D539, preferably from D510 or D537 of the MERS-wtRBD of SEQ ID NO: 200.

Preferably the substitution of the amino acid residue at the position:

- D510 is D510A, more preferably the MERS-mSpike or the fragment thereof comprises, preferably consists of, an amino acid sequence selected from SEQ ID NO: 201;
- D537 is D537K, more preferably the MERS-mSpike or the fragment thereof comprises, preferably consists of, an amino acid sequence selected from SEQ ID NO: 202; and
- D539 is D539K, more preferably the MERS-mSpike or the fragment thereof comprises, preferably consists of, an amino acid sequence selected from SEQ ID NO: 203.

Preferably, the two or more MERS-mSpikes or the fragments thereof differ in their substitution.

More preferably, the one or more nucleic acids according to the present invention comprising a nucleotide sequence encoding for the one or more MERS-mRBDs or the fragments thereof, the one or more MERS-mSpikes or the fragments thereof or the one or more polypeptides or proteins is any one or any combination of the nucleic acids according to the present invention.

Furthermore, in the present invention, it is to be understood that any embodiment disclosed for:

the MERS-mRBD or the fragment thereof; and/or
the MERS-mSpike or the fragment thereof; and/or
the polypeptide or protein comprising the MERS-mRBD or the fragment thereof or the MERS-mSpike; and/or

the nucleic acid nucleic acid according encoding for the same is/are *mutatis mutandis* applicable to the vaccine composition (B) and/or the use (B) in the prevention and/or treatment.

**Method for designing and/or obtaining a VIRUS-mRBD or a fragment thereof**

The present invention further relates to a method for designing and/or obtaining an active ingredient for a vaccine composition comprising the steps of:

(i) providing a mutant receptor binding domain of a virus (VIRUS-mRBD) or a fragment thereof, comprising one or more mutations in a wildtype receptor binding domain of the virus (VIRUS-wtRBD);
(ii) determining, whether the VIRUS-mRBD or the fragment thereof exhibits:

a) a reduced binding strength to a receptor of the receptor binding domain (RBD-receptor) of the virus (VIRUS-RBD-receptor) compared to the VIRUS-wtRBD; and

(iii) selecting the VIRUS-mRBD or the fragment thereof when a) is fulfiled as the active ingredient.

Preferably, the method further comprises:

in step (ii) determining whether the VIRUS-mRBDs or the fragment thereof obtained in step (i) exhibits:

b) a binding to anti-VIRUS-wtRBD neutralizing antibodies (VIRUS-wtRBD-nABs); and

c) optionally, a protein and/or peptide stability,

in step (iii) selecting the VIRUS-mRBD or the fragment thereof when concomitantly a) and b) and optionally, concomitantly a), b) and c) is fulfilled as the active ingredient.

Preferably, steps (i), (ii) and (iii) are conducted in that order.
Preferably, the VIRUS-mRBD or fragment thereof is a mutant receptor-binding domain (mRBD) of a virus spike protein (VIRUS-mRBD) or a fragment thereof.
Preferably, the VIRUS-mRBD of the fragment thereof is a peptide or protein, preferably a protein.
Preferably, the VIRUS-RBD-receptor of the fragment thereof is a soluble and/or membrane-bound form of the VIRUS-RBD-receptor, preferably the soluble form of the VIRUS-RBD-receptor.
Preferably, the mutations are substitutions of amino acid residues.
Preferably, the selection in step (iii) is made by conducting a) or b) successively in any order, wherein it is started with any one of a) or b). More preferably, a VIRUS-mRBD or the fragment thereof, which fulfills the one with which it is started of a) and b) is subjected to any one of the remaining one of a) and b). Then, a VIRUS-mRBD or the fragment thereof, which fulfills the remaining one of a) or b) is selected as concomitabntly fulfilling a) and b). More preferably, the selection in step (iii) is made by conducting a), b) and c) successively in any order, wherein it is started with any one of a), b) or c). Even more preferably, a VIRUS-mRBD or the fragment thereof, which fulfills the one with which it is started of a), b) or c) is subjected to any one of the remaining two of a), b) or c). Then, a VIRUS-mRBD or the fragment thereof, which fulfills the one of the remaining two of a), b) or c) is subjected to the last one of a), b) or c). Then, a VIRUS-mRBD or the fragment thereof, which fulfills the last one of a), b) or c) is selected as concomitantly fulfilling a), b) and c). Preferably, a), b) and optionally c) are successively conducted in the order a), b) and optionally c); or b), a) and optionally c); more preferably in the order a), b) and optionally c).

Preferably, step (ii)c) is performed *in vitro*. The determination *in vitro* whether a protein and/or peptide stability is exhibited can be done by any method known to the person skilled in the art. For instance by an ELISA, which measures the protein and/or peptide stability with specific antibodies that are able to detect the amount of protein using a suitable standard as well as the preservation of protein folding. For instance protein produced by transfection of HEK293 cells as described in example 4 might be produced and secreted into culture supernatants to obtain sufficient protein yields with concentrations >0.5 $\mu$g/ml or preferably higher concentrations. Likewise protein stability may be measured by expression of the protein on the cell surface and detection by a specific, fluorescently labelled antibody followed by FACS analysis. In the present invention, a deep mutational scanning experiment may be performed on a platform that couples genotype-to-phenotype (https://www.nature.com/articles/nmeth.3027.pdf).

Preferably:

step (i) comprises, preferably is performed by, providing a library of VIRUS-mRBDs or the fragments thereof, each comprising one or more mutations in the VIRUS-wtRBD, wherein the VIRUS-mRBDs or the fragments thereof comprised in the library differ at least partially in the mutations;
step (ii) is performed by screening the library for VIRUS-mRBDs or
fragments thereof exhibiting:

a) a reduced binding strength to the VIRUS-RBD-receptor compared to the VIRUS-wtRBD; and

step (iii) comprises selecting from the library one or more VIRUS-mRBDs or the fragments which fulfil a) as the active ingredient.

More preferably:

step (ii) is performed by screening the library for VIRUS-mRBDs or fragments thereof exhibiting:

a) a reduced binding strength to the VIRUS-RBD-receptor compared to the VIRUS-wtRBD; and

b) a binding to VIRUS-wtRBD-nABs; and

c) optionally, a protein and/or peptide stability, and

step (iii) comprises selecting from the library one or more VIRUS-mRBDs or the fragments thereof which concomitantly fulfil a) and b) and optionally, concomitantly fulfil a), b) and c) as the active ingredient.

Preferably, the selection in step (iii) is made by conducting a) or b) successively in any order, wherein it is started with any one of a) or b). More preferably, one or more VIRUS-mRBDs or the fragments thereof, which fulfill the one with which it is started of a) and b) is subjected to any one of the remaining one of a) and b). Then, one or more VIRUS-mRBDs or the fragments thereof, which fulfill the remaining one of a) or b) are selected as concomitantly fulfilling a) and b). More preferably, the selection in step (iii) is made by conducting a), b) and c) successively in any order, wherein it is started with any one of a), b) or c). Even more preferably, one or more VIRUS-mRBDs or the fragments thereof, which fulfill the one with which it is started of a), b) or c) is subjected to any one of the remaining two of a), b) or c). Then, one or more VIRUS-mRBDs or the fragments thereof, which fulfill the one of the remaining two of a), b) or c) are subjected to the last one of a), b) or c). Then, one or more VIRUS-mRBDs or the fragments thereof, which fulfill the last one of a), b) or c) are selected as concomitantly fulfilling a), b) and c). Preferably, a), b) and optionally c) are successively conducted in the order a), b) and optionally c); or b), a) and optionally c); more preferably in the order a), b) and optionally c).

Step (ii)c) may be performed *in vitro* or *in silico*, more preferably, *in vitro*. The screening of the librabry *in vitro* for VIRUS-mRBDs or fragments thereof exhibiting a protein and/or peptide stability can be done by any method known to the person skilled in the art. For instance, the stability can be measured by a deep mutational scanning expepiment. For instance by an ELISA, which measures the protein and/or peptide stability with specific antibodies that are able to detect the amount of protein using a suitable standard as well as the preservation of protein folding. For instance protein produced by transfection of HEK293 cells as described in example 4 might be produced and secreted into culture supernatants to obtain sufficient protein yields with concentrations >0.5 $\mu$g/ml or preferably higher concentrations. Likewise protein stability may be measured by expression of the protein on the cell surface and detection by a specific, fluorescently labelled antibody followed by FACS analysis. In the present invention, a deep mutational scanning experiment may be performed on a platform that couples genotype-to-phenotype (https://www.nature.com/articles/nmeth.3027.pdf).

Preferably, steps (i), (ii)a) and (ii)b) are performed *in vitro* and/or *in silico,* preferably *in vitro.* More preferably, steps (i), (ii)a) and (ii)b) are performed *in silico,* even more preferably, steps (i) is performed *in vitro* and steps (ii)a) and (ii)b) are performed *in silico,* still even more preferably, steps (i), (ii)a) and (ii)b) are performed *in vitro.* The details are described in the following.

*If steps (i), (ii)a) and/or (ii)b) are performed in vitro*

The VIRUS-mRBD or a fragment thereof, comprising one or more mutations in the VIRUS-wtRBD can be provided *in vitro* by any method known to the person skilled in the art. For instance, one or more point mutations can be introduced by recombinant DNA techniques in the VIRUS-wtRBD and the VIRUS-mRBD can be expressed by any suitable expression system, preferably a HEK293 expression system as described in example 4 with the exception that VIRUS-mRBD is expressed on the surface of the cell and thus contains a domain encoding for a transmembrane portion in the polypeptide chain.

The binding strength and the reduced binding strength of the VIRUS-mRBD or the fragment thereof and the VIRUS-wtRBD to the VIRUS-RBD-receptor can be measured and determined as described for the CORONA-mRBD or the fragment thereof and the CORONA-wtRBD to the CORONA-RBD-receptor. Consequently, it is to be understood that any disclosure or embodiment relating to the binding strength and the reduced binding strength disclosed for the CORONA-mRBD or the fragment thereof and the CORONA-wtRBD to the CORONA-RBD-receptor is/are *mutatis mutandis* applicable for measuring the binding strength and the reduced binding strength of the VIRUS-mRBD or the fragment thereof and the VIRUS-wtRBD to the VIRUS-RBD-receptor in the method for designing and/or obtaining an active ingredient for a vaccine composition according to the present invention.

Similarly, the binding of the VIRUS-mRBD or the fragment thereof and the VIRUS-wtRBD to the anti-VIRUS-wtRBD-nABs can be measured and determined as described for the CORONA-mRBD or the fragment thereof and the CORONA-wtRBD to the anti-CORONA-wtRBD-nABs. Consequently, it is to be understood that any disclosure or embodiment relating to the binding disclosed for the CORONA-mRBD or the fragment thereof and the CORONA-wtRBD to the anti-CORONA-wtRBD-nABs is/are *mutatis mutandis* applicable for measuring the binding of the VIRUS-mRBD or the fragment thereof and the VIRUS-wtRBD to the anti-VIRUS-wtRBD-nABs in the method for designing and/or obtaining an active ingredient for a vaccine composition according to the present invention.

Preferably, step (i) comprises, preferably is performed by, providing a library of VIRUS-mRBDs or the fragments thereof, each comprising one or more mutations in the VIRUS-wtRBD, wherein the VIRUS-mRBDs or the fragments thereof comprised in the library differ at least partially in the mutations. The library of such VIRUS-mRBDs or the fragments thereof can be provide by any method known to the person skilled in the art. Preferably, the library can for instance be generated by overlap extension PCR (https://www.sciencedirect.com/science/article/pii/S0022283613004300?via%3 Dihub), by error-prone PCR (https://link.springer.com/protocol/10.1385/1-59259-395-X:3) or by chemical mutagenesis (https://academic.oup.com/nar/article/32/4/1448/1038612?login=true), like strategies that create exactly one codon mutation per gene (https://journals.plos.org/plosone/article?id=10.1371/journal.pone.0052031 ; https://www.sciencedirect.com/science/article/pii/S0003269713005782?casa_t oken=d5JPaeUuTwYAAAAA:-F2ChRH8nmjiVhQYPsl1BF4JpK-m E0CoVBibRWGkDKxCPd7D3IVxW7n-f7rRFCxRPGYJc8Q), wherein the designs may be adjusted to multiple codon mutations per gene to examine the average effects of mutations (https://www.ncbi.nlm.nih.gov/pmc/articles/PMC4104320/) and cloned and expressed from a plasmid or a virus, or an *in vitro* system such as T7 or M13 bacteriophage display, ribosome display, E. coli display, or most preferably, a mammalian cell display.

The screening of the library for VIRUS-mRBDs or fragments thereof exhibiting a) can be done by any method known to the person skilled in the art. Preferably, the binding strength is determined by flow cytometry analysis, e.g. if cells expressing the VIRUS-mRBD or VIRUS-wtRBD on the cell surface are stained with the VIRUS-RBD-receptor, directly or indirectly labelled with a fluorophore, or *vice versa* if cells expressing the VIRUS-RBD-receptor on the cell surface are stained with a soluble VIRUS-mRBD or soluble VIRUS-wtRBD, directly or indirectly labelled with a fluorophore, and counting and positively and/or negatively selecting stained cells, wherein a reduction in the counts of positively stained cells indicates a lower binding strength. The reduced binding strength is then obtained by comparing the binding strength of the VIRUS-wtRBD to the VIRUS-RBD-receptor with the binding strength of the VIRUS-mRBD to the VIRUS-RBD-receptor. In case the binding strength is determined by flow cytometry analysis, the reduced binding strength is preferably determined by comparing the counts of positively stained cells resulting from the binding between the VIRUS-wtRBD to the VIRUS-RBD-receptor with the counts of positively stained cells resulting from the binding between the VIRUS-mRBD to the VIRUS-RBD-receptor. Preferably, a reduced binding strength of the VIRUS-mRBD to the VIRUS-RBD-receptor is indicated by a reduction in the counts of positively stained cells of 50% or more, more preferably of 80% or more, even more preferably of 90% or more, still more preferably of 95% or more and still even more preferably of 98% or more. In order to obtain the best effect in vaccination in case the VIRUS-mRBD is used as a vaccine, the binding strength to the VIRUS-RBD-receptor should be reduced as much as possible. Hence, preferably, there is no upper limit for the reduction in the binding strength to the VIRUS-RBD-receptor. Hence, preferably there is no upper limit for the reduction in positively stained cells and the reduction may be even 100%. Nevertheless, an upper limit for the reduction in the positively stained cells may be 100 % or less, more preferably 99% or less.

The screeing of the library for VIRUS-mRBDs or fragments thereof exhibiting b) can be done by any method known to the preson skilled in the art. Exhibiting b) preferably means that a binding strength to anti-VIRUS-wtRBD-nABs is maintained, i.e. in the range of an only moderate reduction in the binding strength up to an increase of the binding strength when compared with the VIRUS-wtRBD. The anti-VIRUS-wtRBD-nABs may belong to different antibody classes and are *mutatis mutandis* defined as above for the anti-CORONA-wtRBD-nABs, anti-SARS-wtRBD-nABs and/or anti-MERS-wtRBD-nABs.

The binding strength of the VIRUS-mRBD or the fragment thereof and of the VIRUS-wtRBD to anti-VIRUS-wtRBD-nABs can be measured by any method known to the person skilled in the art. Preferably, the binding strength is determined by flow cytometry analysis, e.g. if cells expressing the VIRUS-mRBD or VIRUS-wtRBD on the cell surface are stained with the anti-VIRUS-wtRBD-nABs, directly or indirectly labelled with a fluorophore, or *vice versa* if cells expressing the anti-VIRUS-wtRBD-nABs on the cell surface are stained with a soluble VIRUS-mRBD or soluble VIRUS-wtRBD, directly or indirectly labelled with a fluorophore, and counting positively stained cells. In case of determining the binding strength of the anti-VIRUS-wtRBD-nABs by flow cytometry analysis, the binding strength preferably is the average binding strength of several different monoclonal anti-VIRUS-wtRBD-nABs, preferably of four or more different arbitrarily selected monoclonal anti-VIRUS-wtRBD-nABs.

The maintained binding strength, i.e. the binding strength in the range of an only moderate reduction in the binding strength up to an increase in the binding strength, is then obtained by comparing the binding strength or the average binding strength of the virus-wtRBD to the anti-VIRUS-wtRBD-nABs with the binding strength or the average binding strength, respectively, of the virus-mRBD to the anti-VIRUS-wtRBD-nABs.

Preferably, the binding strength is measured by flow cytometry analysis wherein a reduction in the counts of positively stained cells indicate a lower binding strength. Consequently, the maintained, i.e. whether the binding strength is in the range of an only moderate reduction in the binding strength up to an increase in the binding strength, is preferably determined by comparing the counts of positively stained cells resulting from the binding between the VIRUS-wtRBD and the anti-VIRUS-wtRBD-nABs to the counts of positively stained cells resulting from the binding between the VIRUS-mRBD and the anti-VIRUS-wtRBD-nABs, respectively.

In the case of determining the binding strength by flow cytometry analysis, an only moderate reduction in the binding strength of the virus-mRBD to the RBDanti-VIRUS-wtRBD-nABs is preferably indicated by a reduction in the counts of positively of stained cells and/or in the ED50 of 50% or more, more preferably of 80% or more and even more preferably of 90% or more.

As already stated above, when determining the binding strength by flow cytometric analysis, the binding strength is preferably an average binding strength as defined above. Consequently, the only moderate reduction in the binding strength as defined above is preferably an only moderate reduction in the average binding strength as defined above.

In order to obtain the best effect in vaccination in case the virus-mRBD is used a vaccine, the binding strength to the anti-VIRUS-wtRBD-nABs should be as high as possible. Hence, preferably, the reduction in the binding strength to the anti-VIRUS-wtRBD-nABs should be as moderate as possible and preferably the binding strength of the anti-VIRUS-wtRBD-nABs to the virus-mRBD is preferably not reduced or even increased, i.e. the counts of positively of stained cells are not reduced.

In the case of determining the binding strength by flow cytometry analysis, an increased binding strength of the virus-mRBD to the anti-VIRUS-wtRBD-nABs is preferably indicated by an increase in the counts of positively of stained cells by 1.1-fold or more, more preferably by 1.2-fold or more, even more preferably by 1.5-fold or more, still even more preferably by 2-fold or more. As stated above, in order to obtain the best effect in vaccination in case the virus-mRBD is used as a vaccine, the binding strength to the anti-VIRUS-wtRBD-nABs should be as high as possible. Hence, preferably, there is no upper limit for the increase in the binding strength to the anti-VIRUS-wtRBD-nABs. Hence, preferably there is no upper limit for the increase in positively stained cells. Nevertheless, an upper limit for an increase in positively stained cells may be 10.000-fold or less, more preferably of 1.000-fold or less and still more preferably 100-fold or less.

Preferably, steps (i), (ii)a) and/or (ii)b) are performed by using a deep mutational scanning experiment.

Preferably, if all three steps (i), (ii)a) and/or (ii)b) are preformed *in vitro,* optional step (ii)c) is also performed *in vitro* as described above.

*If step (i) is performed in vitro or in silico and steps (ii)a) and (ii)b) are performed in silico*

If all three steps (i), (ii)a) and/or (ii)b) are performed *in silico* by using a computer, data relating to VIRUS-wtRBD are provided to the computer and processed by bioinformatic analysis, wherein preferably, the data provided to the computer are from crystal structures of the VIRUS-wtRBD or a fragment thereof.

When data of crystal structures are provided, preferably, steps (i) and (ii)a) are combined by providing data from crystal structures of the VIRUS-wtRBD or a fragment thereof in complex with the VIRUS-RBD-receptor, and by providing data of the VIRUS-mRBDs or the fragments thereof or preferably of the library of VIRUS-mRBDs or the fragments thereof to the computer, and processing the data by bioinformatic analysis in order to determine whether the VIRUS-mRBD or fragment thereof exhibits a), or more preferably, in order to perform the screening of the library for VIRUS-mRBDs or fragments thereof exhibiting a). Preferably, the determination whether a) is exhibited is done by identifying, based on the crystal structures of the VIRUS-wtRBD or a fragment thereof in complex with the VIRUS-RBD-receptor, amino acid residues in the VIRUS-wtRBD or a fragment thereof interfering with the VIRUS-RBD-receptor and selecting as exhibiting a) VIRUS-mRBDs, preferably of the library, which comprise one or more mutations in the interfering amino acid residues.

Preferably, the data from crystal structures relating to the complex of VIRUS-wtRBD with the VIRUS-RBD-recepter are preferably crystal structures from one or more experimental structures, or computational models, of the VIRUS-wtRBD in complex with the VIRUS-RBD-receptor, or the relevant domain of the VIRUS-RBD-receptor binding to the VIRUS-mRBD or the fragment thereof.

Similarly, when data of crystal structures are provided, preferably, steps (i) and (ii)b) are combined by providing data from crystal structures of the VIRUS-wtRBD or a fragment thereof in complex with anti-VIRUS-wtRBD-nABs to the computer, and by providing data of the VIRUS-mRBD or the fragment thereof or preferably data of the library of VIRUS-mRBDs or the fragments thereof and processing the data by bioinformatic analysis in order to determine whether the VIRUS-mRBD or fragment thereof exhibits b), or more preferably, in order to perform the screening of the library for VIRUS-mRBDs or fragments thereof exhibiting b). Preferably, the determination whether b) is exhibited is done by identifying, based on the crystal structures of the VIRUS-wtRBD or a fragment thereof in complexes with anti-VIRUS-wtRBD-nABs, amino acid residues in the VIRUS-wtRBD or a fragment thereof non-interfering with the anti-VIRUS-wtRBD-nABs and selecting as exhibiting b) VIRUS-mRBDs, preferably of the library, which comprise one or more mutations in the non-interfering amino acid residues.

More preferably, the non-interfering amino acid residues are selected from the interfering amino acid residues idenfied in the determination of a).

Preferably, the data from crystal structures relating to the complex with the anti-VIRUS-wtRBD-nABs are preferably crystal structures from one or more experimental structures, or computational models, of the the VIRUS-wtRBD in complex with the anti-VIRUS-wtRBD-nABs, or the relevant domain of the anti-VIRUS-wtRBD-nABs binding to the VIRUS-mRBD or the fragment thereof.

Preferably, if all three steps (i), (ii)a) and/or (ii)b) are preformed *in silico* by providing data from crystal structures relating to the complex of the VIRUS-wtRBD with the VIRUS-RBD-recepter, optional step (ii)c) is performed *in vitro* as described above.

If step (i) is performed *in vitro* and steps (ii)a) and (ii)b) are performed *in silico,* then optional step (ii)c) is also performed *in silico,* wherein preferably, step (i) is performed *in vitro* by using one or more deep mutational scanning experiments, and preferably, steps (ii)a) and (ii)b) and optionally step (ii)c) are performed *in silico* by using a computer, wherein data relating to the VIRUS-mRBD and the VIRUS-wtRBD are provided to a computer and processed by bioinformatic analysis, wherein the data provided to the computer are preferably from one or more deep mutational scanning experiments of the VIRUS-mRBD or a fragment thereof and of the VIRUS-wtRBD or a fragment thereof.

Preferably, step (ii)a) is performed using data from one or more deep mutational scanning experiments with a fitness measure reflecting the binding of the VIRUS-mRBD or fragment thereof (query antigen, s) to VIRUS-RBD-receptor ($R_{endo(s)}$), wherein $R_{endo}$ is the log-scale difference in dissociation constant, K, of the query antigen, s, compared to a reference antigen (for example a wildtype), r, for example by titration, wherein the expression is: $Log(K(s))-Log(K(r))$.

Similarly, step (ii)b) is performed using data from one or more deep mutational scanning experiments with a fitness measure of the VIRUS-mRBD or fragments thereof (s) reflecting escape from one or more VIRUS-wtRBD-nABs ($R_{nab(s)}$), wherein $R_{nab}$ is the escape fraction preferably specifying a count with decreased binding compared to a total count as quantified by FACS.

Preferably, additionally step (ii)c) is performed. Step (ii)c) is performed using data from one or more deep mutational scanning experiments with a fitness measure reflecting the stability of the VIRUS-mRBD or fragment thereof (s) ($R_{stab(s)}$), wherein $R_{stab}$ is the log-scale difference in count, c, compared to a to reference antigen, r, for example by fluorescence intensity, wherein the expression is: $Log(c(s))-Log(c(r))$.

Even more preferably:

step (iii) further comprises scoring of the one or more VIRUS-mRBDs or the fragments thereof of the library as to their potential to:

a) reduce the binding strength to the VIRUS-RBD-receptor; and

b) optionally, a binding to VIRUS-wtRBD-nABs; and

c) further optionally, exhibit a protein and/or peptide stability; and

selecting, based on the scoring, from the library one or more VIRUS-mRBDs or fragments thereof having the highest score for a), preferably the highest score for a combination of a) and b) and more preferably the the highest score for a combination of a), b) and c) as the active ingredient.

Preferably a), b) and c) are all weighted equally to each other, e.g. a):b):c) = 1:1:1.

Preferably, the scoring is performed *in silico* by using a computer. Preferably, the scoring comprises scoring of the one or more VIRUS-mRBDs or fragments thereof is based on the, preferably normalized, data of $R_{endo(s)}$, optionally $R_{nab(s)}$ and further optionally $R_{stab(s)}$, wherein:

$$S_{tot(s')} = N_{endo(s')};$$

optionally:

$$S_{tot(s')} = N_{nab(s')} - N_{endo(s')};$$

and
further optionally:

$$S_{tot(s')} = N_{stab(s')} - N_{nab(s')} - N_{endo(s')}$$

wherein

$S_{tot(s')}$ is the total score of a VIRUS-mRBD or fragment thereof;

$N_{endo(s')}$ is normalized binding of the VIRUS-mRBD or fragment thereof (s) to VIRUS-RBD-receptor ($R_{endo(s)}$);

$N_{nab(s')}$ is the normalized escape of the VIRUS-mRBD or fragment thereof from one or more VIRUS-wtRBD-nABs ($R_{nab(s)}$) and

$N_{stab(s')}$ stability of the VIRUS-mRBD or fragment thereof ($R_{stab(s)}$), and

selecting from the library one or more VIRUS-mRBDs or fragments thereof to be used as the active ingredient having the highest score $S_{tot(s')}$.

Preferably, the scoring of the one or more VIRUS-mRBDs or fragments thereof is based on the normalized of $R_{endo(s)}$, $R_{nab(s)}$ and $R_{stab(s)}$, wherein preferably, before the scoring the data of $R_{endo(s)}$ and $R_{stab(s)}$ $R_{stab(s)}$ are preselected, wherein

$R_{stab(s)}$ of a VIRUS-mRBD or a fragment thereof should be at least a factor of exp(-alpha) of the VIRUS-wtRBD; and $R_{endo(s)}$ of a VIRUS-mRBD or a fragment thereof should be at least a factor of exp(-beta) of the VIRUS-wtRBD, wherein preferably threshold values for alpha and beta are set. By the threshold values for alpha and beta it can be controlled how destabilizing and how strongly binding VIRUS-mRBDs should be to be considered for the scoring.

Preferably, the method for designing and/or obtaining is for obtaining the active ingredient.

Preferably, the method for designing and/or obtaining an active ingredient is for designing and/or obtaining an active ingredient for a vaccine composition against a virus.

Preferably, as the active ingredient, the VIRUS-mRBD or fragment thereof or the one or more the VIRUS-mRBDs or fragments thereof may be present as:

- one or more VIRUS-mRBDs or the fragments thereof; and/or

- one or more mutant spike proteins of the virus (VIRUS-mSpike) or fragments thereof comprising the VIRUS-mRBD or the fragment thereof; and/or

- one or more polypeptides or proteins comprising the VIRUS-mRBD or the fragment thereof or the VIRUS-mSpike or the fragment thereof; and/or

- one or more nucleic acids encoding for:

    • the VIRUS-mRBD or the fragment thereof;
    • the VIRUS-mSpike or the fragment thereof; or
    • the polypeptide or protein.

Preferably, in the method for designing and/or obtaining the active ingredient:

- the VIRUS-mRBD is the CORONA-mRBD according to the present invention, more preferably the SARS-mRBD and/or the MERS-mRBD according to the present invention;

- the VIRUS-wtRBD is the CORONA-wtRBD according to the present invention, more preferably the SARS-wtRBD (SEQ ID NO: 1) and/or the MERS-wtRBD (SEQ ID NO: 194) according to the present invention;

- the VIRUS-RBD-receptor is the CORONA-RBD-receptor, more preferably the ACE2 and/or the DPP4;

- the VIRUS-wtRBD-nABs are CORONA-wtRBD-nABs according to the present invention, more preferably SARS-wtRBD-nABs and/or MERS-wtRBD-nABs according to the present invention; and

- VIRUS-mSpike is the CORONA-mSpike according to the present invention, more preferably the SARS-mSpike and/or the MERS-mSpike according to the present invention.

Furthermore, in the present invention, it is to be understood that any embodiment disclosed for:

the CORONA-mRBD, SARS-mRBD and/or the MERS-mRBD or the fragment thereof; and/or
the CORONA-mSpike, SARS-mSpike and/or MERS-mSpike or the fragment thereof;
the polypeptide or protein comprising CORONA-mRBD, SARS-mRBD or MERS-mRBD or the fragment thereof or the CORONA-mSpike, SARS-mSpike or MERS-mSpike;
the nucleic acid nucleic acid encoding for the same according to the present invention;
the vaccine composition comprising the same according to the present invention; and/or
the use in the prevention and/or treatment

is/are *mutatis mutandis* applicable to the method for designing and/or obtaining the active ingredient for a vaccine composition.

VIRUS-mRBD or a fragment thereof obtained by the method for designing and/or obtaining an active ingredient for a vaccine composition according to the present invention as the active ingredient.

Preferably, the VIRUS-mRBD is the CORONA-mRBD according to the present invention, more preferably the SARS-mRBD and/or MERS-mRBD according to the present invention.

Furthermore, the present invention relates to a method for the prevention and/or treatment of diseases caused by coronaviruses, preferably COVID-19 caused by SARS-CoV-2 and/or MERS caused by MERS-CoV, in a subject comprising administering to the subject:

- one or more SARS-mRBDs or the fragments thereof according to the present invention;

- one or more SARS-mSpikes or the fragments according to the present invention;

- one or more polypeptides or proteins according to the present invention;

- one or more nucleic acids according to the present invention; and/or

- the vaccine composition according to the present invention.

Moreover, the present invention relates to a method for inducing an immune response, preferably against coronavirus, SARS-CoV-2, MERS-CoV in a subject comprising administering to the subject:

- one or more SARS-mRBDs or the fragments thereof according to the present invention;

- one or more SARS-mSpikes or the fragments according to the present invention;

- one or more polypeptides or proteins according to the present invention;

- one or more nucleic acids according to the present invention; and/or

- the vaccine composition according to the present invention.

Furthermore, in the present invention, it is to be understood that any embodiment disclosed for the CORONA-mRBD/SARS-mRBD and/or MERS-mRBD or the fragment thereof and/or for the CORONA-mSpike/SARS-mSpike and/or MERS-mSpike or the fragment thereof, the polypeptide or protein comprising CORONA-mRBD/SARS-mRBD or MERS-mRBD or the fragment thereof or the CORONA-mSpike/SARS-mSpike or MERS-mSpike, nucleic acid nucleic acid according encoding for the same, the vaccine composition comprising the same, the use in the prevention and/or treatment and/or the method for obtaining the same is/are *mutatis mutandis* applicable to the method for the prevention and/or treatment of diseases caused by coronaviruses and/or the method for inducing an immune response.

Table A: Selection of corresponding amino acid positions in the SARS-wtRBD/SARS-mRBD and the SARS-wtSpike/SARS-mSpike

| SARS position spike | SARS position RBD |
|---|---|
| S373 | S55 |
| K417 | K99 |
| Y449 | Y131 |
| L452 | L134 |
| L455 | L137 |
| F456 | F138 |
| Y473 | Y155 |
| A475 | A157 |
| T478 | T478 |
| E484 | E166 |
| F486 | F168 |
| Y489 | Y171 |
| G496 | G178 |
| Q498 | Q180 |
| N501 | N183 |
| G502 | G184 |
| Y505 | Y187 |

Table B: Selection of corresponding amino acid positions in the MERS-wtRBD/MERS-mRBD and the MERS-wtSpike/MERS-mSpike

| MERS position spike | MERS position RBD |
|---|---|
| L506 | L140 |
| D510 | D144 |
| E536 | E170 |
| D537 | D171 |

(continued)

| MERS position spike | MERS position RBD |
|---|---|
| D539 | D173 |

The present invention is further illustrated by the following figures and examples.

*BRIEF DESCRIPTION OF THE DRAWINGS*

Figs. 1A-G show that sACE2 interferes with the generation of B-cells secreting cAbs (competitive antibodies; synonymously used to 'nAb') *in vivo* Fig. 1A is a structure of modelled mouse ACE2 with humanized residues and the RBD footprint. Fig. 1B is an overview of the immunization schedule and time points of DropMap analysis. Fig. 1C shows the frequency of B cells secreting cAbs.

Fig. 1D shows the assessment of ACE2-blocking-of-binding to the RBD by ELISA. Fig. 1E shows a dose-response anlaysis of the data of Fig. 1C . Fig. 1F shows a comparative analysis of cAbs secretion upon immunization. Fig. 1G shows a DroMap analysis of splenocytes after full spike immunizations with or without mhACE2.

Figs. 2A-C show the identification *in silico* of SARS-mRBDs with reduced ACE2 binding and preserved binding of SARS-wtRBD specific nABs by using bioinformatics analysis.

Figs. 3A-C show the binding strengths of ACE2 and different monoclonal nABs to the SARS-wtRBD and different SARS-mRBDs having single, double, and triple amino acid substitutions determined *in vitro* by using ELISA.

Figs. 4A-F and Fig. 6 show the binding strengths of ACE2 and different monoclonal nABs to the SARS-wtRBD and different SARS-mRBDs determined *in vitro* by using ELISA.

Fig. 5 lists the EC50 values determined for different monoclonal nABS and ACE2 in view of the SARS-wtRBD and different SARS-mRBDs.

Fig. 7 shows the frequency of B-cells following immunizations with either SARS-wtRBD or different SARS-mRBDs determined *in vivo* by using a mouse model with controlled sACE2 levels.

Figs. 8A and B show an *in silico* modelling of epitope masking by sACE2 for newly emerging SARS-CoV-2 variants comprising a substitution N501Y.

Fig. 9 shows the binding strengths of DPP4 and different monoclonal nABs to either the MERS-wtRBD or different MERS-mRBDs determined *in vitro* by using ELISA.

Fig. 10 shows the results of a pull-down-assay of human DPP4 and rabbit DPP4 from serum by using either the MERS-wtRBD or different MERS-mRBDs.

Fig. 11 shows the peptide sequence of the wild type SARS-CoV-2 full spike protein with highlighted regions.

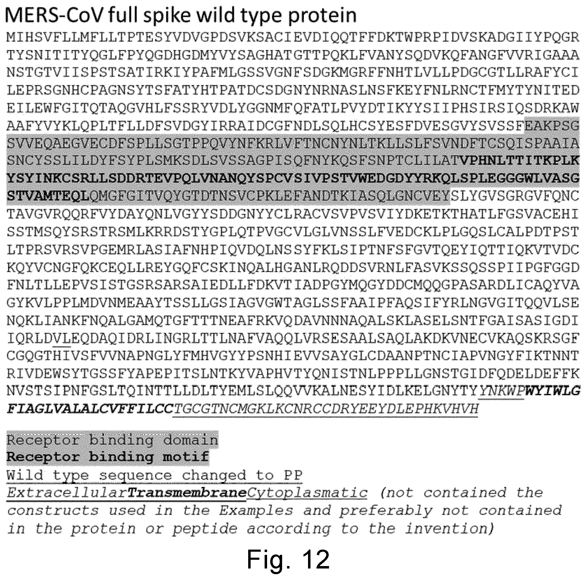

Fig. 12 shows the peptide sequence of the wild type MERS-CoV full spike protein with highlighted regions.

*EXAMPLES*

Annotation: in the following examples in some cases the positions of amino acid substitutions of RBDs are indicated in view of the position in the Spike. In this regards reference is made to Tables A and B above to assign the position in the spike to the respective position in the RBD.

*Example 1*

*Masking of SARS-wtRBD by sACE2 or a nAB impairs B cell and antibody responses in an in vivo mouse model - Figs. 1A-G*

**Fig. 1A-G)** Soluble ACE2 interferes with the generation of B cells secreting antibodies that interfere/compete with ACE2-RBD interaction (cAbs) in vivo.

**Fig. 1A)** Structure of modeled mouse ACE2 with humanized residues depicted in blue and the RBD footprint in black.

**Fig. 1B)** Overview of the immunization schedule and time points of DropMap analysis. Individual splenocytes were encapsulated into micro-droplets for the analysis, and binding to RBD and competition with sACE2 of secreted antibodies were measured by fluorescence relocation.

**Fig. 1C)** The frequency of B cells secreting cAbs (as a percentage of IgG secreting cells) at day 32 are shown for immunizations using RBD (3 μg/immunization, 120 pmol) with indicated masking proficient nAb (18 pmol or 86 pmol) or mhACE2 (4 pmol, 37 pmol, or 92 pmol).

**Fig. 1D)** Mouse sera were collected at day 32, and ACE2 blocking-of-binding to RBD was assessed by ELISA. Optical density for reciprocal serum dilutions and area above curve (AAC) values are shown.

**Fig. 1E)** Dose-response trend of data shown in c when normalized to the % of masked RBM.

**Fig. 1F)** Comparative analysis of cAbs secretion at day 4 (day 32) and 21 (day 49) after 2nd immunization.

**Fig. 1G)** Dropmap analysis of splenocytes at day 49 after full spike immunizations (immunized with 3 μg/immunization, 18 pmol) with or without mhACE2 (0 pmol, 4 pmol, or 92 pmol).

For all experiments shown in Fig. 1 N= 3 mice were used per condition, statistical analyses by two-sided independent Students t-test (***p < 0.001, **p < 0.01 and *p < 0.05). In c, e-f graphs show mean frequencies +/- SD.
This Example has been performed as described for Example 5 with the exception that additionally the ACE2 blocking of binding potential was determined for mouse sera:
Serial dilutions of sera in PBS 1% BSA were added to RBD-coated 96-well plates. Recombinant RBD was immobilized on a high-binding 96 well ELISA plate (Corning, #CLS3690) at 4 μg/ml in PBS (Sigma Aldrich, MO, USA) overnight at +4°C. Plates were blocked for 1h with 1% BSA (Thermo Fisher, Gibco, MA, USA) in PBS at room temperature. After 1h of incubation at room temperature, inhouse produced biotinylated ACE2-hFcg1 was added to a final effective concentration 70 (EC70) in PBS 1% BSA. After another hour at room temperature, plates were incubated with an AP-coupled streptavidin (Southern Biotech, #SBA-7100-04) at a 1:500 dilution in PBS 1% BSA to detect biotinylated ACE2-hFcg1 that was not prevented by serum antibodies from binding to RBD. 50% of maximum ACE2-blocking potentials (BD50) were determined by sigmoid curve fitting with non-linear regression performed in R (stats package). The area under the curve or above curve was determined by GraphPad Prism software.
Upper and lower plateaus of the non-biotinylated ACE2-hFcg1 controls served as a reference.

*Example 2*

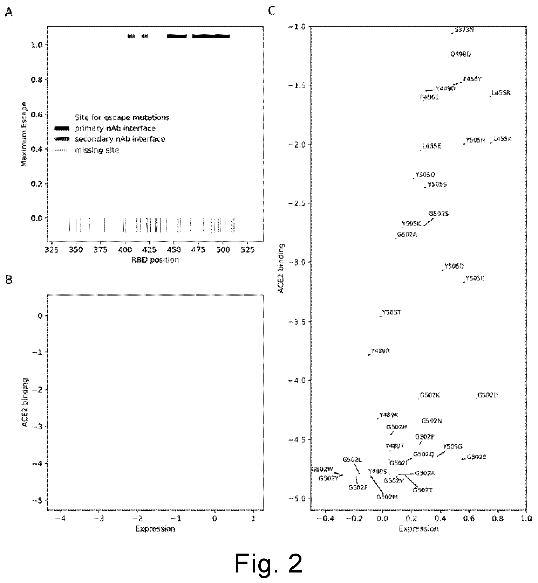

*In silico Identification of SARS-mRBDs with reduced ACE2 binding and preserved binding of SARS-wtRBD specific nABs by using bioinformatics analysis - Figs. 2A, B and C*

The method is particularly useful for producing a protein variant antigen or peptide antigen capable to induce a more potent immune response because the antigen is prevented from binding to or from being masked by membrane bound receptors or soluble receptors, respectively. The antigen is furthermore designed to maintain crucial epitopes to elicit a neutralizing immune response specific for the immunogen.
Production of a "demasking"-antigen comprises the step of introducing of a minimal number of amino acid exchanges in the antigen to prevent binding to receptors expressed in humans or animals, and it comprises the step of screening for preservation of binding to neutralizing antibodies or sera of convalescent donors.
An immunogen is selected and its receptor binding motif is identified for instance by structural studies or by antigen mutagenesis. The receptor binding motif is the direct binding interface between the receptor and the antigen. If applicable, multiple receptor binding motifs are identified. For example, MERS-CoV not only recognizes DPP4 but also sialic acids with two distinct RBMs (https://www.pnas.org/content/114/40/E8508).

A deep mutational scan of the RBM is performed on a platform that couples genotype-to-phenotype (https://www.nature.com/articles/nmeth.3027.pdf). Such a platform includes cell-based assays, where a protein is expressed from a plasmid or a virus, or an *in vitro* system such as T7 or M13 bacteriophage display, ribosome display, E. coli display, or most preferably, a mammalian cell display. A gene-library of mutated variants of the antigen are synthesized and cloned into appropriate plasmids. The mutant libraries can for instance be generated by overlap extension PCR (https://www.sciencedirect.com/science/article/pii/S0022283613004300?via%3 Dihub), by error-prone PCR (https://link.springer.eom/protocol/10.1385/1-59259-395-X:3) or by chemical mutagenesis (https://academic.oup.com/nar/article/32/4/1448/1038612?login=true). Some strategies create exactly one codon mutation per gene (https://journals.plos.org/plosone/article?id=10.1371/journal.pone.0052031 ; https://www.sciencedirect.com/science/article/pii/S0003269713005782?casa t oken=d5JPaeUuTwYAAAAA:-F2ChRH8nmjiVhQYPsl1BF4JpK-m E0CoVBibRWGkDKxCPd7D3IVxW7n-f7rRFCxRPGYJc8Q). The designs can be adjusted to multiple codon mutations per gene to examine the average effects of mutations (https://www.ncbi.nlm.nih.gov/pmc/articles/PMC4104320/). Mutagenesis can be improved by designing primers with equal melting temperatures to prevent a bias by certain mutations

(https://www.sciencedirect.com/science/article/pii/S1931312817301968). For SARS-CoV-2 RBD, deep mutational scanning has been applied by expressing a RBD-mutant library in yeast (https://www.sciencedirect.com/science/article/pii/S0092867420310035). Yeast expression systems are suitable if the antigen does not contain glycosylations within or close to the receptor binding motif. If glycosylations impact antigen-host receptor binding, expression in mammalian cells is preferred. For the generation of human vaccines, human cell lines are most preferred. Similarly, for the generation of porcine vaccines porcine cell lines are most preferred.

Screening of the library can be performed by flow cytometry cell sorting after transfection or transduction of cells with the mutant library. The antigen is cloned into a backbone that allows membrane expression and may be tagged by a fluorescent reporter such as GFP to (i) pre-select antigen expressing cells, and (ii) determine the expression level of the antigen. Transfection conditions are preferred to obtain a single coding variant per cell (https://www.jimmunol.org/content/200/11/3825, https://www.jbc.org/article/S0021-9258(20)35531-9/fulltext, https://science.sciencemag).org)/content/369/6508/1261). The FACS selection is performed by staining the cells with the soluble receptor that binds to the RBM expressed on the cell surface. The soluble receptor can be either directly labelled with a fluorophore or it can be biotinylated and stained with streptavidine-coupled-fluorophore, or it can be tagged with a his-tag, a strep-tag, or a myc-tag followed by staining with a secondary agent detecting the tag. Preferably, the protein is tagged with a Twin-Strep-Tag, and stained with a StrepTactin-coupled fluorophore. Even more preferably, the protein is directly labeled with a fluorophore. In a first step, cells are stained with the soluble receptor and are sorted by a suitable method, for instance by FACS sorting. Binding of the soluble receptor is detected by a fluorophore that enables separation from the reporter protein signal fused to the RBM. Cells will be positively selected to express the RBM-protein/peptide fused to the fluorescent reporter such as GFP. Furthermore, cells will be selected for having lost the binding to the soluble receptor.

As both, the soluble receptor and neutralizing antibodies bind to the RBM, selection for preservation of epitopes recognized by neutralizing antibodies is preferably performed after selecting RBM-variants with reduced binding to the soluble receptor.

In a second step, sorted cells with reduced binding to the soluble receptor will be stained with antibodies recognizing the RBM. Thereby, the sub-library of RBM-mutants is positively selected for having preserved epitopes that are bound by neutralizing antibodies. Sub-library cells are stained with one or, more preferably, multiple recombinant neutralizing antibodies. Most preferentially, an equimolar mixture of a set of neutralizing antibodies is used, which target distinct RBM epitopes. Optimally, the neutralizing antibodies are affinity matched, if not, the antibody mixture is adjusted to account for the distinct binding strengths. The sum of the neutralizing antibodies should cover the entire RBM. The neutralizing antibodies can either be labelled individually with distinct fluorophores, or with biotin to detect antibody binding in bulk. Likewise, antibody binding can be detected by an antibody-specific secondary agent. If recombinant neutralizing antibodies are not available, the screening can be performed by staining cells with sera of convalescent donors (human or immunized animals) and detection of bound antibodies by suitable secondary reagents.

If a transient expression system is used, binding of distinct recombinant neutralizing antibodies or convalescent plasmas are tested in a single staining. If a stable expression system is used, a sub-library can be cultivated and expanded after being sorted for loss-of-receptor binding. The sub-library can be further cultivated in bulk or it can be sub-cloned, for instance as single cells to grow monoclonal cell lines. Thereafter, cells stably expressing the selected RBM-mutants can be stained with a set of recombinant neutralizing antibodies or convalescent sera. Preferably, stainings with distinct recombinant neutralizing antibodies are performed in multiple individual stainings. Mutant antigens expressed by cells that maintain binding of neutralizing antibodies are positively selected. Most preferably, binding of all selected neutralizing antibodies to the mutated RBM is preserved.

Selected cells after transient transfection are sorted directly into medium after the first staining/prior to the second staining and sorting. After the second sorting, cells are sorted into medium, or more preferably, directly into lysis buffer. Selected cells stably expressing the mutant-RBM can be expanded before analysis. Total gDNA or, more preferably, RNA is purified with a kit such as the RNA extraction kit (Quiagen) or the GeneJET RNA purification kit (Thermo Scientific). The target region of interest is amplified with gene specific primers spanning the mutated region spanning the entire mutant library. For RNA extractions, cDNA is reversely transcribed with specific primers using a high fidelity kit such as Transcriptor High Fidelity cDNA Synthesis Kit (Roche) or high fidelity Accuscript (Agilent). The diversified region of the RBM is PCR amplified by one or multiple fragments, depending on the size of the mutated region. PCR amplicons are flanked by adapters for high throughput sequencing, such as Illumina sequencing, containing the sequencing primer, a unique barcode and a flow-cell binding sequence. The amplicons are sequenced by high throughput sequencing that is chosen to suit the length of the amplified fragments and the number of expected mutants. For instance an Illumina MiniSeq, MiSeq, NextSeq, or HiSeq system can be used.

The data are analyzed by determining the frequencies of RBM-mutant variants in the transcripts, comparing their frequency in the selected library to their frequency in the naive vector library. Thereby, a change in the frequency from input to selection for each variant serves as a measure for its ability to abrogate receptor binding and for its ability to preserve neutralizing antibody binding.

The mutant library screening serves as a basis for selecting mutants with minimal binding to the host receptor and for preservation of binding to neutralizing antibodies. In addition to data obtained from mutant-library screenings, their analysis can be combined with structural information on the receptor in complex with its antigen. Furthermore, the analysis can be combined with structural information of neutralizing antibodies in complex with the antigen. The extended bioinformatic analysis thereby supports identification of the most suitable mutations that minimize binding to the host receptor while maximizing the preservation of neutralizing antibodies.

The screening data provides a relationship between a single or multiple residues, in a RBM sequence, on expression/stability of the protein (E, expression), the affinity to the receptor (B, binding) and binding to neutralizing antibodies (N, binding). The RBM-variants are chosen such that E is maximal, while B is minimal, and N is maximal. In addition, the residue of choice should vary in a significant way in the physical interface of the receptor-antigen-structure. Furthermore, the residue of choice should not vary in a significant way in the physical interface of any structure of a neutralizing antibody in complex-with the antigen. Most optimally, the residue of choice is not targeted by any known neutralizing antibody and is also not a target of serum antibodies of convalescent individuals.

In the most simple scenario, scanning of the mutant library reveals the consequences of all single mutations. In addition, libraries can be adapted to study the effect of multiple mutations in combination. To approach the latter bioinformatically, evolutionary couplings between sites by sequence covariation analysis can be estimated.

If RBM mutations are identified from the mutant library screening and/or from the bioinformatical screening, mutants can be selected by ELISA-binding or any other method suitably to determine the binding strength to its receptor or the neutralizing antibodies.

The binding strength of the RBM to the soluble receptor, which can for instance be measured by its Kd or by the EC50 (effective concentration with 50% of maximal binding), should be decreased in a way that the Kd or EC50 is above the serum concentration or tissue concentration of the respective soluble receptor.

**Bioinformatics approach**

The following describes a procedure for prioritizing stable primary sequence variants selection of an antigen to preserve binding to neutralizing antibodies while reducing binding to a specific binding protein. The method relies on data from deep mutational scanning experiments, an experiment that measures a fitness (for a particular selection assay, here binding and stability) for a library, S, of primary sequence variants in the target antigen, A.

**Procedure**

*Experimental data description*

The procedure relies on the following input from experiments:

1) One or more deep mutational scanning experiment with a fitness measure reflecting antigen stability. ($R_{stab}$)

2) One or more deep mutational scanning experiment with a fitness measure reflecting escape from one or more neutralizing antibodies. ($R_{nab}$)

3) One or more deep mutational scanning experiments with a fitness measure reflecting the binding to an endogenous binding partner. ($R_{endo}$)

4) One or more experimental structures, or computational models, of the antigen in complex with neutralizing antibodies.

5) One or more experimental structures, or computational models, of the antigen in complex with the endogenous protein partner, or the relevant domain binding the antigen.

Note: the sequence libraries in experiments 1-3 may or may not fully overlap in coverage. Experiments 4 and 5 are optional.

Experiments 1-3: These data give fitness measures for three different properties over a respective sequence library, S. We refer to the fitnesses of a sequence, s', from the respective library, S, as $R_{stab}(s)$, $R_{nab}(s)$, and $R_{endo}(s)$. The units of the fitnesses are:

1. $R_{stab}$ — log-scale difference in count, c, compared to a to reference antigen, r, for example by flouresence intensity. Expression: $Log(c(s))-Log(c(r))$

2. $R_{nab}$ — escape fraction specifying a count with decreased binding compared to a total count. Quantified by FACS.

3. $R_{endo}$ — log-scale difference in dissociation constant, K, compared to a reference antigen, r, for example by titration. Expression: $Log(K(s))-Log(K(r))$

Experiments 4-5: These experiments give us information about the spatial organization of the antigen with respect to neutralizing antibodies or the endogenous binding partner in the form of three-dimensional coordinates of the atomic positions. We define the *structural interface* to be any amino acid residue in the antigen that has one shortest distance to any atom in the endogenous partner of 0.5 nanometers or less. We define the epitope on the anti-gen for the neutralizing antibody in an identical manner. We define primary and secondary epitopes as follows: primary overlap directly with the structural interface, secondary overlap indirectly, antibody binding may obstruct ACE2 binding through excluded volume effects. From **Fig. 2A** we observe that that the structural information is redundant with other data given, and therefore also optional.

*Pre-processing and standardization*

We select data from experiments 1-3, based upon two constraints:

1. $R_{stab}$ of variant should be at least a factor of exp(-alpha) of wildtype

2. $R_{endo}$ of variant should be at most a factor of exp(-beta) of wildtype

The threshold values alpha and beta control how destabilizing and how strongly binding variants we want to consider in the scoring. Recommended values along with their, along with their interpretation are given in Table 1.

*Table 1, Threshold values for data selection. Recommended thresholds are marked with \*. For alpha smaller values are better and for beta larger values are better.*

| alpha | Interpretation | beta | Interpretation |
|---|---|---|---|
| 0 (>100%) | Only stabilizing | 0 (<100%) | Wildtype-like binding |
| 0.5 (>60%) | Mildly destabilizing * | 1.0 (<36%) | Low binding * |
| 1.0 (>36%) | Destabilizing | 2.0 (<13%) | Very low binding |
| 4.0 (>2%) | Very destabilizing/unstable | 4.0 (<2%) | No binding |

After selecting the variants which fulfill the constraints outlined above, we compute normalized fitness score by first subtracting the sample mean fitness, and then dividing by the sample standard deviation. Mapping all fitness values to a floating point value with zero mean and unit standard deviation. We refer to the corresponding normalized fitness sores as $N_{stab}(s)$, $N_{nab}(s)$, and $N_{endo}(s)$.

*Score computation*

To identify possible candidate variants to test experimentally we compute a summarizing score, based upon the information extracted above.

For every variant s', if defined, in the primary and secondary interfaces, compute the total score with measurements in experiments 1 and 3 we compute the total score.

$$S_{tot(s')} = N_{stab}(s') - N_{nab}(s') - N_{endo}(s').$$

Missing values for experiment 2 are set to 0 (The average under normalization). If primary and secondary interfaces are not defined, compute score for all variants s', fulfilling the constraints defined above. A larger sore is considered better.

*Identifying hotspots*

We identify hotspots by sorting the loci by their highest scoring variant. Negative scoring loci are discarded. A threshold, gamma (range from 0 to 1), represents the fraction of non-negative maximum total-scoring loci to exclude. We set gamma=0.40. A gamma=0.25 correspond to more hits but less specific, gamma=0.75 more specific but less hits.

*Selecting variants libraries*

Selection of a variant library for secondary validation can be done via two strategies: exploitation and exploration. The end-user can decide to explore the highest ranked locus fully, or decide to combine highly ranking variants from all top-ranking loci.

We suggest to first explore: take the highest ranked variant from each locus, and then exploit: investigate lower ranked variants for each locus in subsequent libraries.

```
In-silico CODE
 import pandas as pd
 import numpy as np
 import matplotlib as mpl
 import matplotlib.pyplot as pit
 import matplotlib.patches as patches
 #Load data
 DMS = pd.read_csv("Deep_Mutational_Scan_RBD_stability_ACE2binding.csv")
 dms2 = pd.read_csv("Tableofmutation_antibody-escape_fraction_scores.csv")
 # Identify missing sites
 missing_sites = np.setdiff1d(np.unique(DMS['site_SARS2'].to_numpy()),
 np.unique(dms2['site'].to_numpy()), )
 print(f"Number of sites missing nAb data, {len(missing_sites)}: "+
 str(missing_sites))
 missing_sites2 = np.setdiff1d(np.unique(dms2['site'].to_numpy()),
 np.unique(DMS['site_SARS2'].to_numpy()) )
 print(f"Number of sites missing ACE2 data, {len(missing_sites2)}: "+
 str(missing_sites2))
 # Primary and secondary interfaces determined based on nAb RBD crystal
 structures:
 # 6XC2, 6XCN,6XDG, 6XE1, 6XKQ,7CDI, 7CDJ, 7JMO,
 7JMP,7JMW,7JV2,7JV6,7JVA,7JW0,7K90,7LX5.
 from itertools import chain
 interface_residues = [(443, 463), (469, 507)]
 secondary_interface_residues = [(403, 410), (417, 423)]
 total_interface_range = list(chain(*[range(*r) for r in
 interface_residues+secondary_interface_residues]))
 # pre-proccessing
 antibody_condition, mutation, escape =
 (dms2['condition'].to_numpy(),dms2['wildtype'].to_numpy()+dms2['site'].to_num
 py().astype('str')+dms2['mutation'].to_numpy(),
 dms2['mut_escape'].to_numpy())
```

```
unique_abc = np.unique(antibody_condition)
all_mutations = DMS['mutation'].to_numpy()
all_expression = np.zeros(len(all_mutations)*len(unique_abc))
all_ace_binding = np.zeros(len(all_mutations)*len(unique_abc))
all_escape = np.zeros(len(all_mutations)*len(unique_abc))
j=0
for abc in unique_abc:
  idx_condition = antibody_condition==abc
  for i,m in enumerate(all_mutations):
    candidate=DMS.loc[np.where(DMS['mutation'].to_numpy()==m)[0]]
    all_expression[i+j*len(all_mutations)] = candidate['expr_avg'].to_numpy()
    all_ace_binding[i+j*len(all_mutations)] = candidate['bind_avg'].to_numpy()
    condition_selection = np.where(((mutation==m)*(idx_condition)))[0]
    if len(condition_selection)>0:
      all_escape[i+j*len(all_mutations)] = escape[condition_selection[0]]
    else:
      all_escape[i+j*len(all_mutations)] = np.nan
  j=j+1
finite_values = ~(~np.isfinite(all_expression)+~np.isfinite(all_ace_binding))
all_expression = all_expression[finite_values]
all_ace_binding = all_ace_binding[finite_values]
all_mutations = np.tile(all_mutations, len(unique_abc))[finite_values]
all_resid = np.array([int(a[1:-1]) for a in all_mutations])
all_escape = all_escape[finite_values]
_all_mutations = DMS['mutation'].to_numpy()
all_conditions = np.concatenate([np.tile(abc, len(_all_mutations)) for abc in
unique_abc ])[finite_values]
def standardize(x):
xhat=(x-x[np.isfinite(x)].mean())
return xhat/xhat[np.isfinite(xhat)].std()
def compute_score(expression, escape, binding):
escape_finite = np.isfinite(escape)
escape_impute = np.array(escape)
escape_impute[~escape_finite] = 0.
return expression - binding - escape_impute
#enforce constraints
alpha=0.5
beta=1.0
constraints_fulfilled = ((all_expression>-alpha).astype(int)*(all_ace_binding<-
beta).astype(int)).astype(bool)
#compute score on data fulfilling constraints
tot_score=compute_score(standardize(all_expression[constraints_fulfilled]),
standardize(all_escape[constraints_fulfilled]),
standardize(all_ace_binding[constraints_fulfilled]))
#identify hotspots
mutation_hotspots = {}
for i in np.argsort(tot_score)[::-1]:
y = all_mutations[constraints_fulfilled][i]
resi = all_resid[constraints_fulfilled][i]
  if int(y[1:-1]) in total_interface_range:
    if mutation_hotspots.get(resi):
      mutation_hotspots[resi].append([y,
      all_expression[constraints_fulfilled][i], all_ace_binding[constraints_fulfilled][i],
      tot_score[i]])
    else:
      mutation_hotspots[resi]=[[y, all_expression[constraints_fulfilled][i],
      all_ace_binding[constraints_fulfilled][i], tot_score[i]]]
      # Filtering of low-scoring variants
      max_total_scores = np.array([np.max([I[-1] for I in mutation_hotspots[key]]) for
      key in mutation_hotspots])
      argsort_total_scores = np.argsort(max_total_scores)[::-1] # decending scores
      by index
      positive_max_total_scores = max_total_scores[argsort_total_scores]>0.
```

```
scores_positive_and_sorted =
max_total_scores[argsort_total_scores][positive_max_total_scores]
gamma = 0.40
bisect_res = 1000
score_thres =
np.linspace(0,scores_positive_and_sorted[0],bisect_res)[np.argmin([(gamma-
np.mean(threshold>scores_positive_and_sorted))**2. for threshold in
np.linspace(0,scores_positive_and_sorted[0],bisect_res)])]
print(f"score threshold for gamma={gamma}: ", )
# Diagnostic plot
plt.plot(np.linspace(0,scores_positive_and_sorted[0],bisect_res),
[np.mean(threshold>scores_positive_and_sorted) for threshold in
np.linspace(0,scores_positive_and_sorted[0],bisect_res)])
plt.vlines([score_thres],ymin=0, ymax=[gamma],color='k')
plt.hlines([gamma],xmin=0, xmax=[score_thres],color='k')
plt.xlabel("score threshold")
plt.ylabel(r"$\gamma$")
def stringify_top_mutants(mutation_hotspots, key, site_variant_scores):
sorted_hits = np.argsort(site_variants_scores)[::-1]
hits, counts = np.unique([mutation_hotspots[key][yy][0] for yy in
sorted_hits], return_counts=True)
return ', '.join([hit+f"({count})" for hit,count in zip(hits,counts)])
top_mutants=[]
for i,key in enumerate(mutation_hotspots.keys()):
 if max_total_scores[i]>score_thres:
    site_variants_scores = [variant[-1] for variant in mutation_hotspots[key]]
    print(key,'\t', stringify_top_mutants(mutation_hotspots, key,
    site_variants_scores),'\t', np.round(max_total_scores[i],2))
    top_mutants.append(mutation_hotspots[key][np.argmax(site_variants_scores)][
    0])
    # generate plot summarizing selected variants in the context of all data and
    constraints.
    fig, ax = plt.subplots(4,1, figsize=(4,12), gridspec_kw={'height_ratios': [2,2,1,
    1]},constrained_layout=True )
    ax[0].scatter(dms2['site'].to_numpy(), dms2['site_max_escape'].to_numpy(),
    marker='.',alpha=0.1, label="Site for escape mutations")
    for i,ir in enumerate(interface_residues):
 if i==0:
    ax[0].hlines(1.05, *ir, color='k', lw=5, label="primary nAb interface")
else:
    ax[0].hlines(1.05, *ir, color='k', lw=5)
    for i,ir in enumerate(secondary_interface_residues):
 if i==0:
    ax[0].hlines(1.05, *ir, color='r', lw=5, label="secondary nAb interface")
else:
    ax[0].hlines(1.05, *ir, color='r', lw=5)
    ax[0].vlines(missing_sites, ymin=-0.08, ymax=0.0, color='r',lw=0.7,
    label="missing site" )
    ax[0].set_xlabel("RBD position")
    ax[0].set_ylabel("Maximum Escape")
    ax[0].legend()
    ax[1].scatter(DMS['expr_avg'].to_numpy(),
DMS['bind_avg'].to_numpy(),marker='.',c='k',alpha=0.5)
ax[1].scatter(all_expression[constraints_fulfilled][np.isin(all_mutations[constrain
ts_fulfilled], top_mutants)],
all_ace_binding[constraints_fulfilled][np.isin(all_mutations[constraints_fulfilled],
top_mutants)],marker='o',c='r',alpha=0.8)
    ax[1].set_xlabel("Expression")
    ax[1].set_ylabel("ACE2 binding")
rect = patches.Rectangle((-0.5, -5), 1.5, 4.00, linewidth=0, edgecolor='none',
facecolor='y', alpha=0.2)
    ax[1].add_patch(rect)
    ax[3].scatter(DMS['expr_avg'].to_numpy(),
```

```
DMS['bind_avg'].to_numpy(),marker='.',c='k',alpha=0.5)
ax[3].scatter(all_expression[constraints_fulfilled][np.isin(all_mutations[constrain
ts_fulfilled], top_mutants)],
all_ace_binding[constraints_fulfilled][np.isin(all_mutations[constraints_fulfilled],
top_mutants)],marker='o',c='r',alpha=0.8)
ax[2].scatter(DMS['expr_avg'].to_numpy(),
DMS['bind_avg'].to_numpy(),marker='.',c='k',alpha=0.5)
ax[2].scatter(all_expression[constraints_fulfilled][np.isin(all_mutations[constrain
ts_fulfilled], top_mutants)],
all_ace_binding[constraints_fulfilled][np.isin(all_mutations[constraints_fulfilled],
top_mutants)],marker='o',c='r',alpha=0.8)
ax[2].set_xlim(-0.5,1)
ax[2].set_ylim(-2.1,-1.2)
ax[2].set_xticklabels([])
ax[3].set_xlim(-0.5,1)
ax[3].set_ylim(-4.85,-4.6)
ax[3].set_xlabel("Expression")
ax[3].set_ylabel("ACE2 binding")
ax[2].set_ylabel("ACE2 binding")
for bm in top_mutants:
_idx = (DMS['mutation']==str(bm)).to_numpy().argmax()
ax[2].annotate(str(bm), (DMS['expr_avg'][_idx], DMS['bind_avg'][_idx] ),
va='center', ha='center')
for bm in top_mutants:
_idx = (DMS['mutation']==str(bm)).to_numpy().argmax()
ax[3].annotate(str(bm), (DMS['expr_avg'][_idx], DMS['bind_avg'][_idx] ),
va='center', ha='center')
for _,let in zip(ax, ["A","B","C"]):
_.text(-0.2,1.05, let, fontsize=16, transform=_.transAxes)
#plt.tight_layout()
plt.savefig("nAb_RBD_ACE2_DMS_Bloom.pdf")
RBD_WT_AA_Seq=''.join(DMS['wildtype'].to_numpy().reshape(-1,21)[:,0])
# algorithms and functions to generate csv of top-ranked variants
dna2aa = {
'ATA':'I', 'ATC':'I', 'ATT':'I', 'ATG':'M',
'ACA':'T', 'ACC':'T', 'ACG':'T', 'ACT':'T',
'AAC':'N', 'AAT':'N', 'AAA':'K', 'AAG':'K',
'AGC':'S', 'AGT':'S', 'AGA':'R', 'AGG':'R',
'CTA':'L', 'CTC':'L', 'CTG':'L', 'CTT':'L',
'CCA':'P', 'CCC':'P', 'CCG':'P', 'CCT':'P',
'CAC':'H', 'CAT':'H', 'CAA':'Q', 'CAG':'Q',
'CGA':'R', 'CGC':'R', 'CGG':'R', 'CGT':'R',
'GTA':'V', 'GTC':'V', 'GTG':'V', 'GTT':'V',
'GCA':'A', 'GCC':'A', 'GCG':'A', 'GCT':'A',
'GAC':'D', 'GAT':'D', 'GAA':'E', 'GAG':'E',
'GGA':'G', 'GGC':'G', 'GGG':'G', 'GGT':'G',
'TCA':'S', 'TCC':'S', 'TCG':'S', 'TCT':'S',
'TTC':'F', 'TTT':'F', 'TTA':'L', 'TTG':'L',
'TAC':'Y', 'TAT':'Y', 'TAA':'_', 'TAG':'_',
'TGC':'C', 'TGT':'C', 'TGA':'_', 'TGG':'W',
aa2dna = { #la
dna2aa[key]:key for key in dna2aa.keys() }
def translate(seq):
prot=""
for i in range(0,len(seq),3):
    prot=prot+dna2aa[seq[i:i+3]]
return prot
# reference sites
RBD_start = 954
RBD_start_Bloom = 954+36
RBD_end = 1623
RBD_end_Bloom = 1623-30
```

# 'wildtype' nucleotide sequence spike_reference_sequence="ATGTTTGTCTTCCTGGTCCTGCTGCCTCT-GGTC TCCTCTCAGTGCGTGAACCTGACTACTAGAACTCAGCTGCCTCCCGCTTACA CCAATAGCTTCACCAGGGGCGTGTACTATCCAGACAAGGTGTTTCGCAGCT CCGTGCTGCACTCCACACAGGATCTGTTTCTGCCCTTCTTTTCTAACGTGAC CTGGTTCCACGCCATCCACGT-GTCCGGCACCAATGGCACAAAGAGGTTCGA CAATCCAGTGCTGCCCTTTAACGATGGCGTGTACTTCGCCTC-CACCGAGAA GTCTAACATCATCCGCGGCTGGATCTTTGGCACCACACTGGACAGCAAGAC ACAGTCCCTGCTGATCGTGAACAATGCCACCAACGTGGTCATCAAGGTGTG CGAGTTCCAGTTTTGTAATGATC-CTTTCCTGGGCGTGTACTATCACAAGAAC AATAAGTCTTGGATGGAGAGCGAGTTTAGGGTG-TACTCTAGCGCCAACAATT GCACATTTGAGTATGTGAGCCAGCCATTCCTGATGGACCTGGAGGGCAAGC AGGGCAATTTCAAGAACCTGCGGGAGTTCGTGTTTAAGAATATCGATGGCTA CTTCAAAATCTACTCCAAGCACACCCCATCAACCTGGTGCGGGACCTGCC ACAGGGCTTCTCTGCCCTGGAGCCTCTGGTGGATCTGCCAATCGGCATCAA CATCACCAGGTTTCAGACACT-GCTGGCCCTGCACCGCAGCTACCTGACACC TGGCGACTCCTCTAGCGGATGGACCGCAGGAGCT-GCCGCCTACTATGTGG GCTACCTGCAGCCAAGGACCTTCCTGCTGAAGTATAACGAGAATGGCACCA TCACAGACGCAGTGGATTGCGCCCTGGACCCCCTGTCTGAGACAAAGTGTA CACTGAAGAGCTTTACCGTGGA-GAAGGGCATCTACCAGACAAGCAATTTCC GGGTGCAGCCCACCGAGTCCATCGTGAGATTTCCAAATAT-CACAAACCTGT GCCCCTTTGGCGAGGTGTTCAACGCCACCCGCTTCGCCAGCGTGTATGCCT GGAATAGGAAGCGCATCTCCAACTGCGTGGCCGACTATTCTGTGCTGTACA ACTCCGCCTCTTTCAGCACCTT-TAAGTGTTACGGCGTGAGCCCTACAAAGCT GAATGACCTGTGCTTTACCAACGTGTATGCCGATTCCTTCGT-GATCAGGGG CGACGAGGTGCGCCAGATCGCACCAGGACAGACAGGCAAGATCGCCGACT ACAATTATAAGCTGCCCGACGATTTCACCGGCTGCGTGATCGCCTGGAACT CTAACAATCTGGATAGCAAAGT-GGGCGGCAACTACAATTATCTGTACCGGCT GTTTAGAAAGTCTAATCTGAAGCCTTTCGAGAGGGACATCTC-CACAGAAATC TACCAGGCCGGCTCTACCCCATGCAATGGCGTGGAGGGCTTTAACTGTTAT TTCCCCCTGCAGTCCTACGGCTTCCAGCCTACAAACGGCGTGGGCTATCAG CCATACCGCGTGGTGGTGCT-GTCTTTTGAGCTGCTGCACGCACCAGCAACA GTGTGCGGACCTAAGAAGAGCACCAATCTGGTGAA-GAACAAGTGCGTGAAC TTCAACTTCAACGGCCTGACCGGCACAGGCGTGCTGACCGAGAGCAACAAG AAGTTCCTGCCCTTTCAGCAGTTCGGCCGGGACATCGCAGATACCACAGAC GCCGTGCGGGACCCCCAGAC-CCTGGAGATCCTGGACATCACACCTTGCTC CTTCGGCGGCGTGTCTGTGATCACACCTGGCACCAATACATC-CAACCAGGT GGCCGTGCTGTACCAGGACGTGAATTGTACCGAGGTGCCAGTGGCCATCC ACGCCGATCAGCTGACCCCCACATGGAGGGTGTATAGCACCGGCTCCAAC GTGTTCCAGACACGCGCCGGAT-GCCTGATCGGAGCAGAGCACGTGAACAA TAGCTACGAGTGCGACATCCCCATCGGCGCCGGCATCTGT-GCCTCCTATCA GACCCAGACAAACTCCCCTAGGAGAGCCCGGTCTGTGGCCTCCCAGTCTAT CATCGCCTACACCATGAGCCTGGGCGCCGAGAACAGCGTGGCCTATTCTAA CAATAGCATCGCCATCCCCAC-CAACTTCACAATCAGCGTGACCACAGAGAT CCTGCCTGTGAGCATGACCAAGACATCCGTGGACTGCACAATGT-ACATCTG TGGCGATTCCACCGAGTGCTCTAACCTGCTGCTGCAGTATGGCTCCTTTTGT ACCCAGCTGAATAGAGCCCTGACAGGCATCGCCGTGGAGCAGGACAAGAA CACACAGGAGGTGTTCGCCCAG-GTGAAGCAAATCTACAAGACCCCCCCTAT CAAGGACTTTGGCGGCTTCAACTTCAGCCAGATCCTGCCCGATC-CTTCCAA GCCATCTAAGCGGAGCTTTATCGAGGACCTGCTGTTCAACAAGGTGACCCT GGCCGATGCCGGCTTCATCAAGCAGTACGGCGATTGCCTGGGCGACATCG CAGCCCGGGACCTGATCTGCGCCCAGAAGTTTAATGGCCTGACCGTGCTGC CACCCCTGCTGACAGATGAGAT-GATCGCCCAGTATACATCTGCCCTGCTGG CCGGCACCATCACAAGCGGATGGACCTTCGGCGCAG-GAGCCGCCCTGCAG ATCCCCTTTGCCATGCAGATGGCCTACAGATTCAACGGCATCGGCGTGACC CAGAATGTGCTGTATGAGAACCAGAAGCTGATCGCCAATCAGTTTAACAGC GCCATCGGCAAGATCCAGGACTCTCTGTCCTCTACAGCCAGCGCCCTGGGC AAGCTGCAGGATGTGGTGAAT-CAGAACGCCCAGGCCCTGAATACCCTGGTG AAGCAGCTGAGCAGCAACTTCGGCGCCATCTCTAGCGTGCT-GAATGACATC CTGAGCCGGCTGGACAAAGTTGAGGCAGAGGTGCAGATCGACCGGCTGAT CACAGGCAGACTGCAGTCCCTGCAGACCTACGTGACACAGCAGCTGATCAG GGCAGCAGAGATCAGGGCCTCTGCCAATCTGGCCGCCACCAAGATGAGCG AGTGCGTGCTGGGCCAGTCCAA-GAGAGTGGACTTTTGTGGCAAGGGCTAC CACCTGATGAGCTTCCCACAGTCCGCCCCCCACGGCGTGGTGTT-TCTGCAC GTGACCTATGTGCCTGCCCAGGAGAAGAACTTCACCACAGCCCCAGCCATC TGCCACGATGGCAAGGCACACTTTCCCCGGGAGGGCGTGTTCGTGAGCAA CGGCACCCACTGGTTTGTGACACAGAGAAATTTCTATGAGCCTCAGATCATC ACCACAGACAATACCTTCGT-GAGCGGCAACTGTGACGTGGTCATCGGCATC GTGAACAATACCGTGTACGATCCTCTGCAGCCAGAGCT-GGACTCTTTTAAG GAGGAGCTGGATAAGTATTTCAAGAACCACACCAGCCCCGACGTGGATCTG GGCGACATCTCTGGCATCAATGCCAGCGTGGTGAACATCCAGAAGGAGATC GACAGGCTGAATGAGGTGGCCAAGAATCTGAACGAGAGCCTGATCGATCTG CAGGAGCTGGGCAAGTATGAG-CAGTCCGGCCGCGAGAACCTGTACTTCCA GGGAGGAGGAGGCTCTGGATATATCCCAGAGGCACCTCGGGAT-GGACAGG CCTACGTGAGAAAAGACGGCGAGTGGGTCCTGCTGAGTACCTTCCTGGGG CATCATCATCATCAC-

CATTAA"

# ’2-P variant nucleotide sequence spike_reference_sequence_2p="ATGTTTGTCTTCCTGGTCCTGCTGCCTCTG GTCTCCTCTCAGTGCGTGAACCTGACTACTAGAACTCAGCTGCCTCCCGCTT ACACCAATAGCTTCACCAGGGGCGTGTACTATCCAGACAAGGTGTTTCGCA GCTCCGTGCTGCACTCCACACAGGATCTGTTTCTGCCCTTCTTTTCTAACGT GACCTGGTTCCACGCCATCCACGTGTCCGGCACCAATGGCACAAAGAGGTT CGACAATCCAGTGCTGCCCTT-TAACGATGGCGTGTACTTCGCCTCCACCGA GAAGTCTAACATCATCCGCGGCTGGATCTTTGGCACCACACT-GGACAGCAA GACACAGTCCCTGCTGATCGTGAACAATGCCACCAACGTGGTCATCAAGGT GTGCGAGTTCCAGTTTTGTAATGATCCTTTCCTGGGCGTGTACTATCACAAG AACAATAAGTCTTGGATGGA-GAGCGAGTTTAGGGTGTACTCTAGCGCCAAC AATTGCACATTTGAGTATGTGAGCCAGCCATTCCTGATGGAC-CTGGAGGGC AAGCAGGGCAATTTCAAGAACCTGCGGGAGTTCGTGTTTAAGAATATCGATG GCTACTTCAAAATCTACTCCAAGCACACCCCCATCAACCTGGTGCGGGACC TGCCACAGGGCTTCTCTGCCCT-GGAGCCTCTGGTGGATCTGCCAATCGGCA TCAACATCACCAGGTTTCAGACACTGCTGGCCCTGCACCG-CAGCTACCTGA CACCTGGCGACTCCTCTAGCGGATGGACCGCAGGAGCTGCCGCCTACTAT GTGGGCTACCTGCAGCCAAGGACCTTCCTGCTGAAGTATAACGAGAATGGC ACCATCACAGACGCAGTGGATT-GCGCCCTGGACCCCCTGTCTGAGACAAAG TGTACACTGAAGAGCTTTACCGTGGAGAAGGGCATCTACCA-GACAAGCAAT TTCCGGGTGCAGCCCACCGAGTCCATCGTGAGATTTCCAAATATCACAAAC CTGTGCCCCTTTGGCGAGGTGTTCAACGCCACCCGCTTCGCCAGCGTGTAT GCCTGGAATAGGAAGCGCATCTCCAACTGCGTGGCCGACTATTCTGTGCTG TACAACTCCGCCTCTTTCAGCAC-CTTTAAGTGTTACGGCGTGAGCCCTACAA AGCTGAATGACCTGTGCTTTACCAACGTGTATGCCGATTCCT-TCGTGATCAG GGGCGACGAGGTGCGCCAGATCGCACCAGGACAGACAGGCAAGATCGCCG ACTACAATTATAAGCTGCCCGACGATTTCACCGGCTGCGTGATCGCCTGGA ACTCTAACAATCTGGATAGCAAAGTGGGCGGCAACTACAATTATCTGTACCG GCTGTTTAGAAAGTCTAATCT-GAAGCCTTTCGAGAGGGACATCTCCACAGAA ATCTACCAGGCCGGCTCTACCCCATGCAATGGCGTGGAG-GGCTTTAACTGT TATTTCCCCCTGCAGTCCTACGGCTTCCAGCCTACAAACGGCGTGGGCTAT CAGCCATACCGCGTGGTGGTGCTGTCTTTTGAGCTGCTGCACGCACCAGCA ACAGTGTGCGGACCTAAGAA-GAGCACCAATCTGGTGAAGAACAAGTGCGTG AACTTCAACTTCAACGGCCTGACCGGCACAGGCGTGCTGAC-CGAGAGCAAC AAGAAGTTCCTGCCCTTTCAGCAGTTCGGCCGGGACATCGCAGATACCACA GACGCCGTGCGGGACCCCCAGACCCTGGAGATCCTGGACATCACACCTTG CTCCTTCGGCGGCGTGTCTGT-GATCACACCTGGCACCAATACATCCAACCA GGTGGCCGTGCTGTACCAGGACGTGAATTGTACCGAGGT-GCCAGTGGCCA TCCACGCCGATCAGCTGACCCCCACATGGAGGGTGTATAGCACCGGCTCCA ACGTGTTCCAGACACGCGCCGGATGCCTGATCGGAGCAGAGCACGTGAAC AATAGCTACGAGTGCGACATC-CCCATCGGCGCCGGCATCTGTGCCTCCTAT CAGACCCAGACAAACTCCCCTAGGAGAGCCCGGTCTGT-GGCCTCCCAGTCT ATCATCGCCTACACCATGAGCCTGGGCGCCGAGAACAGCGTGGCCTATTCT AACAATAGCATCGCCATCCCCACCAACTTCACAATCAGCGTGACCACAGAG ATCCTGCCTGTGAGCATGACCAA-GACATCCGTGGACTGCACAATGTACATCT GTGGCGATTCCACCGAGTGCTCTAACCTGCTGCTGCAGTAT-GGCTCCTTTT GTACCAGCTGAATAGAGCCCTGACAGGCATCGCCGTGGAGCAGGACAAG AACACACAGGAGGTGTTCGCCCAGGTGAAGCAAATCTACAAGACCCCCCT ATCAAGGACTTTGGCGGCTTCAACTTCAGCCAGATCCTGCCCGATCCTTCCA AGCCATCTAAGCGGAGCTTTATCGAGGACCTGCTGTTCAACAAGGTGACCC TGGCCGATGCCGGCTTCAT-CAAGCAGTACGGCGATTGCCTGGGCGACATC GCAGCCCGGGACCTGATCTGCGCCCAGAAGTTTAATGGCCT-GACCGTGCT GCCACCCTGCTGACAGATGAGATGATCGCCCAGTATACATCTGCCCTGCT GGCCGGCACCATCACAAGCGGATGGACCTTCGGCGCAGGAGCCGCCCTGC AGATCCCCTTTGCCATGCAGAT-GGCCTACAGATTCAACGGCATCGGCGTGA CCCAGAATGTGCTGTATGAGAACCAGAAGCTGATCGCCAAT-CAGTTTAACAG CGCCATCGGCAAGATCCAGGACTCTCTGTCCTCTACAGCCAGCGCCCTGGG CAAGCTGCAGGATGTGGTGAATCAGAACGCCCAGGCCCTGAATACCCTGGT GAAGCAGCTGAGCAGCAACT-TCGGCGCCATCTCTAGCGTGCTGAATGACAT CCTGAGCCGGCTGGACCCCCCAGAGGCAGAGGTGCAGATC-GACCGGCTGA TCACAGGCAGACTGCAGTCCCTGCAGACCTACGTGACACAGCAGCTGATCA GGGCAGCAGAGATCAGGGCCTCTGCCAATCTGGCCGCCACCAAGATGAGC GAGTGCGTGCTGGGCCAGTC-CAAGAGAGTGGACTTTTGTGGCAAGGGCTA CCACCTGATGAGCTTCCCACAGTCCGCCCCCCACGGCGTGGT-GTTTCTGCA CGTGACCTATGTGCCTGCCCAGGAGAAGAACTTCACCACAGCCCCAGCCAT CTGCCACGATGGCAAGGCACACTTTCCCCGGGAGGGCGTGTTCGTGAGCA ACGGCACCCACTGGTTTGTGACACAGAGAAATTTCTATGAGCCTCAGATCAT CACCACAGACAATACCTTCGT-GAGCGGCAACTGTGACGTGGTCATCGGCAT CGTGAACAATACCGTGTACGATCCTCTGCAGCCAGAGCT-GGACTCTTTTAAG GAGGAGCTGGATAAGTATTTCAAGAACCACACCAGCCCCGACGTGGATCTG GGCGACATCTCTGGCATCAATGCCAGCGTGGTGAACATCCAGAAGGAGATC GACAGGCTGAATGAGGTGGCCAAGAATCTGAACGAGAGCCTGATCGATCTG CAGGAGCTGGGCAAGTATGAG-CAGTCCGGCCGCGAGAACCTGTACTTCCA GGGAGGAGGAGGCTCTGGATATATCCCAGAGGCACCTCGGGAT-

GGACAGG CCTACGTGAGAAAAGACGGCGAGTGGGTCCTGCTGAGTACCTTCCTGGGG CATCATCATCATCAC-
CATTAA"

```
# Default output:
# 1. Nucleotide sequence RBD
# 2. Nucleotide sequence full spike (with 2-P mutation)
# 3. Aminoacid sequence RBD
# 4. Aminoacid sequence full spike (with 2-P mutation)
#
# return 2 dna seqs, 2 prot seqs
#
def build_sequences(wt_dna_full_spike, wt_dna_full_spike_2p, rbd_variant):
 #parse mutation
 origin_aa, site, target_aa = rbd_variant[0],int(rbd_variant[1:-1]), rbd_variant[-
 1]
 site_to_dna = (site-1)*3
 target_codon = aa2dna[target_aa]
 #apply mutation
 mutated_seq_ =
 wt_dna_full_spike[:site_to_dna]+target_codon+wt_dna_full_spike[site_to_dna+
 3:]
 mutated_seq_2p =
 wt_dna_full_spike_2p[:site_to_dna]+target_codon+wt_dna_full_spike_2p[site_t
 o_dna+3:]
 #translate sequences
 translated_sequence = translate(mutated_seq_[RBD_start:RBD_end])
 translated_sequence_2p = translate(mutated_seq_2p)
 return {"Variant_Nucleic_Acid_sequence_RBD":
 mutated_seq_[RBD_start:RBD_end], "Variant_Nucleic_Acid_sequence_2-
 P":mutated_seq_2p,
 "Variant_Amino_Acid_sequence_RBD":translated_sequence,
 "Variant_Amino_Acid_sequence_2-P":translated_sequence_2p[:-1]}
def aggregate(mut, scor, spike_reference=None, spike_reference_2p=None,
thres=0):
out = {}
sequences = {}
for m, s in zip(mut, scor):
   if s>thres:
      if out.get(m):
        out[m].append(s)
      else:
        out[m]=[s]
        _seqs = build_sequences(spike_reference,spike_reference_2p, m)
        for key in _seqs.keys():
           if sequences.get(key):
             sequences[key].append(_seqs[key])
           else:
             sequences[key]=[_seqs[key]]
for key in out.keys():
   out[key] = np.round(np.mean(out[key]),3)
return {**{"mutant":list(out.keys()), "scores":[out[s] for s in out.keys()]},
**sequences}
decending_sort_tot_score = np.argsort(tot_score)[::-1]
mutants = all_mutations[constraints_fulfilled][decending_sort_tot_score]
full_ranking=pd.DataFrame(aggregate(mutants,
tot_score[decending_sort_tot_score],
spike_reference=spike_reference_sequence,
spike_reference_2p=spike_reference_sequence_2p, thres=score_thres))
full_ranking.to_csv("top-variant-ranking_2.csv")
```

**Example data input**

Mutational scanning (MS) data (https://www.sciencedirect.com/science/article/pii/S0092867420310035 https://science.sciencemag.org/content/sci/early/2021/01/22/science.abf9302.fu ll.pdf) were used to correlate escape mutation loci to the structural epitope. Identification of primary and secondary interaction epitopes from the nAb:RBD-SARS-CoV-2 structures of the following neutralizing antibodies: REGN10933, REGN10987 (6XDG), C144 7K90, P2B-2F6 & P2B-2F11 (https://www.nature.com/articles/s41467-020-20501-9), CC12.1 6XC2, S2H13 7JV6, full spike; 7JV2, CC12.3 6XC4, C105 6XCN, CV30 6XE1, S2A4 7JVA, S304 7JWO, CV07-250 6XKQ, COVA1-16 Fab 7JMW, COVA2-39 7JMP, COVA2-04 7JMOWNb 2 and WNb 10 7LX5.

**Example results**

The results below are example outcomes of the analysis outlined above for the receptor binding domain of the SARS-CoV-2 virus' spike protein. Thresholds: alpha=0.5, beta=1.0, gamma=0.40.

*Table 2: Top loci along with top-scoring variants (number of neutralizing antibody conditions where variant scored well), and best score obtained for each locus; Hotspot loci and top variants.*

| Locus | Top Variants | Max. total score |
|---|---|---|
| 502 | G502A(3), G502C(3), G502D(3), G502E(3), G502F(3), G502H(3), G502I(3), G502K(3), G502L(3), G502M(3), G502N(3), G502P(3), G502Q(3), G502R(3), G502S(3), G502T(3), G502V(3), G502W(3), G502Y(3) | 4.79 |
| 505 | Y505A(3), Y505C(3), Y505D(3), Y505E(3), Y505G(3), Y505I(3), Y505K(3), Y505L(3), Y505M(3), Y505N(3), Y505Q(3), Y505R(3), Y505S(3), Y505T(3), Y505V(3) | 4.11 |
| 455 | L455D(3), L455E(3), L455K(3), L455R(3), L455Y(3) | 3.11 |
| 489 | Y489A(3), Y489C(3), Y489E(3), Y489I(3), Y489K(3), Y489L(3), Y489M(3), Y489N(3), Y489P(3), Y489Q(3), Y489R(3), Y489S(3), Y489T(3), Y489V(3) | 3.04 |
| 456 | F456A(3), F456C(3), F456E(3), F456G(3), F456I(3), F456K(3), F456N(3), F456Q(3), F456R(3), F456S(3), F456T(3), F456W(3), F456Y(3) | 1.94 |
| 498 | Q498C(3), Q498D(3), Q498I(3), Q498K(3), Q498L(3), Q498V(3) | 1.61 |
| 486 | F486C(3), F486D(3), F486E(3) | 1.28 |
| 449 | Y449A(3), Y449C(3), Y449D(3), Y449E(3), Y449F(3), Y449G(3), Y449H(3), Y449I(3), Y449L(3), Y449M(3), Y449N(3), Y449P(3), Y449Q(3), Y449S(3), Y449T(3), Y449V(3), Y449W(3) | 1.24 |

*Table 3: Detailed ranking indicating a score for each locus*

| *mutant* | *scores* |
|---|---|
| *G502E* | *4.793* |
| *G502D* | *4.708* |
| *Y505G* | *4.11* |
| *G502P* | *3.548* |
| *Y505E* | *3.47* |
| *G502T* | *3.391* |
| *G502N* | *3.389* |
| *G502Q* | *3.318* |
| *G502R* | *3.195* |
| *G502V* | *3.195* |

(continued)

| mutant | scores |
|--------|--------|
| G502K | 3.145 |
| L455K | 3.11 |
| L455R | 3.047 |
| Y489S | 3.039 |
| G502I | 2.791 |
| Y505D | 2.791 |
| Y489T | 2.783 |
| Y505N | 2.719 |
| G502H | 2.692 |
| G502M | 2.453 |
| Y489K | 2.209 |
| G502L | 2.179 |
| G502F | 2.062 |
| F456Y | 1.936 |
| G502S | 1.937 |
| G502Y | 1.749 |
| G502W | 1.671 |
| Y505S | 1.629 |
| Y505Q | 1.586 |
| Q498D | 1.607 |
| L455E | 1.592 |
| S373N | 1.528 |
| Y505T | 1.475 |
| Y489R | 1.47 |
| Y505K | 1.36 |
| F486E | 1.241 |
| G502A | 1.249 |
| Y449D | 1.243 |

**Fig 2.** is a visual analysis of results on SARS-CoV-2 RBD. It shows, that bioinformatics analysis using a published SARS-COV-2 RBM mutant screening and published structures of neutralizing antibodies identifies RBM mutants of desired properties. Several amino acid positions can be mutated into distinct residues to minimize ACE2 receptor binding, maximize protein expression, and minimally disrupt binding of published neutralizing antibodies.

**Fig 2A)** Depiction of neutralizing antibody (nAb) maximum escape for distinct RBD position (locus, dots), compared to primary and secondary interfaces identified via crystal structures (black and grey lines). Missing data indicated with thin vertical lines.

**Fig 2B)** Scatter plot of Expression (x-axis) versus ACE2 binding (y-axis), all data shown with black dots. Highest scoring variants for each top locus are shown with grey dots.

**Fig 2C)** is a Zoom-in into lower right grey box of **Fig 2B).** Amino acid exchanges of candidates identified by the screening are depicted.

*Example 3*

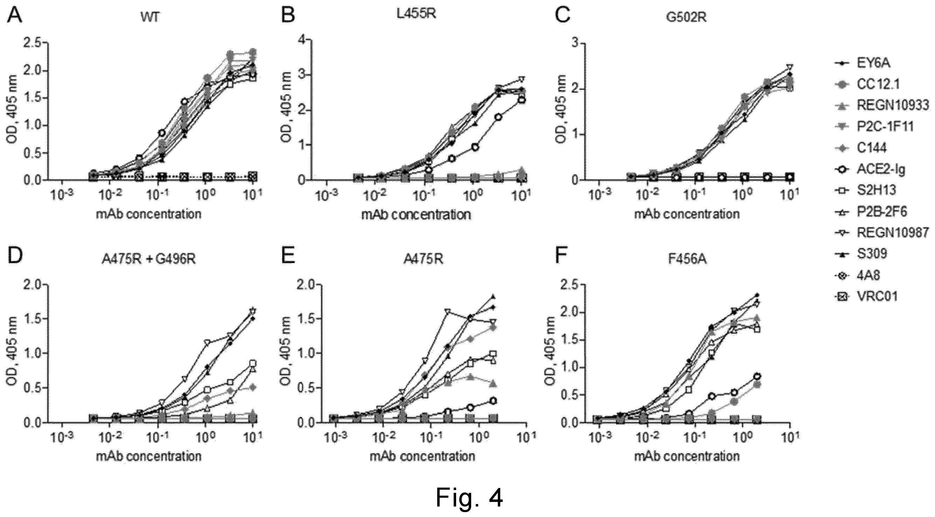

*Determination of the binding strengths in vitro of ACE2, different monoclonal nABs and different SARS-mRBDs having single, double, and triple amino acid substitutions by using ELISA — Figs. 4A-C5A-D*

Distinct single, double, and triple point mutations were introduced into the SARS-COV-2 RBD. The experiment has been performed as described in Example 4.

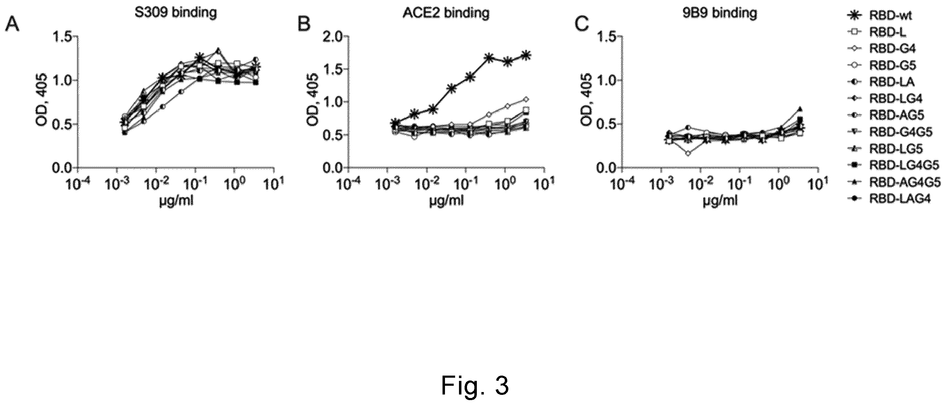

**Fig. 3A)** All mutations preserved binding to the neutralizing antibody S309, as determined by ELISA.

**Fig. 3B)** The majority of the variants largely or completely abrogated binding to ACE2 in ELISA.

**Fig. 3C)** The negative control antibody 9B9 of irrelevant specificity is not bound by the RBD variant, excluding nonspecific binding.

In **Fig. 3**, the abbreviations stand for:

| | |
|---|---|
| RBD-L: | RBD-L455R (SEQ ID NO: 55) |
| RBD-G4: | RBD-G496R (SEQ ID NO: 95) |
| RBD-G5: | RBD-G502R (SEQ ID NO: 15) |
| RBD-AG5: | RBD-A475R-G502R (SEQ ID NO: 96) |
| RBD-G4G5: | RBD-G496R-G502R (SEQ ID NO: 97) |
| RBD-AG4G5: | RBD-A475R-G496R-G502R (SEQ ID NO: 98) |
| RBD-LAG4: | RBD-L455R-A475R-G496R (SEQ ID NO: 99) |

This Example has been performed as described for Example 4 with the exception that 9B9 has been used a control antibody.

*Example 4*

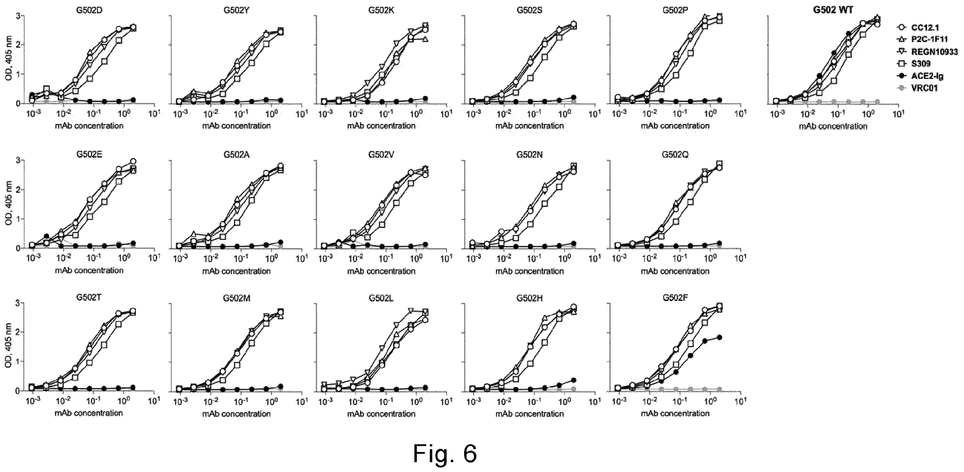

*Determination of the binding strengths in vitro of ACE2 and different monoclonal nABs to the SARS-wtRBD and different SARS-mRBDs bv using ELISA - Figs. 4A-F and Fig. 6, and*

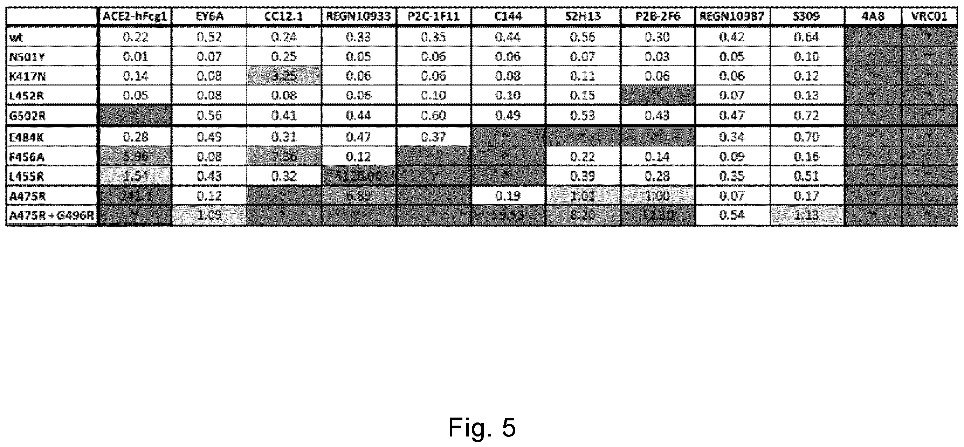

*Determination of EC50 values for different monoclonal nABS and ACE2 in view of the SARS-wtRBD and different SARS-mRBDs — Fig. 5*

**Fig. 4** confirms experimentally, that the bioinformatically screened RBM mutants L455R (SEQ ID NO: 55) and G502R (SEQ ID NO: 15) reduce ACE2 binding but preserve binding of RBD specific neutralizing antibodies. Similar for **Fig. 6**, where further G502-mutants G502D (SEQ ID NO: 5), G502Y (SEQ ID NO: 20), G502K (SEQ ID NO: 9), G502S (SEQ ID NO: 16), G502P (SEQ ID NO: 13), G502E (SEQ ID NO: 2), G502A (SEQ ID: 3) G502V (SEQ ID NO: 18), G502N (SEQ ID NO: 12), G502Q (SEQ ID NO: 14), G502T (SEQ ID NO: 17), G502M (SEQ ID NO: 11), G502H (SEQ ID NO: 7), G502L (SEQ ID NO: 10) and G502F (SEQ ID NO: 6) were experimentally screened. While G502R completely abrogates ACE2 binding in this ELISA based assay setup, L455R reduced ACE2 binding less well. In addition, the G502R mutation preserved binding of all tested neutralizing antibodies of 4 distinct classes namely Class-I (C12.1, P2C-1F11, REGN10933, C144) class-II, (C144, S2H13, P2B-2F6, REGN10987), Class-III (S309), and class IV (EY6A). No reduction of binding of neutralizing antibodies was observed for the G502R mutant. Furthermore, RBM mutants do not unspecifically recognize control antibodies VRC01 (anti-HIV GP140) or 4A8 (anti-SARS-CoV- spike N-terminal domain). The RBD mutations A475R+G496R, A475R and F456A reduce ACE2 binding, however binding of neutralizing antibodies is less well preserved as for L455R and G502R.

**Fig. 5** shows the effective concentration in $\mu$g/ml at which 50% of binding is achieved (EC50) for indicated neutralizing antibodies or ACE2-hFcg1 shown in **Figs. 5** low EC50 values indicate high binding affinities, ~ indicates that no binding was detected.

ACE2-hFcg1 was cloned by fusing recombinant human *ACE2* Q18-V739 fragment to human IgG1-Fc portion (E99-K330 portion, where 1st amino acid is G encoded by J-CH1 fusion). An RBD containing the signal peptide spanning amino acids M1-Q14 and R319-F541 of the Wuhan SARS-CoV-2 variant (GenBank: MN908947.3) was complemented by a Twin-Strep-tag sequence (WSHPQFEKGGGSGGGSGGSAWSHPQFEK) upstream of the C-terminal hexahistidine tag and cloned into the pcCDNA3.1 vector. Amino acid mRBD modifications, G502R, L455R, A475R, G496R, G502D, G502Y, G502K G502S, G502P, G502E, G502V, G502N, G502Q, G502T, G502M, G502H, G502L, G502F, were introduced by PCR mutagenesis to create a RBD-mutant with abrogated ACE2 binding (T. Zhou et al., 2020b). Likewise RBD mutations of novel coronavirus variants N501Y, K417N, E484K were generated. Heavy and light chain sequences of the following monoclonal antibodies were cloned into IgG1 heavy and kappa or lambda light chain expression vectors from Oxford Genetics: EY6A(D. Zhou et al., 2020a), P2B-2F6 and P2C-1F11 (Ge et al., 2021), REGN10933 and REGN10987(Hansen et al., 2020) CC12.1(Rogers et al., 2020), C144(Robbiani et al., 2020), VRC01(Wu et al., 2010), S2H13(Piccoli et al., 2020), S309(Pinto et al., 2020), 4A8(Chi et al., 2020).

For production of recombinant proteins, plasmids were used to transfect HEK293 cells that were grown in culture medium at 37°C in a humidified 8% $CO_2$ incubator. Cells were grown to a density of 2.5 million cells per mL, transfected using PEI (4 μg/mL in cell suspension) and DNA (1200 ng/ml in cell suspension), and cultivated for 3 days. The supernatants were harvested and proteins purified by His SpinTrap columns (Cytiva, 95056-290) or protein G columns according to manufacturer's instructions. The eluted protein was transferred to phosphate-buffered saline (PBS) via buffer exchange using Amicon Ultra-4 centrifugal filter units with 50 kDa cutoff (Millipore, UFC805008). Protein concentration was determined by His-tag specific ELISA using a mouse anti-His-tag antibody (Abcam, #ab18184) and a goat anti-mouse IgG Fc antibody conjugated to alkaline phosphatase (Southern Biotech, cat #SBA-1033-04) as detection reagent. Protein production was confirmed by SDS-PAGE and western blot using a mouse anti-His antibody (Abcam, #ab18184) and an IRDye 800CW donkey anti-mouse antibody (Li-Cor Biosciences, #925-32212).

Recombinant RBDs were immobilized on a high-binding 96 well ELISA plate (Corning, CLS3690) at 4 μg/ml in PBS (Sigma Aldrich, MO, USA) overnight at +4°C. Plates were blocked for 1h with 1% BSA (Thermo Fisher, Gibco, MA, USA) in PBS at room temperature. Recombinant antibodies and ACE2-hFcg1 were diluted in PBS 1% BSA to indicated serial dilutions, added to coated plates and incubated for 1h at room temperature. Plates were developed with an anti-human IgG-alkaline phosphatase (AP) coupled antibody (SouthernBiotech cat#2040-04) diluted 1:500 in PBS 1% BSA. Bicarbonate buffer with 4-Nitrophenyl phosphate disodium salt hexahydrate substrate (SIGMA, Cat No S0942-50TAB) was added and absorbance was measured at 405 nm in a Cytation 5 device (BioTek). Between all indicated incubation steps, plates were washed 3 times with PBS 0.1% Tween-20.

### Example 5

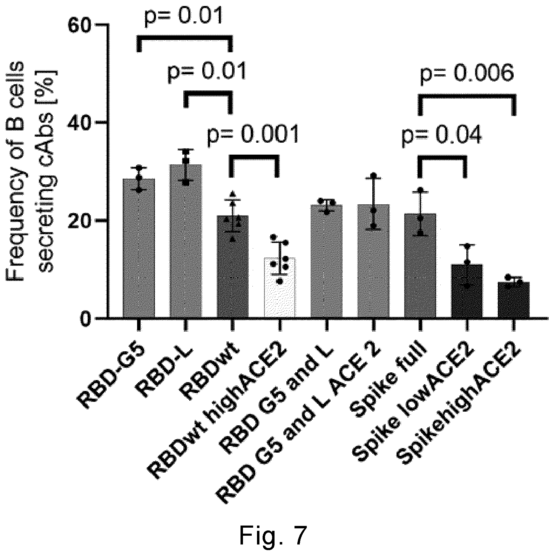

*In vivo determination of the frequency of B-cells following immunizations with either SARS-wtRBD or different SARS-mRBDs using a mouse model with controlled sACE2 levels — Fig. 7*

**Fig. 7** shows that in a controlled mouse *in vivo* model, masking of the RBD by soluble ACE2 during immunizations reduced the amount of B cells producing antibodies, which interfere with the RBD-ACE2 interaction (competing antibodies; cAbs). Furthermore, **Fig. 7** shows that RBD mutants G502R (SEQ ID NO: 15) (abbreviated in **Fig. 7** with RBD-G5) and L455R (SEQ ID NO: 55) (abbreviated in **Fig. 7** with RBD-L) can abrogate masking of the RBD by soluble ACE2 during in vivo immunizations. Therefore, introduced mutations improved the generation of B cells producing antibodies, which interfere with RBD-ACE2 interactions. Furthermore, **Fig. 7** shows that two RBD mutants G502R and L455R can be successfully combined in one immunization to prevent masking of soluble ACE2. Furthermore, **Fig. 7** shows that RBD masking also occurs during immunizations with the full-spike, which is a frequent antigen of SARS-CoV-2 vaccines, which were approved for treatment.

Immunization scheme: mice were immunized with RBD (wild type RBD [SEQ ID NO: 1], RBD mutant G502R [SEQ ID NO: 15] and RBD mutant L455R [SEQ ID NO: 55]) alone or RBD together with sACE2. Spleens were analyzed 4-21 days after booster immunization. Single cell technology (DropMap) was used to analyze antibodies secreted by single B cells in droplets. The percentage of B cells producing antibodies able to compete with ACE2 binding to RBD are shown. N= 3 mice were immunized for each condition.

Recombinant RBD-proteins were produced as described for **Fig. 4 and 5** (see Example 4).

Recombinant mhACE2 was constructed by introduction of the following mutations in the wild-type S19-V739 murine *ACE2* fragment: L20T, N24Q, N30D, N31K, Q34H, K60Q, S63N, E64N, Y73L, E74K, K78T, T79L, S82M, F83Y, T90N, P91L, I92T, I93V, S103N, H353K, N368D, R387A. MuranACE2 contained an N-terminal His tag and was cloned into mammalian expression pcDNA3.1 vector containing a signal peptide (SP) (MGWSCIILFLVATATGVHS) and a molecular tag composed of eight histidine moieties (His-tag) connected to the N-terminus of ACE2 via a GSSGSSGSS-linker. We determined experimentally that C-end His-tag is not suitable for efficient protein purification likely due to the cleavage by proteases such as ADAM17 expressed by HEK293 cells. Therefore, a His-tag was introduced between the SP and the N-terminus of the *ACE2* fragment.

Despite the RBD, wildtype full spike comprising a 2-P-mutation (substitutions K986P and V987P) (SEQ ID NO: 259) was used as an immunogen. The full length spike M1-Q1208 of the Wuhan SARS-CoV-2 variant (GenBank: MN908947.3) was cloned into the pcCDNA3.1 vector followed by the amino acid sequence SGRENLYFQGGGGGSGYIPE-APRDGQAYVRKDGEWVLLSTFLGHHHHHH* that contains a protease recognition site, a GGGS-linker, a trimerization domain and a hexahistidine tag.

In **Fig. 7,** the three bars from the right indicated with spike full, spike low ACE2 and Spike high ACE2 correspond to **Fig. 1G**, wherein low ACE2 is 18% RBM binding and high ACE2 is 100% RBM binding.

BALB/cJRj mice (age 6-8 weeks at start, all female) were purchased from Janvier Labs and housed in the animal facilities of ETH Zürich during experimentation. After two weeks of acclimatization, the mice were immunized at the indicated days (primary immunization day 0, booster immunization day 28) using RBD-His6 (final amount 3 $\mu$g/immunization, diluted in sterile PBS); adsorbed 1:1 to Alhydrogel® adjuvant 2% (vac-alu-250, InvivoGen) for 2 h at least. In indicated cases, muranACE2 (final amount 9.2, 1.8 or 0.37 $\mu$g/immunization, diluted in sterile PBS), neutralizing mouse IgG2b (final amount 13.5, 2.7 or 0.54 $\mu$g/immunization, 40592-MM57, Sino Biological, diluted in sterile PBS), SuperMuran ACE (final amount 0.37 $\mu$g/immunization, diluted in sterile PBS), NoMuran ACE2 (final amount 9.2 $\mu$g/immunization, diluted in sterile PBS), or full-length spike protein (final amount 3 $\mu$g/immunization, diluted in sterile PBS) were added to the antigen-adjuvant mixture shortly (<10 min) before injection. Mice were immunized *intraperitoneal* with a total volume of 100 $\mu$l of the respective antigen/adjuvant mixture. All immunizations were performed in triplicates. At the indicated time points after the boost, spleens were harvested and IgG-SCs prepared for encapsulation as described elsewhere(Bounab et al., 2020). In short, untouched cells from the B cell lineage were extracted using a Pan B cell purification kit II from Miltenyi. The cells were always kept on ice or at 4°C throughout the experiment. In parallel, whole blood was collected from the mice, allowed to coagulate and the serum was afterwards purified and stored at 4°C for subsequent titer measurements. The Cantonal ethics committee of Zurich validated and approved all the experiments described in this study under the license number ZH215/19.

The microfluidic polydimethylsiloxane chip for droplet generation and the 2D observation chamber were fabricated as described elsewhere(Bounab et al., 2020) and the assembly was used to generate droplets of a volume of around 50 pL. The emulsion was directly guided into the 2D observation chamber, and subsequent to complete filling the chamber was mounted onto an inverted fluorescence microscope (Ti2 Eclipse, Nikon), and the data was generated as described below.

The purified cells from B cell lineage were collected by centrifugation (400g, 5 min, 4°C) and re-suspended to a final concentration of $2 \times 10^6$ cells/ml in staining solution (Hanks' Balanced Salt Solution with added 5 $\mu$M of Cell Trace Violet, both ThermoFisher) to enable the selection of droplets containing B cells during analysis. Immediately before encapsulation, cells were collected (400g, 5 min, 4°C) and washed once in droplet media comprising of RPMI1640 without phenol red (cat.no. 11835030, ThermoFisher). Droplet media further contained 5% Knockout Serum Replacement (ThermoFisher), 0.5% recombinant human serum albumin (cat.no. A9986, SigmaAldrich), 25 mM 2-[4-(2-hydroxyethyl)piperazin-1-yl]ethanesulfonic acid (HEPES) pH 7.4, 1X Penicillin-Streptomycin and 0.1% Pluronic F-127 (all supplied by ThermoFisher). The cells were re-suspended in droplet media to achieve a final encapsulation $\lambda$ (mean number of cells per droplet) of 0.2-0.4; i.e., around 20-30% of droplets contained one individual cell.

Paramagnetic nanoparticles were prepared as described before(Bounab et al., 2020). Beads were prepared a few hours in advance, and the nanoparticles were re-suspended thoroughly by pipetting before each measurement. The final in-droplet concentrations of reagents were 25 nM *wt* RBD-His6, 25 nM AlexaFluor750 human ACE2-hFc (10108-H02H, Sino Biological, labeled in house), 30 nM AlexaFluor488 labelled anti-His6 (ab237336, Abcam) and 75 nM AlexaFluor647 labelled anti-IgG Fc (315-606-046, Jackson ImmunoResearch).

Images of droplets and encapsulated cells were acquired using a 10$\times$ objective (NA 0.45, Nikon, and an array of 10$\times$10 images was acquired after 1 hour of incubation at room temperature. This allowed to image roughly 4-50'000 droplets per experiment, and resulted in 10-20'000 analyzed cells. Excitation light was provided by a LED source (SOLA light engine, Lumencor Inc.), and the emitted fluorescence was recorded using appropriate band pass filters (DAPI, FITC, Cy5 and Cy7 filter sets, all Semrock), camera settings (Orca Flash 4, Hamamatsu) at room temperature (25°C) and ambient oxygen concentration. To align the bead lines during the measurements vertically (for data analysis), two strong neodymium magnets (BZX082, K&J Magnetics) were placed on each side of the 2D observation chamber.

Acquired data were analyzed using a custom-made Matlab script (Mathworks, a version can be found on GitHub under https://github.com/LCMD-ESPCI/dropmap-analyzer). In specific, bright-field images were used to detect and isolate the droplets, DAPI images were used to check for the presence of a living cell within each droplet, and all other fluorescence channels for relocation of fluorescence onto the beadline(Bounab et al., 2020). The resulting raw data were exported to Excel (Microsoft), and sorted for droplets that included a living cell. Droplets containing a cell were further sorted for an increase in anti-IgG relocation (threshold 1.3; corresponding to 1.1 nM IgG1 or 2.6 nM IgG2a/b). After assuring the presence of an IgG-secreting cell, relocation in Alexa488 (RBD, antigen) and Alexa750 (ACE2, competitor) were analyzed within the same droplet, and the ratio of Alexa488/750 (RBD/ACE2 relocation) was generated; and plotted against the relocation in Alexa488. At this stage, 50-600 IgG-secreting cells were selected in each sample. Competing antibodies were identified as ratios of RBD/ACE2 relocation higher than $\tilde{x}$(Alexa488 relocation$_{\text{empty droplets}}$/Alexa750 relocation$_{\text{empty droplets}}$)+0.02. Using this threshold, calibration samples with cAbs were reliably identified as cAbs (93 $\pm$ 8%, N= 6), whereas the number of falsely-identified cAbs remained low (1.4 $\pm$ 0.5%, N= 6). If not mentioned otherwise, the average of the frequency of identified cAbs measured in the individual mice is displayed (N= 3), and standard deviation is indicated in the **Fig. 7.** All measured frequencies were above the level of a negative control experiment (no added RBD 1.1 $\pm$ 1.1 %), although lower frequencies were not-significantly different, specifically high nAb and high muranACE2.

*Example 6*

*In silico modelling of epitope masking bv soluble ACE2 for newly emerging SARS-CoV-2 variants comprising a substitution N501Y — Figs. 8A and B*

The **Figs. 8A and B** show that ACE2 masking of the RBD increases for newly emerging virus variants.

*In silico* modelling of epitope masking represented as % of bound RBD in the presence of distinct ACE2 serum concentrations. The N501Y mutation occurring in newly emerging SARS-CoV-2 viruses from UK and South Africa increases affinity and therefore masking by ACE2 is also increased. The masking effect, here represented by 36% of bound RBD would increase with decreasing affinities (Kds), as shown in the correlation.

Therefore, masking potential correlates with affinity of the RBD against its receptor and novel variants with increased affinities will lead to an increased masking at lower sACE2 serum concentrations.

*Example 7*

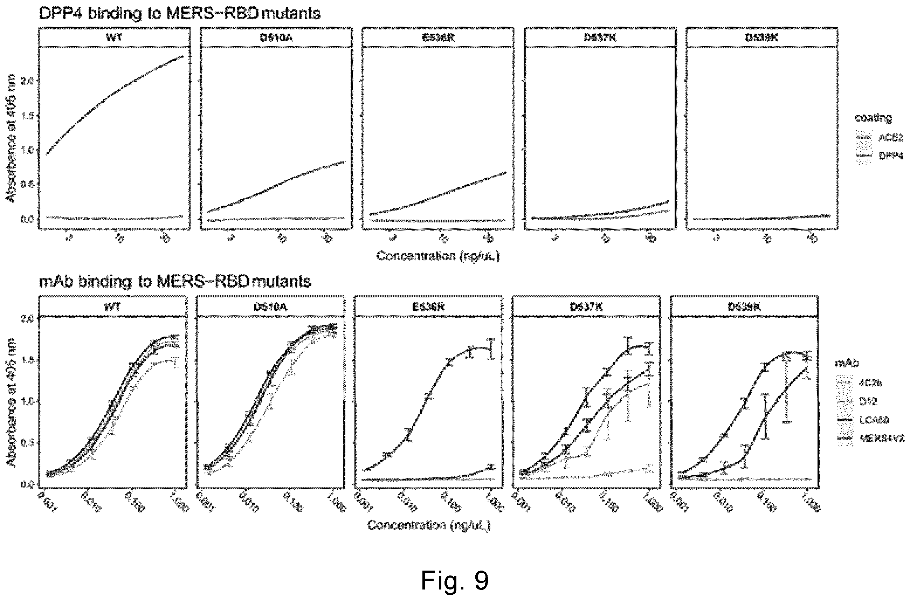

*Determination of the binding strengths in vitro of DPP4 and different monoclonal nABs to either the MERS-wtRBD or different MERS-mRBDs bv using ELISA — Fig. 9*

**Fig. 9** shows that MERS-CoV RBD mutations D510A (SEQ ID NO: 195), E536R (SEQ ID NO: 198), D537K (SEQ ID NO: 196), and D539K (SEQ ID NO: 197) abrogate binding to the DPP4 receptor to varying degrees, with D510 and D539 being the most effective loss-of-receptor-binding mutations. Furthermore, **Fig. 9** shows that binding of the MERS-CoV neutralizing antibodies 4C2h, D12, LCA60, and MERS4V2 are maintained by D510A and bind similarly well to RBD-D510A compared to RBD-WT. The D171K mutation preserved 3 of the 4 neutralizing antibodies, albeit with lower binding affinity. Therefore, D510A and D171K RBD mutants were selected for animal immunizations.

For cloning of human DPP4 and monoclonal antibodies, protein N-termini were preceded by an IgH signal peptide (SP) sequence, with the sequence MGWSCIILFLVATATGVHS, to facilitate protein secretion. Non-antibody proteins included either an 8xHis-tag or a 6xHis-tag on the N- or C-terminus for purification purposes. Gibson Assembly cloning was performed using the Gibson Assembly Master Mix (New England Biolabs, #E2611) according to the manufacturer's instructions. Chemically competent E. coli for transformation of plasmids were produced in-house and transformed conforming to the NEB transformation protocol (New England Biolabs, #E1601). Isolation of plasmids from bacterial cultures was done using the PureYieldTM Plasmid Miniprep System (Promega, #A1222) and PureYieldTM Plasmid Midiprep System (Promega, #A2496) according to the manufacturer's instructions. The sequences of the expression cassettes were verified by DNA sequencing (LGC Genomics GmbH).

The gene for recombinant DPP4 with an N-terminal 8xHis-tag (hDPP4-N'His) containing the S38-P766 fragment of human DPP4 (Uniprot, P27487) was synthesized by GenScript and cloned into mammalian expression pSF vector (Oxford Genetics) containing an SP using Gibson Assembly.

The MERS-CoV RBD amino acids G372-L588 were cloned in front of a GGGS-linker, upstream of a Twin Strep tag sequence (WSHPQFEKGGGSGGGSGGGSAWSHPQFEK) followed by a C-terminal 8xHis-tag. This was introduced into the mammalian expression vector pcDNA3.1+ containing the signal peptide spanning amino acids M1-Q14 of the Wuhan SARS-CoV-2 variant (GenBank: MN908947.3), an AviTagTM and a GG linker. Four amino acid modifications; D510A, E536R, D171K and D539K; were introduced by PCR mutagenesis (see table 4) to create six RBD-mutants with abrogated DPP4 binding using the Q5 Site-Directed Mutagenesis Kit (New England Biolabs, #E0554) according to the manufacturer's instructions.

*Table 4. Primer sequences for MERS-CoV RBD mutagenesis*

| Mutation | Primer | Sequence (5'-3') | SEQ ID NO: |
|---|---|---|---|
| D510A | MERS_RBD_D510A fw | GACGCCCGCACCGAGGTGCCCCAGC | 247 |
| D510A | MERS_RBD_D510A rev | AGACAGCAGCCTGGAGCACTTATTG | 248 |
| E536R | MERS_RBD_E536R fw | ACCGTGTGGAGGGACGGCGATTAC | 249 |
| E536R | MERS_RBD_E536R rev | AGAAGGCACGATGCTCACGC | 250 |
| D537K | MERS_RBD_D537K fw | GAGAAGGGCGATTACTATAGGAAGC | 251 |
| D537K | MERS_RBD_D537K rev | CCACACGGTAGAAGGCACGATG | 252 |
| D539K | MERS_RBD_D539K fw | GGCAAGTACTATAGGAAGCAGCTGAGC | 253 |

(continued)

| Mutation | Primer | Sequence (5'-3') | SEQ ID NO: |
|----------|--------|------------------|------------|
| D539K | MERS_RBD_D539K rev | GTCCTCCCACACGGTAGAAGGC | 254 |

Synthetic V(D)J inserts of monoclonal antibodies LCA60, 4C2h, D12 and MERS-4V2 were individually generated by overlapping PCR (see table 4-5). PCR products were recovered using ProNex beads (Promega, #NG2002) in agreement with the manufacturer's instructions. Subsequently, using Gibson Assembly, the PCR products were cloned into pSF expression vectors containing an SP and the Ig kappa constant region R1-C107 (light chain) or the IgG1 H constant region A1-K330 (heavy chain).

*Table 5. Primer sequences for generation of monoclonal antibody constructs by overlapping PCR*

| Antibody | Primer | Chain | Sequence (5'-3') | SEQ ID NO: |
|----------|--------|-------|------------------|------------|
| LCA60 | F1 | Heavy | CTGCAACCGGTGTACATTCTGAGGTGCAGCTGCTGGAGAGCGGCGGCGGCCTGGTGAAGCCCGGCGGCAGCCT | 206 |
| LCA60 | F2 | Heavy | GGTGAAGCCCGGCGGCAGCCTGAGGCTGAGCTGCGAGGCCAGCGGCCTGACCTTCAGCAACGTGTGGATGAGCTGGGTGAGGCAGGCCCCCGGCAAGGGCCTGGAGTGGGTGGGCAGGAT | 207 |
| LCA60 | R1 | Heavy | TGCAGGTACACGGTGTTCTTGCTGTCGTCCCTGCTCAGGGTGAACCTGCCCTTCACGGGGGCGCCGTAGTCGGTGGTGGCGCCCTCGCTCTTCCTCTTGATCCTGCCCACCCACTCCAGG | 208 |
| LCA60 | R2 | Heavy | CCAGTAGTCGAAGTAGTAGCTGCTGCTCCACACGTCGCCGCCCCTGGTCAGGGTGCTGCAGTAGTACACGGCGGTGTCGTCGATCTTCAGGCTGTTCATCTGCAGGTACACGGTGTTCTT | 209 |
| LCA60 | R3 | Heavy | CTAGGTCCCTTGGTCGACGCGCTGCTCACGGTCACCAGGGCGCCCTGGCCCCAGTAGTCGAAGTAGTAG | 210 |
| LCA60 | F1 | Light | CTGCAACCGGTGTACATTCACAGAGCGCCCTGACCCAGCCCGCCAGCGTGAGCGGCAGCCCCGGCCAGAGCATCACCATCAGCTGCACCGGCACC | 211 |
| LCA60 | F2 | Light | ACCATCAGCTGCACCGGCACCAGCAGCGACGTGGGCACCTACGACCTGGTGAGCTGGTACCAGCAGCACCCCGGCAAGAGCCCCAAGCTGATGATCTACGCCGACATCAAGAGGCCCAGC | 212 |

(continued)

| Antibody | Primer | Chain | Sequence (5'-3') | SEQ ID NO: |
|---|---|---|---|---|
| LCA60 | R1 | Light | CTGGGGGCGGCCACCGTACGGGTCACCTTGGTGCCGCCGCCGAAGATCACGCTGGTGCTGCTGCCGGCGTACAGGCAGCAGTAGTAGTCGGCC | 213 |
| LCA60 | R2 | Light | GGCAGCAGTAGTAGTCGGCCTCGTCGGCGCTCTGCAGGCCGCTGATGGTCAGGCTGGCGGTGTTGCCGCTCTTGCTGCCGCTGAACCTGTGGCTCACGCCGCTGGGCCTCTTGATGTCGG | 214 |
| 4C2h | F1 | Heavy | CTGCAACCGGTGTACATTCTCAGGTGCAGCTGCAGGAGAGCGGCGGCGGCCTGGTGAAGCCCGGCGGCAGCCTGAAGCTGAGCTGCGCCGCCAGCGGCTTCACCT | 215 |
| 4C2h | F2 | Heavy | GCGCCGCCAGCGGCTTCACCTTCAGCAGCTACACCATGAGCTGGGTGAGGCAGACCCCCGACAAGAGGCTGGAGTGGGTGGCCACCATCAGCAGCGGCGGCAGCTACACCTACTACCCCG | 216 |
| 4C2h | R1 | Heavy | CTAGGTCCCTTGGTCGACGCGCTGCTCACGGTCACCATGGTGCCCTGGCCCCAGTAGTCGTAGTCGTTGCCGTCCCTGGCGCAGTAGTACATGGCGGTGTCCTC | 217 |
| 4C2h | R2 | Heavy | TAGTACATGGCGGTGTCCTCGCTCTTCAGGCTGCTCATCTGCAGGTACAGGGTGTTCTTGGCGTTGTCCCTGCTGATGGTGAACCTGCCCTTCACGCTGTCGGGGTAGTAGGTGTAGCTG | 218 |
| 4C2h | F1 | Light | CTGCAACCGGTGTACATTCAGACATCCAGCTGACCCAGAGCCCCAGCAGCCTGAGCGCCAGCATCGGCGACAGGGTGACCATCACCTGCCAG | 219 |
| 4C2h | F2 | Light | AGGGTGACCATCACCTGCCAGGCCAGCCAGGACATCAGCAACTACCTGAACTGGTACCAGCAGAGGCCCGGCCAGGCCCCCAAGCTGCTGATCTACTACACCAGCAGGCTGCACAGCGGC | 220 |
| 4C2h | R1 | Light | CTGGGGGCGGCCACCGTACGCTTGATCTCCACCCTGGTGCCGCCGCCGAAGGTCCTGGGCAGGGTGTTGCCCTGCTGGCAGAAGTAGGTG | 221 |

(continued)

| Antibody | Primer | Chain | Sequence (5'-3') | SEQ ID NO: |
|---|---|---|---|---|
| 4C2h | R2 | Light | CCTGCTGGCAGAAGTAGGTGCCGATGTCCT CGGCCTGGAAGCTGTTGATGGCGATGGTGA AGCTGGTGCCGCTGCCCCTGCCGCTGAACC TGCTGGGCACGCCGCTGTGCAGCCTGCTGG | 222 |
| D12 | F1 | Heavy | CTGCAACCGGTGTACATTCTGAGGTGAAGCT GGTGGAGAGCGGCGGCGGCCTGGTGAAGC CCGGCGGCAGCCTGAAGCTGAGCTGCGCCG CCAGCGGCTTCACCT | 223 |
| D12 | F2 | Heavy | GCGCCGCCAGCGGCTTCACCTTCAGCAGCT ACGCCATGAGCTGGGTGAGGCAGACCCCCG AGAAGAGGCTGGAGTGGGTGGCCACCATCA GCAGCGGCGGCACCTACACCTACTACCCCG | 224 |
| D12 | R1 | Heavy | CTAGGTCCCTTGGTCGACGCGCTGCTCACG GTCACGCTGGTGCCCTGGCCCCAGTAGTCC ATGCTGTTGCCGTCCCTCACGCAGTAGTACA TGGCGGTGTCCTC | 225 |
| D12 | R2 | Heavy | TAGTACATGGCGGTGTCCTCGCTCCTCAGG CTGCTCATCTGCAGGTACAGGGTGTTCTCGG CGTTGTCCTGCTGATGGTGAACCTGCCCTT CACGCTGTCGGGGTAGTAGGTGTAGGTG | 226 |
| D12 | F1 | Light | CTGCAACCGGTGTACATTCAGACATCCAGAT GACCCAGACCACCAGCAGCCTGAGCGCCAG CCTGGGCGACAGGGTGACCATCATCTGCAG G | 227 |
| D12 | F2 | Light | AGGGTGACCATCATCTGCAGGGCCAGCCAG GACATCAACAACTACCTGAACTGGTACCAGA AGCCCGACGGCACCGTGAAGCTGCTGATCT ACTACACCAGCAGGCTGCACAGCGGC | 228 |
| D12 | R1 | Light | CTGGGGGCGGCCACCGTACGCCTCAGCTCC AGCTTGGTGCCGGCGCCGAAGGTGGGGGG CAGGGTGTTGGCCTGCTGGCAGAAGTAGGT G | 229 |
| D12 | R2 | Light | CCTGCTGGCAGAAGTAGGTGGCGATGTCCT CCTGCTCCAGGTTGCTGATGGTCAGGCTGTA GTCGCTGCCGCTGCCGCTGCCGCTGAACCT GCTGGGCACGCCGCTGTGCAGCCTGCTGG | 230 |

(continued)

| Antibody | Primer | Chain | Sequence (5'-3') | SEQ ID NO: |
|---|---|---|---|---|
| MERS-4V2 | F1 | Heavy | CTGCAACCGGTGTACATTCTGAGGTGCAGCTGGTGGAGAGCGGCGGCGGCCTGGTGCAGCCCGGCAGGAGCCTGAGGCTGAGCTGCGCCGCCAGCGGCT | 231 |
| MERS-4V2 | F2 | Heavy | TGAGCTGCGCCGCCAGCGGCTTCACCTTCAGCAACTACGCCATGTACTGGGTGAGGCAGGCCCCCGGCAAGGGCCTGGAGTGGGTGGCCCTGATCAGCTACGACATCAGCACCGACTACT | 232 |
| MERS-4V2 | R1 | Heavy | CTAGGTCCCTTGGTCGACGCGCTGCTCACGGTCACCAGGGTGCCCTGGCCCCAGTAGTAGGTGTTGGTGCAGTAGTACAGGGCGGTGTCCTCGGTCCT | 233 |
| MERS-4V2 | R2 | Heavy | AGGGCGGTGTCCTCGGTCCTCAGGTTGTTCATCTGCAGGTAGATGGTGTTCTTGCTGTTGTCCCTGCTGATGGTGAACCTGCCCTTCACGCTGTCGGCGTAGTAGTCGGTGCTGATGTCG | 234 |
| MERS-4V2 | F1 | Light | CTGCAACCGGTGTACATTCACAGAGCGTGCTGACCCAGAGCCCCAGCGCCAGCGGCACCCCCGGCCAGAGGGTGACCATCAGCTGCAGCGGCAGCA | 235 |
| MERS-4V2 | F2 | Light | CCATCAGCTGCAGCGGCAGCAGCAGCAACATCGGCAACAACTACGTGTACTGGTACCAGCAGCTGCCCGGCACCGCCCCCAAGCTGCTGATCTACTGGAACGACCAGAGGCCCAGCGGCG | 236 |
| MERS-4V2 | R1 | Light | CTGGGGGCGGCCACCGTACGCAGCACGGTCAGCTGGGTGCCGCCGCCGAACACGGCGCCGCTCAGGCTGTCGTCCCAGGCGGCGCAGTAGTAGTC | 237 |
| MERS-4V2 | R2 | Light | CAGGCGGCGCAGTAGTAGTCGGCCTCGTCCTCGCTCCTCAGGCCGCTGATGGCCAGGCTGGCGCTGGTGCCGCTCTTGCTGCCGCTGAACCTGTCGGGCACGCCGCTGGGCCTCTGGTCG | 238 |

*Table 6. Synthetic V(D)J inserts generated by overlapping PCR.* LCA60 sequences were obtained from PDB file 6nb3 (doi: 10.1016/j.cell.2018.12.028); 4C2h sequences were obtained from Li et al; 2015 (doi: 0.1038/cr.2015.113); D12 sequences were obtained from PDB file 4zpt (doi: 10.1038/ncomms8712) and MERS-4V2 sequences were obtained from Zhang et al.; 2018 (doi: 10.1016/j.celrep.2018.06.041).

| Monoclonal antibody | Heavy chain insert | SEQ ID NO: | Light chain insert | SEQ ID NO: |
|---|---|---|---|---|
| LCA60 | EVQLLESGGGLVKPGG SLRLSCEASGLTFSNV WMSWVRQAPGKGLE WVGRIKRKSEGATTDY GAPVKGRFTLSRDDSK NTVYLQMNSLKIDDTA VYYCSTLTRGGDVWS SSYYFDYWGQGALVT VSS | 239 | QSALTQPASVSGSPGQSI TISCTGTSSDVGTYDLVS WYQQHPGKSPKLMIYADI KRPSGVSHRFSGSKSGN TASLTISGLQSADEADYY CCLYAGSSTSVIFGGGTK VT | 240 |
| 4C2h | QVQLQESGGGLVKPG GSLKLSCAASGFTFSS YTMSWVRQTPDKRLE WVATISSGGSYTYYPD SVKGRFTISRDNAKNT LYLQMSSLKSEDTAMY YCARDGNDY DYWGQGTMVTVSS | 241 | DIQLTQSPSSLSASIGDRV TITCQASQDISNYLNWYQ QRPGQAPKLLIYYTSRLH SGVPSRFSGRGSGTSFTI AINSFQAEDIGTYFCQQG NTLPRTFGGGTRVEIK | 242 |
| D12 | EVKLVESGGGLVKPGG SLKLSCAASGFTFSSY AMSWVRQTPEKRLEW VATISSGGTYTYYPDS VKGRFTISRDNAENTL YLQMSSLRSEDTAMYY CVRDGNSMDYWGQG TSVTVSS | 243 | DIQMTQTTSSLSASLGDR VTIICRASQDINNYLNWYO QKPDGTVKLLIYYTSRLHS GVPSRFSGSGSGSDYSLT ISNLEQEDIATYFCQQANT LPPTFGAGTKLELR | 244 |
| MERS-4V2 | EVQLVESGGGLVQPG RSLRLSCAASGFTFSN YAMYWVRQAPGKGLE WVALISYDISTDYYADS VKGRFTISRDNSKNTIY LQMNNLRTEDTALYYC TNTYYWGQGTLVTVSS | 245 | QSVLTQSPSASGTPGQR VTISCSGSSSNIGNNYVY WYQQLPGTAPKLLIYWND QRPSGVPDRFSGSKSGT SASLAISGLRSEDEADYY CAAWDDSLSGAVFGGGT QLTVL | 246 |

The concentration ELISA aims to calculate the amount of protein that was produced using a recombinant purchased protein of similar size as a positive control. Two of the three concentration ELISAs that were used in this project are: DPP4-ELISA (measuring all human DPP4 proteins) and His-ELISA (measuring concentration of all His containing proteins (see Table 6)).

Firstly, protein concentration of self-made proteins was estimated by diving the absorption at 280 nm measured on a spectrophotometer (Biozym, #31DS-11FXPLUS) between theoretical extinction coefficient (ProtParam tool of Expasy, Swiss Institute of Bioinformatics; see Table 7. With this in mind, 96 well half-area plates (Greiner Bio-One GmbH, #675061) were coated with serial dilutions of self-made proteins, with a starting concentration of 1000 ng/ml for DPP4 concentration ELISAs or a 200-fold diluted sample for the His-concentration ELISAs. Standard proteins for the ELISAs were coated in the same plate as indicated in Table 7. Coated plates were incubated overnight at 4 °C.

*Table 7. Protein information. Extinction coefficient estimated by ProtParam tool of Expasy. *His tag is not detected in His ELISA*

| Protein | Information | Size | Extinction coefficient |
|---|---|---|---|
| MERS-CoV-RBD | Twin-Strep, His | ~ 32 kDa | 12250 |
| MERS-CoV-Spike | Twin-Strep, His | ~ 180 kDa | 77000 |
| MERS-CoV-RBD_D510A | Twin-Strep, His | ~ 32 kDa | 12250 |
| MERS-CoV-RBD_E536K | Twin-Strep, His | ~ 32 kDa | 12250 |
| MERS-CoV-RBD_D537K | Twin-Strep, His | ~ 32 kDa | 12250 |
| MERS-CoV-RBD_D539K | Twin-Strep, His | ~ 32 kDa | 12250 |
| MERS-CoV-Spike_D510A | Twin-Strep, His | ~ 180 kDa | 77000 |
| MERS-CoV-Spike_D537K | Twin-Strep, His | ~ 180 kDa | 77000 |
| BG505 | Twin-Strep, His | ~ 140 kDa | 147397 |
| DPP4 | His* | ~ 86 kDa | 26375 |
| SARS2-CoV-RBD | Twin-Strep, His | ~ 35 kDa | 14125 |
| SARS2-CoV-Spike | Twin-Strep, His | ~ 180 kDa | 70250 |
| LCA60 | IgG | ~ 150 kDa | ~210000 |
| D12 | IgG | ~ 150 kDa | ~210000 |
| MERS4V | IgG | ~ 150 kDa | ~210000 |
| h4C2 | IgG | ~ 150 kDa | ~210000 |

*Table 8. Material necessary to perform the DPP4- and His- concentration ELISAs*

| | DPP4 concentration ELISA | | His-concentration ELISA | |
|---|---|---|---|---|
| Positive control and starting concentration | recDPP4 (Sigma-Aldrich Chemie, #D3446) | 600 ng/ml | SARS-CoV-2 Spike protein (Biozol, #SIN-40592-V08B-100) | 4 ng/ml |

| Negative control | N´His-ACE2 (self-made) | 600 ng/ml | | |
|---|---|---|---|---|
| Primary antibody and concentration | Begelomab (self-made) | 10 ng/ml | Mouse anti 6x His tag (Abcam, #ab18184) | 1 µg/ml |
| Secondary antibody (AP-coupled) | Goat anti-human IgG Fc (Biozol, #SBA-2040-04) | 2 µg/ml | Goat anti-mouse IgG Fc (Biozol, #SBA-1033-04) | 2 µg/ml |

Plates were washed three times with PBS 1% Tween (PBS-T) in an automatized washer (Biotek, EL406). Plates were blocked with 25 µl of PBS 1% BSA for one hour at RT. The plates were washed three times with PBS-T and 25 µl of primary antibody (Table 7) was added to each well and incubated for 1 hour at RT. Plates were washed three times with PBS-T and 25 µl of secondary antibody (Table 7) was added to each well and incubated for 1 hour at RT. Plates were washed three times with PBS-T and 50 µl of 4-Nitrophenyl phosphate disodium salt hexahydrate (pNPP) (Sigma, #S0492-50TAB) was added. Exactly after 30 minutes of pNPP addition, absorbance of wells at 405 nm and 620 nm was measured (BioTek, Cytation 5).

Correlation of values obtained in positive controls with their actual concentration was used to calculate the concentration of the samples.

Concentration of self-made antibodies is determined using ELISA. Goat anti-IgG human (Southern Biotech, #2040-04) is used to coat a plate using 25 µl of 2 µg/ml. Plate is incubated at 4 °C overnight.

The plate is washed three times with PBS-T and block using 25 µl PBS 1% BSA for 1 hour at RT. The plate is washed three times with PBS-T and 25 µl of serial dilutions of the samples and positive control (Southern Biotech, #0150-01) at a starting dilution of 200-fold and 2 µg/ml, respectively, was added and incubated for 1 hour at RT. Then, the plate was washed three times with PBS-T and 25 µl of goat anti-human IgG (Southern Biotech, #2040-04) and incubated for 1 hour at RT. Plates were washed three times with PBS-T and 50 µl pNPP (Sigma, #S0492-50TAB) was added. Exactly after 30 minutes of pNPP addition, absorbance of wells at 405 nm and 620 nm is measured (BioTek, Cytation 5).

Binding of Twin Strep tagged MERS-CoV-RBD and MERS-CoV-spike to DPP4 was studied in an ELISA. 96 well half-area plates (Greiner Bio-One GmbH, #675061) were coated using 25 µl of 4 µg/ml self-made DPP4 diluted in PBS and incubated overnight at 4 °C.

Then plates were washed three times with 100ul/well PBS-T (Biotek, EL406). Plates were blocked with 25 µl of PBS 1% BSA for one hour at RT. The plates were washed three times with 100 ul/well PBS-T and 25 µl of serial dilutions of MERS-CoV-RBD or MERS-CoV-spike proteins was added to each well and incubated for 1 hour at RT. Serial dilutions were generated with a three-fold dilution with a starting concentration of 50 µg/ml Plates were washed three times with PBS-T and 25 µl of 2 µg/ml AP-coupled Streptavidin (Southern Biotech, #0150-01) was added to each well and incubated for 1 hour at RT. Plates were washed three times with PBS-T and 50 µL pNPP (Sigma, #S0492-50TAB) was added. Exactly after 30 minutes of pNPP addition, absorbance of wells at 405 nm and 620 nm is measured (BioTek, Cytation 5).

Increased absorbance at 405 nm indicated DPP4 binding to the sample Twin Strep tagged protein.

HEK293 were cultured in suspension at 37°C in a humidified 8% CO2 incubator. For protein production, cells were grown to a density of 2*106 cells per ml, transfected using 1 mg/ml PEI (Polysciences Inc., # 23966-1) and 38 µg plasmid DNA, and cultivated for 3 days. Following this, cells were centrifuged for 10 min at 4000 g at RT and the supernatant was collected. Supernatant was filtrated using 0.45 µm Polyethersulfone membranes. Proteins were purified from the resulting supernatant by His SpinTrapTM columns according to manufacturer's instructions (Cytiva, #95056-290). Eluted proteins were transferred to phosphate-buffered saline (PBS) via buffer exchange. Buffer exchange comprises six consecutive concentration and dilution steps at 4 °C in a centrifuge for 4-14 min at 4000 g using Amicon Ultra-4 centrifugal filter units with 10 kDa cutoff or 50 kDa (Millipore; #UFC801008, #UFC805008), for RBD proteins or hDPP4-N'His and monoclonal antibodies, respectively. Protein concentration was determined by ELISA for all proteins. Protein production was confirmed by SDS-PAGE and Western Blot.

*Example 10*

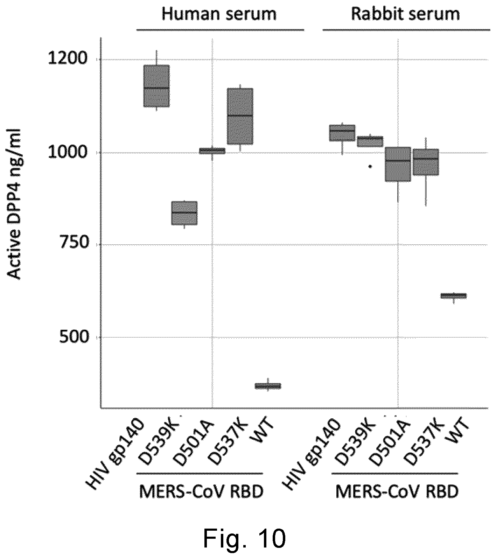

*Pull-down-assav of human DPP4 and rabbit DPP4 from serum by using either the MERS-wtRBD or different MERS-mRBDs — Fig. 10*

**Fig. 10** shows that MERS-CoV RBD mutations D510A (SEQ ID NO: 195), D537K (SEQ ID NO: 196), and D539K (SEQ ID NO: 197) abrogate the pull down of human and rabbit DPP4 from serum. Therefore, D510A, D537, and D539 mutations are suited to reduce masking of MERS-CoV RBD by serum DPP4.

Soluble DPP4 was pull down from 200 μl of serum using MERS-RBD-TwinStrep coupled to streptavidin magnetic beads (IBA Life Science, #2-4090-010). First, 120 μl of streptavidin magnetic beads was placed in a 1,5 ml tube. Using a magnetic rack, streptavidin magnetic beads were separated from the liquid. Liquid was removed and the pellets beads were washed twice with 1 ml 1×Wash buffer (IBA Life Science, #2-1003-100) and twice with 1 ml PBS. After washing, beads were resuspended in 50 μl of PBS.

TwinStrep containing proteins were coupled to streptavidin magnetic beads by addition of 24 μg of desired protein and incubation of the solution at 37°C for 1 hour shaking at 1400 rpm (Starlab, #S8012-0000). After 1 hour, beads were washed three times with 1 ml PBS using the magnetic rack to pellet and exchange the supernatant. Blocking of the beads was necessary to occupied potential empty spaces in the streptavidin magnetic beads. Blocking of the beads was done incubating the beads in 1 ml 1% BSA-PBS for 1 hour at RT shaking at 1400 rpm.

After the blocking step, beads were washed three times with 1 ml PBS and finally resuspended in 120 μl of PBS. 120 μl of beads was transferred to a 2 ml tube. 200 μl of serum was added to the 2 ml tube. The 2 ml tube was placed inside a 50 ml tube together with paper towels to ensure stability inside it. The 50 ml tube was incubated overnight at 4°C rolling.

Following the overnight incubation, 2 ml tubes are placed in the magnetic rack and serum is collected for further analysis. Pellet beads are washed three times with 1 ml of PBS and stored for further analysis.

Enzymatic activity of soluble DPP4 (sDPP4) in serum was measured as follows:

First, in a 96-well plate (Sigma, #M0812-100EA) 10 μL of serum samples were supplied with 80 μl assay buffer (50 mM TrisHCl, pH 9.0 at 37°C), which was pre-warmed at 37 °C. Second, the plate was incubated at 37°C for 15 minutes. Third, the substrate Gly-Pro p-nitroanilide (Biozol, #BAC-4025614.0250) was freshly prepared in assay buffer to a concentration of 5 mM. Then, 10 μl of substrate was added to each sample and samples were measured by the absorbance at 405 nm in a kinetic measurement at 37°C for 2 hours measuring every 15 minutes (BioTek, Cytation 5). To relate the absorbance to the production of p-nitroaniline (pNA) and subsequently to DPP4 concentration, pNA (Santa Cruz Biotechnology Inc, #sc-272000A) and recombinant DPP4 (SigmaAldrich Chemie, #D3446,) standards were included on the plate and prepared as indicated in table 4 and 5

*Table 9. p-nitroaniline standard pipetting protocol from left to right*

| Standard conc. in end volume (μM) | Vol. of standard stock solution (μl) | 1%PBS/BSA (μl) | Assay buffer vol. (μl) | Substrate vol. (μl) |
|---|---|---|---|---|
| 1200 | 24 | 10 | 56 | 10 |
| 1000 | 20 | 10 | 60 | 10 |
| 800 | 16 | 10 | 64 | 10 |
| 600 | 12 | 10 | 68 | 10 |
| 400 | 8 | 10 | 72 | 10 |
| 300 | 6 | 10 | 74 | 10 |
| 200 | 4 | 10 | 76 | 10 |
| 100 | 2 | 10 | 78 | 10 |

*Table 9. recombinant DPP4 standard pipetting protocol from left to right*

| Standard conc. in end volume (ng/ml) | Vol. of standard stock solution (μl) | 1%PBS/BSA (μl) | Assay buffer vol. (μl) | Substrate vol. (μl) |
|---|---|---|---|---|
| 210 | 17.5 | 10 | 62.5 | 10 |
| 180 | 15 | 10 | 65 | 10 |

(continued)

| Standard conc. in end volume (ng/ml) | Vol. of standard stock solution (μl) | 1%PBS/BSA (μl) | Assay buffer vol. (μl) | Substrate vol. (μl) |
|---|---|---|---|---|
| 150 | 12.5 | 10 | 67.5 | 10 |
| 120 | 10 | 10 | 70 | 10 |
| 90 | 7.5 | 10 | 72.5 | 10 |
| 60 | 5 | 10 | 75 | 10 |
| 30 | 2.5 | 10 | 77.5 | 10 |
| 0 | 0 | 10 | 80 | 10 |
| **Table 5. recombinant DPP4 standard pipetting protocol from left to right** | | | | |

The analysis of DPP4 activity assay is done entirely using RStudio.

Firstly, the mean of two blank values are subtracted from all of the values in a time dependent manner. Subsequently, the timepoint zero measurement is subtracted from every sample, avoiding sample fluorescence to interfere in further calculations.

Intercept-free linear regression of known concentrations of p-nitroaniline (pNA) versus the absorbance at 405 nm is performed. The data chosen for this analysis is the one belonging to the timepoint 45 minutes. The slope obtained in it, is then used to measure the amount of pNA that was being released during the analysis by every sample.

Activity of the control DPP4 samples (nmol pNA/min) are then analysed. The highest concentration used for DPP4 (0.3 μg/ml) show a linear increase of absorbance between 15-60 minutes. Therefore, optimal substrate cleavage for high as well as low concentrations of DPP4 takes place between 45-60 minutes. Later timepoints are not optimal for high concentrations of DPP4. By optimal substrate cleavage is meant that the r-squared of the linear regression of the concentrations of DPP4 versus activity is higher than 0.96. However, samples containing low amount of DPP4 should be measured at later timepoints; in which case, high concentrated control DPP4 samples must be filtered out until achievement of optimal substrate cleavage.

Intercept-free linear regression of known concentrations of DPP4 versus pNA released at optimal substrate cleavage is performed. The slope is used to measure the concentration of active DPP4 (aDPP4) in the samples.

## Claims

1. Mutant receptor-binding domain (mRBD) of a coronavirus (CORONA-mRBD) or a fragment thereof having a reduced binding strength to a RBD-receptor of the coronavirus (CORONA-RBD-receptor) compared to a wild type receptor-binding domain of the coronavirus (CORONA-wtRBD).

2. CORONA-mRBD or the fragment thereof according to claim 1, wherein

   (A)

       the coronavirus is SARS-CoV-2 (severe acute respiratory syndrome coronavirus type 2),
       the CORONA-mRBD is a mutant receptor-binding domain of SARS-CoV-2 (SARS-mRBD) or a fragment thereof,
       the wild type receptor-binding domain is SARS-wtRBD (wildtype receptor binding domain of SARS-CoV-2), and
       the RBD-receptor is ACE2 (angiotensin-converting enzyme 2); or

   (B)

       the coronavirus is MERS-CoV (middle east respiratory syndrome coronavirus),
       the CORONA-mRBD is a mutant receptor-binding domain of MERS-CoV (MERS-mRBD) or a fragment thereof,
       the wild type receptor-binding domain is MERS-wtRBD (wildtype receptor binding domain of MERS-CoV), and

the RBD-receptor is DPP4 (dipeptidylpeptidase 4).

3. CORONA-mRBD or the fragment thereof according to claim 1 or 2, wherein

(A) the SARS-mRBD or the fragment thereof comprises an amino acid sequence or a fragment thereof comprising one or more substitutions of amino acid residues at positions selected from G184, Y187, L137, Y171, F138, Q180, F168, Y131 or S55 of the SARS-wtRBD of SEQ ID NO: 1, wherein the SARS-mRBD or the fragment thereof has, except for the substitutions, an amino acid sequence identity of 85% or more to SEQ ID NO: 1; and/or
(B) the MERS-mRBD or the fragment thereof comprises an amino acid sequence or a fragment thereof comprising one or more substitutions of amino acid residues at positions selected from L140, D144, E170, D171 or D173 of the MERS-wtRBD of SEQ ID NO: 194 or the fragment thereof, wherein the MERS-mRBD or the fragment thereof has, except for the substitutions, an amino acid sequence identity of 85% or more to SEQ ID NO: 194.

4. CORONA-mRBD or the fragment thereof according to any of claims 1 to 3,

(A) wherein, for the SARS-mRBD or the fragment thereof, the substitution of the amino acid residue at the position:

- G184 is selected from G184A, G184C, G184D, G184E, G184F, G184H, G184I, G184K, G184L, G184M, G184N, G184P, G184Q, G184R, G184S, G184T, G184V, G184W or G184Y;
- Y187 is selected from Y187A, Y187C, Y187D, Y187E, Y187G, Y187I, Y187K, Y187L, Y187M, Y187N, Y187Q, Y187R, Y187S, Y187T or Y187V;
- L137 is selected from L137D, L137E, L137K, L137R or L137Y;
- Y171 is selected from Y171A, Y171C, Y171E, Y171I, Y171K, Y171L, Y171M, Y171N, Y171P, Y171Q, Y171R, Y171S, Y171T or Y171V;
- F138 is selected from F138A, F138C, F138E, F138G, F138I, F138K, F138N, F138Q, F138R, F138S, F138T, F138W or F138Y;
- Q180 is selected from Q180C, Q180D, Q180I, Q180K, Q180L or Q180V;
- F168 is selected from F168C, F168D or F168E;
- Y131 is selected from Y131A, Y131C, Y131D, Y131E, Y131F, Y131G, Y131H, Y131I, Y131L, Y131M, Y131N, Y131P, Y131Q, Y131S, Y131T, Y131V and Y131W; and/or
- S55 is S55N, and/or

(B) wherein, for the MERS-mRBD or the fragment thereof, the substitution of the amino acid residue at the position:

- L140 is L140A;
- D144 is D144A;
- E170 is E170R;
- D171 is D171K; and/or
- D173 is 173K.

5. CORONA-mRBD or the fragment thereof according to any of claims 1 to 4, wherein

(A) the SARS-mRBD or the fragment thereof comprises an amino acid sequence or a fragment thereof comprising one substitution of an amino acid residue at a position selected from G184 or L137 of the SARS-wtRBD of SEQ ID NO: 1 or the fragment thereof, wherein the substitution of the amino acid residue at the position:

- G184 is selected from G184R or G184D; and
- L137 is L137R; and/or

(B) the MERS-mRBD or the fragment thereof comprises an amino acid sequence or a fragment thereof comprising one substitution of an amino acid residue at a position selected from D144 and D171 of the MERS-wtRBD of SEQ ID NO: 194 or the fragment thereof, wherein the substitution of the amino acid residue at the position:

- D144 is D144A; and
- D171 is D171K.

6. CORONA-mRBD or the fragment thereof according to any of claims 1 to 5, wherein

(A) the SARS-mRBD or the fragment thereof comprises an amino acid sequence selected from even more preferably selected from SEQ ID NO: 15, SEQ ID NO: 17, SEQ ID NO: 5, SEQ ID NO: 20, SEQ ID NO: 9, SEQ ID NO: 16, SEQ ID NO: 13, SEQ ID NO: 2, SEQ ID NO: 3, SEQ ID NO: 18, SEQ ID NO: 12, SEQ ID NO: 14, SEQ ID NO: 11, SEQ ID NO: 7 or SEQ ID NO: 10; SEQ ID NO: 55 or SEQ ID NO: 30; and
(B) the MERS-mRBD or the fragment thereof comprises an amino acid sequence selected from SEQ ID NO: 195 or SEQ ID NO: 196.

7. Mutant spike protein of a coronavirus (CORONA-mSpike) or a fragment thereof comprising the CORONA-mRBD or the fragment thereof according to any of claims 1 to 6.

8. Polypeptide or protein comprising the CORONA-mRBD or the fragment thereof according to any of claims 1 to 6 or the CORONA-mSpike or the fragment thereof according to claim 7.

9. Nucleic acid comprising a nucleotide sequence encoding for:

- the CORONA-mRBD or the fragment thereof according to any of claims 1 to 6;
- the CORONA-mSpike or the fragment thereof according to claim 7; or
- the polypeptide or protein according to claim 7.

10. Vaccine composition comprising as an active ingredient:

- one or more CORONA-mRBDs or the fragments thereof according to any of claims 1 to 6; and/or
- one or more CORONA-mSpikes or the fragments thereof according to claim 7; and/or
- one or more polypeptides or proteins according to claim 8; and/or
- one or more nucleic acids according to claim 9.

11. One or more CORONA-mRBDs or the fragments thereof according to any of claims 1 to 6; and/or

one or more CORONA-mSpikes or the fragments according to claim 7; and/or
*one* or more polypeptides or proteins according to claim 8; and/or one or more nucleic acids according to any of claims 9; and/or
the vaccine composition according to claim 10,
for use in the prevention and/or treatment of diseases caused by coronaviruses in a subject.

12. Method for designing and/or obtaining an active ingredient for a vaccine composition comprising the steps of:

(i) providing a mutant receptor binding domain of a virus (VIRUS-mRBD) or a fragment thereof, comprising one or more mutations in a wildtype receptor binding domain of the virus (VIRUS-wtRBD);
(ii) determining, whether the VIRUS-mRBD or the fragment thereof exhibits:

a) a reduced binding strength to a receptor of the receptor binding domain (RBD-receptor) of the virus (VIRUS-RBD-receptor) compared to the VIRUS-wtRBD; and

(iii) selecting the VIRUS-mRBD or the fragment thereof when a) is fulfilled as the active ingredient.

13. Method according to claim 12, further comprising:
in step (ii) determining whether the VIRUS-mRBDs or the fragment thereof exhibits:

b) a binding to anti-VIRUS-wtRBD neutralizing antibodies (VIRUS-wtRBD-nABs); and
c) optionally, a protein and/or peptide stability,

in step (iii) selecting the VIRUS-mRBD or the fragment thereof when concomitantly a) and b) and optionally, concomitantly fulfil a), b) and c) are fulfilled as the active ingredient.

14. Method according to claim 12 or 13, wherein:

step (i) comprises providing a library of VIRUS-mRBDs or the fragments thereof, each comprising one or more mutations in the VIRUS-wtRBD, wherein the VIRUS-mRBDs or the fragments thereof comprised in the library differ at least partially in the mutations;

step (ii) is performed by screening the library for VIRUS-mRBDs or fragments thereof exhibiting:

a) a reduced binding strength to the VIRUS-RBD-receptor compared to the VIRUS-wtRBD; and

step (iii) comprises selecting from the library one or more VIRUS-mRBDs or the fragments thereof which fulfil a) as the active ingredient.

15. Method according to any of claims 12 to 14, wherein:

step (ii) is performed by screening the library for VIRUS-mRBDs or fragments thereof exhibiting:

a) a reduced binding strength to the VIRUS-RBD-receptor compared to the VIRUS-wtRBD; and
b) a binding to VIRUS-wtRBD-nABs; and
c) optionally, a protein and/or peptide stability, and

step (iii) comprises selecting from the library one or more VIRUS-mRBDs or the fragments thereof which concomitantly fulfil a) and b) and optionally, concomitantly fulfil c) as the active ingredient.

16. Method according to any of claims 12 to 15, wherein:
step (iii) further comprises scoring of the one or more VIRUS-mRBDs or the fragments thereof of the library as to their potential to:

a) reduce the binding strength to the VIRUS-RBD-receptor compared to the VIRUS-wtRBD; and
b) optionally, a binding to VIRUS-wtRBD-nABs; and
c) further optionally, exhibit a protein and/or peptide stability; and

selecting, based on the scoring, from the library one or more VIRUS-mRBDs or fragments thereof having the highest score for a), or the highest score for a combination of a) and b) and optionally c) as the active ingredient.

17. Method according to any of claims 12 to 16, wherein steps (i) and (ii) are performed *in vitro* and/or the scoring in step (iii) is performed *in silico.*

18. Method according to any of claims 12 to 17, wherein:

- the VIRUS-mRBD is the CORONA-mRBD according to the present invention, more preferably the SARS-mRBD and/or the MERS-mRBD according to the present invention;
- the VIRUS-wtRBD is the CORONA-wtRBD according to the present invention, more preferably the SARS-wtRBD (SEQ ID NO: 1) and/or the MERS-wtRBD (SEQ ID NO: 194) according to the present invention;
- the VIRUS-RBD-receptor is the CORONA-RBD-receptor, more preferably the ACE2 and/or the DPP4;
- the VIRUS-wtRBD-nABs are CORONA-wtRBD-nABs according to the present invention, more preferably SARS-wtRBD-nABs and/or MERS-wtRBD-nABs according to the present invention; and
- VIRUS-mSpike is the CORONA-mSpike according to the present invention, more preferably the SARS-mSpike and/or the MERS-mSpike according to the present invention.

19. VIRUS-mRBD or the fragment thereof obtained by the method according to any of claims 12 to 18 as the active ingredient.

Fig. 1

Fig. 2

EP 4 144 751 A1

Fig. 3

Fig. 4

| | ACE2-hFcg1 | EY6A | CC12.1 | REGN10933 | P2C-1F11 | C144 | S2H13 | P2B-2F6 | REGN10987 | S309 | 4A8 | VRC01 |
|---|---|---|---|---|---|---|---|---|---|---|---|---|
| wt | 0.22 | 0.52 | 0.24 | 0.33 | 0.35 | 0.44 | 0.56 | 0.30 | 0.42 | 0.64 | ~ | ~ |
| N501Y | 0.01 | 0.07 | 0.25 | 0.05 | 0.06 | 0.06 | 0.07 | 0.03 | 0.05 | 0.10 | ~ | ~ |
| K417N | 0.14 | 0.08 | 3.25 | 0.06 | 0.06 | 0.08 | 0.11 | 0.06 | 0.06 | 0.12 | ~ | ~ |
| L452R | 0.05 | 0.08 | 0.08 | 0.06 | 0.10 | 0.10 | 0.15 | ~ | 0.07 | 0.13 | ~ | ~ |
| G502R | ~ | 0.56 | 0.41 | 0.44 | 0.60 | 0.49 | 0.53 | 0.43 | 0.47 | 0.72 | ~ | ~ |
| E484K | 0.28 | 0.49 | 0.31 | 0.47 | 0.37 | ~ | ~ | ~ | 0.34 | 0.70 | ~ | ~ |
| F456A | 5.96 | 0.08 | 7.36 | 0.12 | ~ | ~ | 0.22 | 0.14 | 0.09 | 0.16 | ~ | ~ |
| L455R | 1.54 | 0.43 | 0.32 | 4126.00 | ~ | ~ | 0.39 | 0.28 | 0.35 | 0.51 | ~ | ~ |
| A475R | 241.1 | 0.12 | ~ | 6.89 | ~ | 0.19 | 1.01 | 1.00 | 0.07 | 0.17 | ~ | ~ |
| A475R+G496R | ~ | 1.09 | ~ | ~ | ~ | 59.53 | 8.20 | 12.30 | 0.54 | 1.13 | ~ | ~ |

Fig. 5

Fig. 6

Fig. 7

Fig. 8

EP 4 144 751 A1

Fig. 9

Fig. 10

# SARS-CoV-2 full spike wild type protein

```
MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQDVNCTEV
PVAIHADQLTPTWRVYSTGSNVFQTRAGCLIGAEHVNNSYECDIPIGAGICASYQTQTNSPR
RARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDKVEAEVQ
IDRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT
```

Receptor binding domain
**Receptor binding motif**
Wild type sequence changed to PP
Trimerization domain changed to PEAPRDGQAYVRKDGEWV
*Extracellular***Transmembrane***Cytoplasmatic (not contained the*
*constructs used in the Examples and preferably not contained*
*in the protein or peptide according to the invention)*

# Fig. 11

# MERS-CoV full spike wild type protein

MIHSVFLLMFLLTPTESYVDVGPDSVKSACIEVDIQQTFFDKTWPRPIDVSKADGIIYPQGR
TYSNITITYQGLFPYQGDHGDMYVYSAGHATGTTPQKLFVANYSQDVKQFANGFVVRIGAAA
NSTGTVIISPSTSATIRKIYPAFMLGSSVGNFSDGKMGRFFNHTLVLLPDGCGTLLRAFYCI
LEPRSGNHCPAGNSYTSFATYHTPATDCSDGNYNRNASLNSFKEYFNLRNCTFMYTYNITED
EILEWFGITQTAQGVHLFSSRYVDLYGGNMFQFATLPVYDTIKYYSIIPHSIRSIQSDRKAW
AAFYVYKLQPLTFLLDFSVDGYIRRAIDCGFNDLSQLHCSYESFDVESGVYSVSSFEAKPSG
SVVEQAEGVECDFSPLLSGTPPQVYNFKRLVFTNCNYNLTKLLSLFSVNDFTCSQISPAAIA
SNCYSSLILDYFSYPLSMKSDLSVSSAGPISQFNYKQSFSNPTCLILAT**VPHNLTTITKPLK**
**YSYINKCSRLLSDDRTEVPQLVNANQYSPCVSIVPSTVWEDGDYYRKQLSPLEGGGWLVASG**
**STVAMTEQL**QMGFGITVQYGTDTNSVCPKLEFANDTKIASQLGNCVEYSLYGVSGRGVFQNC
TAVGVRQQRFVYDAYQNLVGYYSDDGNYYCLRACVSVPVSVIYDKETKTHATLFGSVACEHI
SSTMSQYSRSTRSMLKRRDSTYGPLQTPVGCVLGLVNSSLFVEDCKLPLGQSLCALPDTPST
LTPRSVRSVPGEMRLASIAFNHPIQVDQLNSSYFKLSIPTNFSFGVTQEYIQTTIQKVTVDC
KQYVCNGFQKCEQLLREYGQFCSKINQALHGANLRQDDSVRNLFASVKSSQSSPIIPGFGGD
FNLTLLEPVSISTGSRSARSAIEDLLFDKVTIADPGYMQGYDDCMQQGPASARDLICAQYVA
GYKVLPPLMDVNMEAAYTSSLLGSIAGVGWTAGLSSFAAIPFAQSIFYRLNGVGITQQVLSE
NQKLIANKFNQALGAMQTGFTTTNEAFRKVQDAVNNNAQALSKLASELSNTFGAISASIGDI
IQRLDVLEQDAQIDRLINGRLTTLNAFVAQQLVRSESAALSAQLAKDKVNECVKAQSKRSGF
CGQGTHIVSFVVNAPNGLYFMHVGYYPSNHIEVVSAYGLCDAANPTNCIAPVNGYFIKTNNT
RIVDEWSYTGSSFYAPEPITSLNTKYVAPHVTYQNISTNLPPPLLGNSTGIDFQDELDEFFK
NVSTSIPNFGSLTQINTTLLDLTYEMLSLQQVVKALNESYIDLKELGNYTY*YNKWP***WYIWLG***
***FIAGLVALALCVFFILCC**TGCGTNCMGKLKCNRCCDRYEEYDLEPHKVHVH*

Receptor binding domain
**Receptor binding motif**
Wild type sequence changed to PP
*Extracellular**Transmembrane**Cytoplasmatic (not contained the*
*constructs used in the Examples and preferably not contained*
*in the protein or peptide according to the invention)*

# Fig. 12

EP 4 144 751 A1

# EP 4 144 751 A1

Europäisches
Patentamt

European
Patent Office

Office européen
des brevets

## EUROPEAN SEARCH REPORT

Application Number

EP 21 19 4414

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AZAD TAHA ET AL: "Nanoluciferase complementation-based bioreporter reveals the importance of N-linked glycosylation of SARS-CoV-2 S for viral entry", MOLECULAR THERAPY, vol. 29, no. 6, 1 June 2021 (2021-06-01), pages 1984-2000, XP055862562, US ISSN: 1525-0016, DOI: 10.1016/j.ymthe.2021.02.007 Retrieved from the Internet: URL:https://www.ncbi.nlm.nih.gov/pmc/artic les/PMC7872859/pdf/main.pdf> * abstract * * page 1992, column 2, paragraph 1 - page 1994, column 1, paragraph 1 * * page 1998, column 1, paragraph 4 * * figures 1,5,7 * | 1-11,19 | INV. C07K14/005 A61K39/12 |
| X | CHAKRABORTY SANDIPAN ED – NERI DARIO ET AL: "Evolutionary and structural analysis elucidates mutations on SARS-CoV2 spike protein with altered human ACE2 binding affinity", BIOCHEMICAL AND BIOPHYSICAL RESEARCH COMMUNICATIONS, ELSEVIER, AMSTERDAM NL, vol. 534, 28 November 2020 (2020-11-28), pages 374-380, XP086429330, ISSN: 0006-291X, DOI: 10.1016/J.BBRC.2020.11.075 [retrieved on 2020-11-28] * abstract * | 1,2,4-7, 19 | |
| | -/-- | | TECHNICAL FIELDS SEARCHED (IPC) C07K A61K C12N |

The present search report has been drawn up for all claims

| Place of search | Date of completion of the search | Examiner |
|---|---|---|
| Munich | 27 April 2022 | Mossier, Birgit |

EPO FORM 1503 03.82 (P04C01)

Europäisches
Patentamt

European
Patent Office

Office européen
des brevets

**EUROPEAN SEARCH REPORT**

Application Number

**EP 21 19 4414**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/163536 A2 (ALTIMMUNE INC [US]) 19 August 2021 (2021-08-19) * abstract * * paragraph [0009] * * paragraph [0010] * * paragraph [0115] * * claim 11; figure 17 * | 1-11,19 | |
| A | ZAHRADNÍK JIRÍ ET AL: "SARS-CoV-2 variant prediction and antiviral drug design are enabled by RBD in vitro evolution", NATURE MICROBIOLOGY, NATURE PUBLISHING GROUP UK, LONDON, vol. 6, no. 9, 16 August 2021 (2021-08-16) , pages 1188-1198, XP037548549, DOI: 10.1038/S41564-021-00954-4 [retrieved on 2021-08-16] * abstract * * tables 1,2 * | 1-11 | |
| A | HARVEY WILLIAM T ET AL: "SARS-CoV-2 variants, spike mutations and immune escape", NATURE REVIEWS MICROBIOLOGY, NATURE PUBLISHING GROUP, GB, vol. 19, no. 7, 1 June 2021 (2021-06-01), pages 409-424, XP037482862, ISSN: 1740-1526, DOI: 10.1038/S41579-021-00573-0 [retrieved on 2021-06-01] * abstract * * page 422, column 1, paragraph 3 - column 2; figure 1 * | 1-11 | TECHNICAL FIELDS SEARCHED (IPC) |

-/--

The present search report has been drawn up for all claims

| Place of search | Date of completion of the search | Examiner |
|---|---|---|
| Munich | 27 April 2022 | Mossier, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

Application Number

EP 21 19 4414

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RUI WANG ET AL: "Vaccine-escape and fast-growing mutations in the United Kingdom, the United States, Singapore, Spain, South Africa, and other COVID-19-devastated countries", ARXIV.ORG, CORNELL UNIVERSITY LIBRARY, 201 OLIN LIBRARY CORNELL UNIVERSITY ITHACA, NY 14853, 21 March 2021 (2021-03-21), XP081910925, * abstract; tables 3,4 * | 1-11 | |
| A | DUAN LIANGWEI ET AL: "The SARS-CoV-2 Spike Glycoprotein Biosynthesis, Structure, Function, and Antigenicity: Implications for the Design of Spike-Based Vaccine Immunogens", FRONTIERS IN IMMUNOLOGY, vol. 11, 7 October 2020 (2020-10-07), XP055893073, DOI: 10.3389/fimmu.2020.576622 * abstract * * page 6, column 1, paragraph 3 - page 8, column 1, paragraph 2; figure 2 * | 1-11 | |
| X | LANYING DU ET AL: "Introduction of neutralizing immunogenicity index to the rational design of MERS coronavirus subunit vaccines", NATURE COMMUNICATIONS, vol. 7, 22 November 2016 (2016-11-22), page 13473, XP55486939, DOI: 10.1038/ncomms13473 | 19 | |
| A | * abstract * * page 3, column 2, paragraph 1 * | 1-11 | |

TECHNICAL FIELDS SEARCHED (IPC)

-/--

The present search report has been drawn up for all claims

| Place of search | Date of completion of the search | Examiner |
|---|---|---|
| Munich | 27 April 2022 | Mossier, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Dickey Thayne H. ET AL: "Design of the SARS-CoV-2 RBD vaccine antigen improves neutralizing antibody response", bioRxiv, 10 May 2021 (2021-05-10), XP055915754, DOI: 10.1101/2021.05.09.443238 Retrieved from the Internet: URL:https://www.biorxiv.org/content/10.1101/2021.05.09.443238v1.full.pdf [retrieved on 2022-04-26] * abstract * * page 3, paragraph 2 – page 6, paragraph 1 * * page 11; figures 1-3; table 3 * | 12-18 | |
| A | STARR TYLER N. ET AL: "Deep Mutational Scanning of SARS-CoV-2 Receptor Binding Domain Reveals Constraints on Folding and ACE2 Binding", CELL, vol. 182, no. 5, 1 September 2020 (2020-09-01), pages 1295-1310.e20, XP055850377, Amsterdam NL ISSN: 0092-8674, DOI: 10.1016/j.cell.2020.08.012 Retrieved from the Internet: URL:https://www.sciencedirect.com/science/article/pii/S0092867420310035/pdfft?md5=1834ea9b3dee52139e4fadc0053f144e&pid=1-s2.0-S0092867420310035-main.pdf> * abstract * * page 1298, column 2, paragraph 4 – page 1299, column 1, paragraph 1 * * page 1305, column 2, paragraph 2 – paragraph 3 * | 12-18 | TECHNICAL FIELDS SEARCHED (IPC) |

-/--

The present search report has been drawn up for all claims

| Place of search | Date of completion of the search | Examiner |
|---|---|---|
| Munich | 27 April 2022 | Mossier, Birgit |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

......................................................

& : member of the same patent family, corresponding
    document

EPO FORM 1503 03.82 (P04C01)

| | Europäisches Patentamt<br>European Patent Office<br>Office européen des brevets | **EUROPEAN SEARCH REPORT** | **Application Number**<br><br>**EP 21 19 4414** |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TAI WANBO ET AL: "Recombinant Receptor-Binding Domains of Multiple Middle East Respiratory Syndrome Coronaviruses (MERS-CoVs) Induce Cross-Neutralizing Antibodies against Divergent Human and Camel MERS-CoVs and Antibody Escape Mutants", JOURNAL OF VIROLOGY, vol. 91, no. 1, 1 January 2017 (2017-01-01), XP055892495, US ISSN: 0022-538X, DOI: 10.1128/JVI.01651-16 Retrieved from the Internet: URL:https://journals.asm.org/doi/pdf/10.1128/JVI.01651-16> * abstract * * figure 1; tables 1,2 * | 12-18 | |
| A | DESHPANDE ASHLESHA ET AL: "Epitope Classification and RBD Binding Properties of Neutralizing Antibodies Against SARS-CoV-2 Variants of Concern", FRONTIERS IN IMMUNOLOGY, vol. 12, 4 June 2021 (2021-06-04), XP055915891, DOI: 10.3389/fimmu.2021.691715 * abstract; tables 1,2 * | 1-19 | TECHNICAL FIELDS SEARCHED (IPC) |
| A | CHATTERJEE RAHUL ET AL: "Next-Generation Bioinformatics Approaches and Resources for Coronavirus Vaccine Discovery and Development-A Perspective Review", VACCINES, vol. 9, no. 8, 22 July 2021 (2021-07-22), page 812, XP055915892, DOI: 10.3390/vaccines9080812 * abstract; figure 1; table 1 * | 1-19 | |

-/--

The present search report has been drawn up for all claims

| Place of search | Date of completion of the search | Examiner |
|---|---|---|
| Munich | 27 April 2022 | Mossier, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

**EP 21 19 4414**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VERMA JYOTI ET AL: "A comparative study of human betacoronavirus spike proteins: structure, function and therapeutics", ARCHIVES OF VIROLOGY, vol. 166, no. 3, 22 January 2021 (2021-01-22), pages 697-714, XP037369917, ISSN: 0304-8608, DOI: 10.1007/S00705-021-04961-Y * abstract; figures 1-4; tables 1,2 * | 1-19 | |

TECHNICAL FIELDS
SEARCHED       (IPC)

The present search report has been drawn up for all claims

| Place of search | Date of completion of the search | Examiner |
|---|---|---|
| Munich | 27 April 2022 | Mossier, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

| | | Application Number |
|---|---|---|
| Europäisches Patentamt European Patent Office Office européen des brevets | | **EP 21 19 4414** |

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

**12-19(completely); 1-11(partially)**

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**

**SHEET B**

Application Number

EP 21 19 4414

The Search Division considers that the present European patent application does not comply with the requirements of unity of invention and relates to several inventions or groups of inventions, namely:

1. claims: 1-11(partially)

    Mutant receptor-binding domain (mRBD) of a coronavirus (CORONA-mRBD) or a fragment thereof having a reduced binding strength to a RBD-receptor of the coronavirus (CORONA-RBD-receptor) compared to a wild type receptor-binding domain of the coronavirus (CORONA-wtRBD), wherein the coronavirus is SARS-CoV-2 (severe acute respiratory syndrome coronavirus type 2), the CORONA-mRBD is a mutant receptor-binding domain of SARS-CoV-2 (SARS-mRBD) or a fragment thereof, the wild type receptor-binding domain is SARS-wtRBD (wildtype receptor binding domain of SARS-CoV-2), and the RBD-receptor is ACE2 (angiotensin-converting enzyme 2) and the subject-matter relating thereto.
    ---

2. claims: 1-11(partially)

    Mutant receptor-binding domain (mRBD) of a coronavirus (CORONA-mRBD) or a fragment thereof having a reduced binding strength to a RBD-receptor of the coronavirus (CORONA-RBD-receptor) compared to a wild type receptor-binding domain of the coronavirus (CORONA-wtRBD), wherein the coronavirus is MERS-CoV (middle east respiratory syndrome coronavirus), the CORONA-mRBD is a mutant receptor-binding domain of MERS-CoV (MERS-mRBD) or a fragment thereof, the wild type receptor-binding domain is MERS-wtRBD (wildtype receptor binding domain of MERS-CoV), and the RBD-receptor is DPP4 (dipeptidylpeptidase 4) and the subject-matter relating thereto.
    ---

3. claims: 12-19

    Method for designing and/or obtaining an active ingredient for a vaccine composition comprising the steps of:(i) providing a mutant receptor binding domain of a virus (VIRUS-mRBD) or a fragment thereof, comprising one or more mutations in a wildtype receptor binding domain of the virus (VIRUS-wtRBD);(ii) determining, whether the VIRUS-mRBD or the fragment thereof exhibits:a) a reduced binding strength to a receptor of the receptor binding domain (RBD-receptor) of the virus (VIRUS-RBD-receptor) compared to the VIRUS-wtRBD; and(iii) selecting the VIRUS-mRBD or the fragment thereof when a) is fulfilled as the active ingredient and the subject-matter relating thereto.
    ---

## ANNEX TO THE EUROPEAN SEARCH REPORT
## ON EUROPEAN PATENT APPLICATION NO.

EP 21 19 4414

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-2022

| Patent document cited in search report | | Publication date | Patent family member(s) | | Publication date |
|---|---|---|---|---|---|
| WO 2021163536 | A2 | 19-08-2021 | CN 114096675 | A | 25-02-2022 |
| | | | TW 202144383 | A | 01-12-2021 |
| | | | US 2021260180 | A1 | 26-08-2021 |
| | | | US 2022072121 | A1 | 10-03-2022 |
| | | | WO 2021163536 | A2 | 19-08-2021 |

EPO FORM P0459

For more details about this annex : see Official Journal of the European Patent Office, No. 12/82